# EUROPEAN PATENT APPLICATION

(11) **EP 1 466 975 A1**
(43) Date of publication of application: **13.10.2004**
(21) Application number: 02788621.7
(22) Date of filing: 20.11.2002
(51) Int. Cl.: C12N 15/12, C12N 1/21, C12P 21/02, C07K 14/435, C07K 16/18, C12Q 1/68, A61K 45/00, A61K 38/00, A61K 39/395, A61P 25/00, A61P 25/28, A61P 43/00, G01N 33/50, G01N 33/15, G01N 33/53

(54) **POSTSYNAPTIC PROTEINS**

(30) Priority: 20.11.2001 JP 2001354678; 22.02.2002 JP 2002046786; 07.08.2002 JP 2002229863
(71) Applicant: DAIICHI PHARMACEUTICAL CO., LTD., Chuo-ku, Tokyo 103-8234 (JP); Kazusa DNA Research Institute Foundation, Kisarazu-shi, Chiba 292-0818 (JP)
(72) Inventor: OHARA, Osamu, c/o Kazusa DNA Res.Inst.Found., Kisarazu-shi, Chiba 292-0818 (JP); NAGASE, Takahiro, Kazusa DNA Res. Inst. Found., Kisarazu-shi, Chiba 292-0818 (JP); OHISHI, Michio, Kazusa Dna Res. Inst. Foundation, Kisarazu-shi, Chiba 292-0818 (JP); YOKOTA, Hiroshi, c/o Daiichi Pharm. Co., Ltd., Tokyo 134-8630 (JP); ARAI, Yasuko, c/o Daiichi Pharmaceutical Co., Ltd., Tokyo 134-8630 (JP)
(74) Representative: Krauss, Jan B., Dr.
(86) International application number: PCT/JP2002/012102
(87) International publication number: WO 2003/044196

(57) **Abstract**

A protein A that binds to the N-methyl-D-aspartate (NMDA) receptor and a protein B that interacts with protein A are provided. Furthermore, based on the marked promotion of the NMDA receptor mediated signal transduction by these proteins, providing a controlling agent (an inhibiting agent or a promoting agent) and a controlling method for the expression and/or the function of these proteins makes possible the elucidation of diseases caused by an anomaly in the NMDA receptor mediated signal transduction or in memory recall, including such neurodegenerative diseases as Alzheimer's disease, Parkinson's disease and polyglutamine disease, and allows for the prevention, improvement or treatment thereof.

## Description

### TECHNICAL FIELD

The present invention relates to a postsynaptic density protein (hereinafter referred to as protein A), which possesses a PDZ domain and is capable of forming a complex with an N-methyl-D-aspartate (hereinafter abbreviated as NMDA) receptor; and a protein (hereinafter referred to as protein B), which interacts with protein A; as well as a controlling agent (an inhibiting agent or a promoting agent) of an NMDA receptor mediated signal transduction, which inhibits or promotes the formation of the complex of protein A and the NMDA receptor; a controlling agent of NMDA receptor mediated signal transduction, which inhibits or promotes the interaction of protein A with protein B; a method for controlling an NMDA receptor mediated signal transduction by inhibiting or promoting the formation of the complex of protein A and the NMDA receptor; and a method for controlling an NMDA receptor mediated signal transduction by inhibiting or promoting the interaction of protein A with protein B. More specifically, it relates to a polypeptide or peptide having complete or a partial amino acid sequence of protein A or protein B; a polynucleotide containing a nucleotide sequence coding for the polypeptide or peptide, or containing the complementary nucleotide sequences thereof; a recombinant vector containing the polynucleotide; a transformant containing the recombinant vector; an antibody directed against the polypeptide or peptide; a compound that interacts with the polypeptide or peptide; a pharmaceutical composition containing one or more of the above; a method for manufacturing the polypeptide or peptide; a method for identifying compounds that interact with the polypeptide or peptide; a method for measuring the polypeptide or peptide; and a reagent kit.

### TECHNICAL BACKGROUND

A postsynaptic density protein (hereinafter abbreviated as PSD) forms the postsynaptic densities that are present in postsynaptic cells and receive the synaptic information during signal transduction between nerve cells. In the postsynaptic membrane, PSD is involved in the localization and accumulation of functional membrane proteins (such as receptors and ion channels), or the formation of bio-multisomes.

Meanwhile, proteins (that are involved in biological functions such as the formation and maintenance of complex cell membrane structures (such as synapses and tight junctions), the accumulation of membrane protein, signal transduction, the formation of complexes and the maintenance of cell polarity) were reported to possess a peptide-binding domain thought to be involved in these functions as a module that bears protein-protein interactions (Non-Patent Reference 1). This domain is called a PDZ domain and comprises 90 to 100 amino acid residues. Among these biological functions, the PDZ domain is thought to play a more important role in functions such as localizing a specific protein to a specific intracellular site, or promoting the binding of a protein containing the PDZ domain to a target protein. For example, involvement of the PDZ domain in the localization of signal transduction proteins to protein complexes in the cell membrane may be necessary to achieve appropriate signal transduction.

In protein-protein interactions, the PDZ domain recognizes the C-terminal amino acid sequence of a target protein. The target protein is often a transmembrane receptor or channel. For example, PSD-95 and PSD-93 (chapsyn 110 or KAP-5), which belong to the MAGUK protein (membrane associated guanylate kinase protein) family that is present in the synapse of vertebrates or invertebrates, and hdlg (a human homolog of Drosophila dlg protein) have been reported to recognize the C-terminus of an NMDA receptor and the Shaker-type potassium ion channel Kir 1.4, through their second and third PDZ domains (Non-Patent Reference 2).

By analyzing the in vivo localization of proteins that possess the PDZ domain, and the localization of target proteins of the transmembrane type that have been identified by in vitro biochemical analyses, it was demonstrated that PSD-95, PSD-93, and hdlg all showed the same localization as an NMDA receptor, or a potassium ion channel, in each tissue as well as in each cell species of the nervous system (Non-Patent Reference 3, Non-Patent Reference 4, Non-Patent Reference 5, and Non-Patent Reference 6).

In addition, when PSD-95 is co-expressed in fibroblasts with the NMDA receptor or a potassium ion channel, clustering of these proteins on the cell surface is observed, while when they are each expressed alone, only a diffuse localization is observed (Non-Patent Reference 4). Meanwhile, the splicing isoform, which lacks the C-terminal amino acid motif (S/TXV) that is recognized by PSD-95, shows a diffuse localization (Non-Patent Reference 7). From the foregoing, it is thought that the MAGUK protein is directly involved in the cross-linking and accumulation of cell membrane proteins through interaction via the PDZ domain.

In addition, activation of PKA (Protein Kinase A) in fibroblasts into which Kir has been transfected, results in the phosphorylation of serine at position 440 (S440) of the amino acid sequence of Kir, dissociation of Kir from PSD-95, and the diminution of potassium ion conductivity. When S440 is replaced with alanine, such effect by PKA is not observed. These results show that binding of Kir to PSD-95 clusterizes the channels, and furthermore renders channel conductivity susceptible to PKA.

PSD-95 is a postsynaptic density protein, which is known to be involved in a number of functions such as embryonic development, neurogenesis, neurotransmission, signal transduction, and protein complex formation. In addition, transferase activity, kinase activity, activity as an anchor protein in coupling membrane proteins (such as receptors and ion channels) to the cytoskeleton, and activity as an adhesin/agglutinin that participates in the aggregation and conjunction of cells, have been reported (Non-Patent References 4 and 8). From the foregoing, PSD-95 is thought to be involved in the control of neurotransmitter release, and in the inhibition of cell proliferation and cancers (Non-Patent References 9, 10, and 11).

PSD-95 has three PDZ domains, one SH3 domain (Src homology region 3 domain), and one GK domain (guanylate kinase domain) in the amino acid sequence thereof.

PSD-95 binds via the PDZ domain thereof to the 2B subunit, which is a component of the NMDA receptor present in the postsynaptic membrane (Non-Patent References 2, 12, 13, and 14). It is thus believed that PSD-95 is a cytoskeletal protein involved in the signal transduction mediated by the receptor, or the like.

In addition, PSD95 has been reported to interact via the GK domain thereof with PSD95/SAP90-associated protein-3 (hereinafter abbreviated as SAPAP-3), in a rat (Non-Patent Reference 15). Rat SAPAP-3 is expressed specifically in nerve cells, and is present abundantly in the postsynaptic density fraction. In this report, rat SAPAP-3 is thought to be involved in the maintenance of postsynaptic density structures by assembling postsynaptic density components, such as PSD95/SAP90, in the membrane region.

Meanwhile, the NMDA receptor is one type of glutamate receptor, which is thought to form an ion channel in the cell membrane, since it possesses a higher order structure, such as second or third structure, common to ligand-dependent ion channels. The ion channel is permeable to calcium ions and has the physiological function of increasing the calcium concentration inside the postsynaptic membrane. The NMDA receptor is normally obstructed by magnesium ions present in the synaptic cleft and is thought not to be involved in a single synapse transmission. However, when the membrane potential is depolarized as a result of frequent stimulation or the like, obstruction by the magnesium ions is removed, and the NMDA receptor is activated. Glutamic acid and aspartic acid can be exemplified as in vivo ligands. Since memory impairment is provoked by administering amino-phosphono-valeric acid or MK-801 and the like (which are known as inhibitors of the NMDA receptor) directly into the brain of a mammal, it is suggested that the NMDA receptor is involved in the establishment of memory.

As described in the foregoing, the PDZ domain is anticipated to function in a considerably wide range of biological phenomena. Therefore, by elucidating and controlling the complexes formed by proteins that possess the PDZ domain and the proteins involved in these complexes, it becomes possible to prevent, treat, and diagnose diseases caused by anomalies in protein-protein interactions, such as the formation of cytoskeletal structures, accumulation of membrane proteins, signal transduction, formation of complexes, and maintenance of cell polarity. For example, by discovering a novel PSD that possesses the PDZ domain, it becomes possible to elucidate diseases in which an anomaly of the PSD is involved, for example, neurodegenerative diseases, as well as the prevention, treatment, and diagnosis thereof. That is to say, there is a need for finding a protein that possesses the PDZ domain and a protein that interacts with that protein, as well as a method for controlling the interactions of the two proteins, in the field of study of protein-protein interaction and recognition mechanism through the PDZ domain, as well as in the field of pharmaceutical development.

The references cited in the description of the present technical background are listed below.
Non-Patent Reference 1: *Saibokogaku,* 2001, Vol. 20, pp. 1345-1349.
Non-Patent Reference 2: Niethammer, M., et al., Journal of Neuroscience, 1996, Vol. 16, pp. 2157-2163.
Non-Patent Reference 3: Kornau, H.C., et al., Science, 1995, Vol. 269, pp. 1737-1740.
Non-Patent Reference 4: Kim, E., et al., Nature, 1995, Vol. 378, pp. 85-88.
Non-Patent Reference 5: Kim, E., et al., Neuron, 1996, Vol. 17, pp. 103-113.
Non-Patent Reference 6: Miller, B.M., et al., Journal of Neuroscience, 1995, Vol. 15, pp. 2354-2366.
Non-Patent Reference 8: Stathakis, D., et al., Genomics, 1997, Vol. 44, pp. 71-82.
Non-Patent Reference 9: Hanada, T., et al., Journal of Biological Chemistry, 2000, Vol. 275, pp. 28774-28784.
Non-Patent Reference 10: Migaud, M., et al., Nature, 1998, Vol. 396, pp. 433-439.
Non-Patent Reference 11: Sattler, R., et al., Science, 1999, Vol. 284, pp.1845-1848.
Non-Patent Reference 12: Monyer, H., et al., Science, 1992, Vol. 256, pp. 1217-1221.
Non-Patent Reference 13: Ishii, T., et al., Journal of Biological Chemistry, 1993, Vol. 268, pp. 2836-2843.
Non-Patent Reference 14: Sheng, M., et al., Nature, 1994, Vol. 368, pp. 144-147.
Non-Patent Reference 15: Takeuchi, M., et al., Journal of Biological Chemistry, 1997, Vol. 272, pp. 11943-11951.

### DISCLOSURE OF THE INVENTION

The present invention was achieved by way of discovering and using a protein A that comprises a specific amino acid sequence having a PDZ domain acting as a module for carrying out protein-protein interactions and is capable of forming a complex with an NMDA receptor; a protein B that interacts with the protein A; and genes coding therefor.

That is to say, one aspect of the present invention is an agent for controlling NMDA receptor mediated signal transduction, wherein the agent inhibits or promotes the binding of a polypeptide having the amino acid sequence set forth in SEQ ID NO: 1 in the Sequence Listing, to the NMDA receptor, and/or, inhibits or promotes the interaction of a polypeptide having the amino acid sequence set forth in SEQ ID NO: 3 in the Sequence Listing, with the polypeptide having the amino acid sequence set forth in SEQ ID NO: 1 in the Sequence Listing.

In addition, one aspect of the present invention is an agent for inhibiting NMDA receptor mediated signal transduction, wherein the agent inhibits binding of the polypeptide having the amino acid sequence set forth in SEQ ID NO: 1 in the Sequence Listing, to the NMDA receptor, and/or inhibits the interaction of the polypeptide having the amino acid sequence set forth in SEQ ID NO: 3 in the Sequence Listing, with the polypeptide having the amino acid sequence set forth in SEQ ID NO: 1 in the Sequence Listing.

In addition, one aspect of the present invention is an agent for enhancing NMDA receptor mediated signal transduction, wherein the agent promotes the binding of the polypeptide having the amino acid sequence set forth in SEQ ID NO: 1 in the Sequence Listing, to the NMDA receptor, and/or promotes the interaction of the polypeptide having the amino acid sequence set forth in SEQ ID NO: 3 in the Sequence Listing with the polypeptide having the amino acid sequence set forth in SEQ ID NO: 1 in the Sequence Listing.

In addition, one further aspect of the present invention is a method for controlling NMDA receptor mediated signal transduction, wherein the method includes inhibiting or promoting the binding of the polypeptide having the amino acid sequence set forth in SEQ ID NO: 1 in the Sequence Listing, to the NMDA receptor, and/or, inhibiting or promoting the interaction of the polypeptide having the amino acid sequence set forth in SEQ ID NO: 3 in the Sequence Listing, with the polypeptide having the amino acid sequence set forth in SEQ ID NO: 1 in the Sequence Listing.

In addition, one further aspect of the present invention is a method for inhibiting NMDA receptor mediated signal transduction, wherein the method includes inhibiting the binding of the polypeptide having the amino acid sequence set forth in SEQ ID NO: 1 in the Sequence Listing, to the NMDA receptor, and/or inhibiting the interaction of the polypeptide having the amino acid sequence set forth in SEQ ID NO: 3 in the Sequence Listing, with the polypeptide having the amino acid sequence set forth in SEQ ID NO: 1 in the Sequence Listing.

In addition, one aspect of the present invention is a method for promoting NMDA receptor mediated signal transduction, wherein the method includes promoting the binding of the polypeptide having the amino acid sequence set forth in SEQ ID NO: 1 in the Sequence Listing, to the NMDA receptor, and/or promoting the interaction of the polypeptide having the amino acid sequence set forth in SEQ ID NO: 3 in the Sequence Listing, with the polypeptide having the amino acid sequence set forth in SEQ ID NO: 1 in the Sequence Listing.

In addition, one aspect of the present invention is a polypeptide selected from the following polypeptides:
i. a polypeptide having the amino acid sequence set forth in SEQ ID NO: 1 or 2 in the Sequence Listing;
ii. a polypeptide containing the polypeptide set forth above in (i);
iii. a polypeptide having at least approximately 70% homology to the polypeptide set forth above in (i) at the amino acid sequence level and binding to the NMDA receptor/2B subunit; or
iv. a polypeptide having a mutation, which is a deletion, a substitution, an addition, or an insertion of one to several amino acids in the amino acid sequence of the polypeptide set forth above in (i), and being capable of binding to the NMDA receptor/2B subunit.

In addition, one further aspect of the present invention is a polypeptide selected from the following polypeptides, wherein the polypeptide binds to an NMDA receptor/2B subunit:
i. a polypeptide having the amino acid sequence set forth in SEQ ID NO: 1 or 2 in the Sequence Listing;
ii. a polypeptide containing the polypeptide set forth above in (i);
iii. a polypeptide having at least approximately 70% homology to the polypeptide set forth above in (i) at the amino acid sequence level; or
iv. a polypeptide having a mutation, which is a deletion, a substitution, an addition, or an insertion of one to several amino acids in the amino acid sequence of the polypeptide set forth above in (i).

In addition, one further aspect of the present invention is a polypeptide selected from the following polypeptides:
i. a polypeptide having the amino acid sequence set forth in SEQ ID NO: 3 in the Sequence Listing;
ii. a polypeptide containing the polypeptide set forth above in (i);
iii. a polypeptide having at least approximately 70% homology to the polypeptide set forth above in (i) at the amino acid sequence level, and interacting with the polypeptide having the amino acid sequence set forth in SEQ ID NO: 1 in the Sequence Listing; or
iv. a polypeptide having a mutation, which is a deletion, a substitution, an addition, or an insertion of one to several amino acids in the amino acid sequence of the polypeptide set forth above in (i) and interacting with the polypeptide having the amino acid sequence set forth in SEQ ID NO: 1 in the Sequence Listing.

In addition, one aspect of the present invention is a polypeptide selected from the following polypeptides, wherein the polypeptide interacts with the polypeptide having the amino acid sequence set forth in SEQ ID NO: 1 in the Sequence Listing:
i. a polypeptide having the amino acid sequence set forth in SEQ ID NO:, 3 in the Sequence Listing;
ii. a polypeptide containing the polypeptide set forth above in (i);
iii. a polypeptide having at least approximately 70% homology to the polypeptide set forth above in (i) at the amino acid sequence level; or
iv. a polypeptide having a mutation, which is a deletion, a substitution, an addition, or an insertion of one to several amino acids, in the amino acid sequence, of the polypeptide set forth above in (i).

In addition, one aspect of the present invention is a polypeptide selected from the following polypeptides, wherein the polypeptide amplifies NMDA receptor mediated signal transduction, in the presence of the polypeptide having the amino acid sequence set forth in SEQ ID NO: 1 in the Sequence Listing:
i. a polypeptide having the amino acid sequence set forth in SEQ ID NO: 3 in the Sequence Listing;
ii. a polypeptide containing the polypeptide set forth above in (i);
iii. a polypeptide having at least approximately 70% homology to the polypeptide set forth above in (i) at the amino acid sequence level; or
iv. a polypeptide having a mutation, which is a deletion, a substitution, an addition, or an insertion of one to several amino acids in the amino acid sequence of the polypeptide set forth above in i).

In addition, one aspect of the present invention is one of the following polypeptides, which binds to the NMDA receptor/2B subunit but does not interact with the polypeptide having the amino acid sequence set forth in SEQ ID NO: 3 in the Sequence Listing:
i. a polypeptide having at least approximately 70% homology at the amino acid sequence level to the polypeptide having the amino acid sequence set forth in SEQ ID NO: 1 or 2 in the Sequence Listing; or
ii. a polypeptide having a mutation, which is a deletion, a substitution, an addition, or an insertion of one to several amino acids, in the amino acid sequence of the polypeptide set forth above in (i).

In addition, one further aspect of the present invention is a peptide comprising at least five consecutive amino acid residues from the amino acid sequence set forth in SEQ ID NO: 1 or 2 in the Sequence Listing.

In addition, one aspect of the present invention is a peptide comprising at least five consecutive amino acid residues within the amino acid sequence set forth in SEQ ID NO: 1 or 2 in the Sequence Listing and binding to the NMDA receptor.

In addition, one aspect of the present invention is a peptide comprising at least five consecutive amino acid residues within the amino acid sequence set forth in SEQ ID NO: 1 in the Sequence Listing, and interacting with the polypeptide having the amino acid sequence set forth in SEQ ID NO: 3 in the Sequence Listing.

In addition, one further aspect of the present invention is a peptide comprising at least five consecutive amino acid residues within the amino acid sequence set forth in SEQ ID NO: 3 in the Sequence Listing.

In addition, one aspect of the present invention is a peptide comprising at least five consecutive amino acid residues within the amino acid sequence set forth in SEQ ID NO: 3 in the Sequence Listing, and interacting with the polypeptide having the amino acid sequence set forth in SEQ ID NO: 1 in the Sequence Listing.

In addition, one aspect of the present invention is an agent for controlling NMDA receptor mediated signal transduction, wherein the agent includes an effective amount of at least one kind of polypeptide or peptide selected from the aforementioned polypeptides and peptides.

In addition, one aspect of the present invention is an agent for inhibiting NMDA receptor mediated signal transduction, wherein the agent includes an effective amount of at least one kind of polypeptide or peptide selected from the aforementioned polypeptides and peptides.

In addition, one aspect of the present invention is an agent for promoting NMDA receptor mediated signal transduction, wherein the agent includes an effective amount of at least one type of polypeptide or peptide selected from the aforementioned polypeptides and peptides.

In addition, one aspect of the present invention is a method for controlling NMDA receptor mediated signal transduction, wherein the method includes using at least one kind of polypeptide or peptide selected from the aforementioned polypeptides and peptides.

In addition, one aspect of the present invention is a method for inhibiting NMDA receptor mediated signal transduction, wherein the method includes using at least one kind of polypeptide or peptide selected from the aforementioned polypeptides and peptides.

In addition, one aspect of the present invention is a method for promoting NMDA receptor mediated signal transduction, wherein the method includes using at least one kind of polypeptide or peptide selected from the aforementioned polypeptides and peptides.

In addition, one further aspect of the present invention is a polynucleotide including a nucleotide sequence coding for any of the aforementioned polypeptides or peptides, or a complementary nucleotide sequence thereof.

In addition, one further aspect of the present invention is a polynucleotide having a nucleotide sequence set forth in SEQ ID NO: 4 or 5 in the Sequence Listing, or a complementary nucleotide sequence thereof.

In addition, one aspect of the present invention is a polynucleotide having a nucleotide sequence set forth in SEQ ID NO: 6 in the Sequence Listing, or a complementary nucleotide sequence thereof.

In addition, one further aspect of the present invention is a polynucleotide that hybridizes with the aforementioned polynucleotide under stringent conditions.

Furthermore, one aspect of the present invention is a recombinant vector that contains the aforementioned polynucleotide.

Furthermore, one aspect of the present invention is the aforementioned recombinant vector, wherein the recombinant vector is a recombinant expression vector.

In addition, one aspect of the present invention is a transformant that has been transfected with the aforementioned recombinant vector.

In addition, one aspect of the present invention is a method for manufacturing the aforementioned polypeptide or peptide, wherein the method includes a step of culturing the transformant that has been transformed with the aforementioned recombinant vector, or a cell-free protein synthesis means that uses the recombinant vector.

Furthermore, one aspect of the present invention is an antibody that immunologically recognizes the aforementioned polypeptide and/or peptide.

Furthermore, one additional aspect of the present invention is the aforementioned antibody that inhibits the function of the aforementioned polypeptide.

In addition, one aspect of the present invention is the aforementioned antibody which inhibits the interaction of the polypeptide having the amino acid sequence set forth in SEQ ID NO: 1 in the Sequence Listing, with the polypeptide having the amino acid sequence set forth in SEQ ID NO: 3 in the Sequence Listing.

In addition, one further aspect of the present invention is a method for identifying a compound that interacts with the aforementioned polypeptide and inhibits or promotes the function thereof and/or a compound that interacts with the aforementioned polynucleotide and inhibits or promotes the expression thereof, wherein the method includes using at least one selected from the group consisting of the aforementioned polypeptides, polynucleotides, recombinant vectors, transformants, and antibodies.

In addition, one further aspect of the present invention is a method for identifying a compound that interacts with the aforementioned polypeptide and inhibits or promotes the function thereof, and/or a compound that interacts with the aforementioned polynucleotide and inhibits or promotes the expression thereof, wherein the method includes contacting the compound with the polypeptide or the polynucleotide under conditions where the interaction of the compound with the polypeptide or the interaction of the compound with the polynucleotide are allowed, and determining whether the compound interacts with the polypeptide or the polynucleotide and inhibits or promotes the function of the polypeptide or the expression of the polynucleotide by detecting presence, absence, or variation of a signal which results from the interaction of the compound with the polypeptide or the interaction of the compound with the polynucleotide.

In addition, one further aspect of the present invention is a method for identifying a compound that interacts with the aforementioned polypeptides and inhibits or promotes the function thereof, and/or a compound that interacts with the polynucleotides and inhibits or promotes the expression thereof, wherein the method includes contacting the compound with the transformant, and determining whether the compound inhibits or promotes the expression or function of the polypeptide by using a system that uses a signal and/or a marker being capable of detecting presence or absence of the expression or the function of the polypeptide to detect presence, absence, or variation of the signal and/or marker.

In addition, one further aspect of the present invention is a method for identifying a compound that inhibits or promotes the binding of the polypeptide having the amino acid sequence set forth in SEQ ID NO: 1 in the Sequence Listing, to the NMDA receptor, wherein the method includes using at least one selected from the group consisting of the aforementioned polypeptides, polynucleotides, recombinant vectors, transformants, and antibodies.

In addition, one further aspect of the present invention is a method for identifying a compound that inhibits or promotes the interaction of the polypeptide having the amino acid sequence set forth in SEQ ID NO: 1 in the Sequence Listing, with the polypeptide having the amino acid sequence set forth in SEQ ID NO: 3 in the Sequence Listing, wherein the method includes using at least one selected from the group consisting of the aforementioned polypeptides, polynucleotides, recombinant vectors, transformants, and antibodies.

In addition, one aspect of the present invention is a compound that has been identified by any of the aforementioned methods.

Furthermore, one additional aspect of the present invention is a compound that interacts with the aforementioned polypeptide and inhibits or promotes the function thereof.

In addition, one aspect of the present invention is a compound that interacts with the aforementioned polypeptide and inhibits or promotes the interaction of the polypeptide with the polypeptide having the amino acid sequence set forth in SEQ ID NO: 1 in the Sequence Listing.

In addition, one aspect of the present invention is a compound that interacts with the aforementioned polypeptide and inhibits or promotes the amplification of the NMDA receptor mediated signal transduction in the presence of the polypeptide and the polypeptide having the amino acid sequence set forth in SEQ ID NO: 1 in the Sequence Listing.

Furthermore, one additional aspect of the present invention is a compound that interacts with the aforementioned polynucleotide and inhibits or promotes the expression thereof.

In addition, one further aspect of the present invention is a pharmaceutical composition comprising an effective dose of at least one selected from the group consisting of the aforementioned polypeptides, peptides, polynucleotides, recombinant vectors, transformants, antibodies, compounds, controlling agents, inhibiting agents, and promoting agents.

In addition, one further aspect of the present invention is an agent for preventing, treating, or improving a disease caused by an anomaly in the NMDA receptor mediated signal transduction, wherein the agent includes an effective dose of at least one selected from the group consisting of the aforementioned polypeptides, peptides, polynucleotides, recombinant vectors, transformants, antibodies, compounds, controlling agents, inhibiting agents, and promoting agents.

In addition, one aspect of the present invention is an agent for preventing, treating, or improving a disease caused by an anomaly in memory recall, wherein the agent includes an effective dose of at least one selected from the group consisting of the aforementioned polypeptides, peptides, polynucleotides, recombinant vectors, transformants, antibodies, compounds, controlling agents, inhibiting agents, and promoting agents.

In addition, one aspect of the present invention is an agent for preventing, treating, or improving a neurodegenerative disease, wherein the agent includes an effective dose of at least one selected from the group consisting of the aforementioned polypeptides, peptides, polynucleotides, recombinant vectors, transformants, antibodies, compounds, controlling agents, inhibiting agents, and promoting agents.

In addition, one aspect of the present invention is an agent for preventing, treating, or improving Alzheimer's disease, wherein the agent includes an effective dose of at least one selected from the group consisting of the aforementioned polypeptides, peptides, polynucleotides, recombinant vectors, transformants, antibodies, compounds, controlling agents, inhibiting agents, and promoting agents.

In addition, one further aspect of the present invention is a method for preventing, treating, or improving a disease caused by an anomaly in the NMDA receptor mediated signal transduction, wherein the method includes administering at least one selected from the group consisting of the aforementioned polypeptides, peptides, polynucleotides, recombinant vectors, transformants, antibodies, compounds, controlling agents, inhibiting agents, and promoting agents.

In addition, one further aspect of the present invention is a method for preventing, treating or improving a disease caused by an anomaly in memory recall, wherein the method includes administering at least one selected from the group consisting of the aforementioned polypeptides, peptides, polynucleotides, recombinant vectors, transformants, antibodies, compounds, controlling agents, inhibiting agents, and promoting agents.

In addition, one further aspect of the present invention is a method for preventing, treating, or improving a neurodegenerative disease, wherein the method includes administering at least one selected from the group consisting of the aforementioned polypeptides, peptides, polynucleotides, recombinant vectors, transformants, antibodies, compounds, controlling agents, inhibiting agents, and promoting agents.

In addition, one further aspect of the present invention is a method for preventing, treating, or improving Alzheimer's disease, wherein the method includes administering at least one selected from the group consisting of the aforementioned polypeptides, peptides, polynucleotides, recombinant vectors, transformants, antibodies, compounds, controlling agents, inhibiting agents, and promoting agents.

Furthermore, one additional aspect of the present invention is a method for quantitatively or qualitatively measuring the aforementioned polypeptides or polynucleotides.

Furthermore, one aspect of the present invention is a reagent kit containing at least one selected from the group consisting of the aforementioned polypeptides, peptides, polynucleotides, recombinant vectors, transformants, and antibodies.

### BRIEF DESCRIPTION OF THE DRAWINGS

Fig. 1 is a schematic drawing comparing the characteristics of the amino acid sequences of a novel PSD (referred to as Protein-X) and PSD-95.
Fig. 2 is an electropherogram obtained when a 6×His-tag fusion protein of the novel PSD (Protein-X) was expressed in *Escherichia coli* cultured at 37°C.
Fig. 3 is an electropherogram of the 6×His-tag fusion protein of the novel PSD (Protein-X), showing that the amount expressed in the soluble fraction of *Escherichia coli* cultured at 37°C is low.
Fig. 4 is an electropherogram showing that the 6×His-tag fusion protein of the novel PSD (Protein-X) is expressed in the soluble fraction of *Escherichia coli* cultured at 25°C.
Fig. 5 shows a result of Western-blotting, demonstrating the expression and the expressed amount of the 6×His-tag fusion protein of the novel PSD (Protein-X) or of a PDZ domain deletion mutant thereof.
Fig. 6 shows a result of Western-blotting, demonstrating the expression of a GST fusion protein of an NMDA receptor/2B subunit.
Fig. 7 shows a result of Western-blotting, demonstrating the expression and the expressed amount of the GST fusion protein of the NMDA receptor/2B subunit as well as the expression and the expressed amount of GST.
Fig. 8 is a result obtained by the Overlay method, showing that the novel PSD (Protein-X) binds to a NMDA receptor/2B subunit, whereas the PDZ domain deletion mutant of the novel PSD (Protein-X) does not.
Fig. 9 shows that the novel PSD (Protein-X) and the PDZ domain deletion mutant thereof do not bind to GST.
Fig. 10 is an electropherogram obtained when the 6×His-tag fusion protein of the PDZ domain deletion mutant of the novel PSD (Protein-X) was expressed in *Escherichia coli* cultured at 37°C.
Fig. 11 is an electropherogram showing that the 6×His-tag fusion protein of the PDZ domain deletion mutant of the novel PSD (Protein-X) is expressed in the soluble fraction of *Escherichia coli* cultured at 37°C.
Fig. 12 is an electropherogram showing the expression of the GST fusion protein of the NMDA receptor/2B subunit.
Fig. 13 is a restriction map of a vector carrying a novel PSD (Protein-X) gene.
Fig. 14 is a restriction map of a vector carrying an NMDA receptor/2B subunit gene.
Fig. 15 is a restriction map of a vector carrying an NMDA receptor I gene.
Fig. 16 is a restriction map of a vector carrying a PSD-95 gene.
Fig. 17 shows that an electrical response generated by an NMDA receptor stimulation is enhanced by the novel PSD (Protein-X).
Fig. 18A is an electropherogram showing that the induction of the expression of a His-tag fused human PJ01087 protein (can be referred to as Protein-Y) in *Escherichia coli* was demonstrated by Western-blotting using an anti-His-tag antibody. In the figure, the arrow indicates the human PJ01087 protein (Protein-Y).
Fig. 18B is an electropherogram showing that the induction of the expression of a GST fused human PJ01087 protein (can be referred to as Protein-Y) in *Escherichia coli*, was demonstrated by Western-blotting using an anti-GST antibody. In the figure, the arrow indicates the human PJ01087 protein (Protein-Y).
Fig. 19 is a restriction map of a vector carrying a human PJ01087 gene (SEQ ID NO: 6 in the Sequence Listing).
Fig. 20 shows that the human PJ01087 protein (Protein-Y) further intensifies the enhancement effect by the novel PSD (Protein-X) in the electrical response generated by the NMDA receptor stimulation, but does not intensify the enhancement effect by PSD-95.
Fig. 21 shows that the enhancement, by the novel PSD (Protein-X), of the magnitude of variation in the electrical current generated by the NMDA receptor stimulation is further intensified by the human PJ01087 protein (Protein-Y).
Fig. 22 shows a result obtained by mixing the His-tag fused human PJ01087 protein (Protein-Y) with the GST fused novel PSD (Protein-X) (hj02537), followed by immunoprecipitating them with the anti-GST antibody, and finally detecting the immunoprecipitated proteins with the anti-GST antibody.
Fig. 23 shows a result obtained by mixing the His-tag fused human PJ01087 protein (Protein-Y) and the GST fused novel PSD (Protein-X) (hj02537), followed by immunoprecipitating them with the anti-GST antibody, and finally detecting the immunoprecipitated proteins with the anti-His-tag antibody.

### DETAILED DESCRIPTION OF THE INVENTION

The present invention claims priority from Japanese Patent Application Numbers 2001-354678, 2002-46786, and 2002-229863, which are incorporated herein by reference.

Technical and scientific terms used in the present specification, unless separately defined, have meanings that are normally understood by those skilled in the art to which the present invention belongs. In the present specification, reference is made to a variety of methods known to those skilled in the art. Data from publications and the like that disclose such cited well-known methods are completely incorporated herein, in their entirety, by reference.

Hereinafter, for the present invention, a mode of embodiment of the invention is described in more detail. The following detailed description is illustrative and merely explanatory, and does not limit the present invention in any way.

### Novel PSD and protein interacting with the PSD

The present invention provides a novel human PSD and a protein interacting with the PSD, as well as the genes coding for each of these.

The novel human PSD is a protein coded by clone hj02537, which was selected from a cDNA library (constructed by human brain mRNA and covered relatively long length DNA), as a cloned gene that has a PDZ domain, a SH3 domain and a GK domain. This protein was obtained as expressed product of *Escherichia coli* that was transformed with an expression plasmid carrying the hj02537 gene.

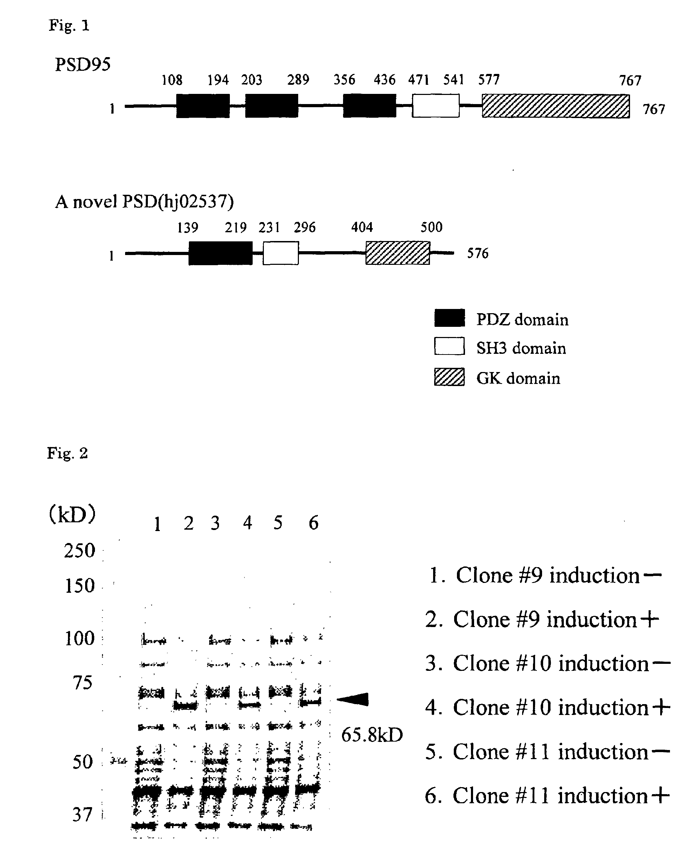

Hereinafter, the human PSD of the present invention may be referred to as Protein-X, and the gene coding for the PSD may be referred to as the Protein-X gene. The Protein-X gene comprises 4941 bps (SEQ ID NO: 4 in the Sequence Listing), which contains the full length of an open reading frame (ORF) including 1731 bps; the gene product thereof comprising 576 amino acid residues (SEQ ID NO: 1 in the Sequence Listing). Similarly to the known PSD, the Protein-X possesses a PDZ domain from isoleucine (Ile) 139 to glycine (Gly) 219, an SH3 domain from methionine (Met) 231 to arginine (Arg) 296, and a GK domain from threonine (Thr) 404 to asparagine (Asn) 500, in the amino acid sequence thereof. From the foregoing, Protein-X was inferred to be a homolog of DLG-like PDZ proteins that include PSD-95 (refer to Fig. 1). Therefore, Protein-X is thought to have similar activities to PSD-95, so that it is anticipated to exist in the vicinity of membrane receptors and ion channels to form an apparatus (which means a functional structure); to participate in the control of neurotransmitter release and signal transfer between cells.

Indeed, similarly to PSD-95, Protein-X can bind to an NMDA receptor/2B subunit via the PDZ domain; and furthermore, it can exhibit an amplification activity greater than that of PSD-95, in response to a signal resulting from the stimulation of the NMDA receptor by a ligand. That is to say, Protein-X is thought to form a complex with an NMDA receptor and enhance the NMDA receptor mediated signal transduction. However, Protein-X and PSD-95 carry different numbers of PDZ domains: three in PSD-95, and one in Protein-X. Thus, there is a possibility that Protein-X carries a receptor signal different from that of PSD-95. Alternately, Protein-X can probably support for PSD-95-like signal transduction or exhibit binding selectivity to other receptors and the like due to the number of PDZ domains.

In addition, the present invention provides a polypeptide in which the 85 amino acids on the N-terminal side of Protein-X have been deleted (SEQ ID NO: 2 in the Sequence Listing). This polypeptide is encoded by a gene clone pj02677 obtained from a cDNA library (constructed by human brain hippocampus mRNA and covered relatively long length DNA). The pj02677 is a polynucleotide comprising 4370 bases (SEQ ID NO: 5 in the Sequence Listing), which codes for a polypeptide comprising 491 amino acids (SEQ ID NO: 2 in the Sequence Listing). The amino acid sequence of the polypeptide encoded by the pj02677 is identical to that of Protein-X with the exception of the deletion of the 85 amino acid residues on the N-terminal side. Therefore, it possesses one PDZ domain, one SH3 domain, and one GK domain in the amino acid sequence thereof; this fact indicate that it may bind to a NMDA receptor/2B subunit in a similar manner as Protein-X does and may exhibit a similar biological activity as that of Protein-X. The expression of pj02677 was observed extensively in various organs such as the brain (including cerebellum and the like), the heart, the liver, the skeletal muscle, the kidneys, the pancreas, the spleen, and the spinal cord. As pj02677 is believed to result from the aforementioned hj02537 with a deletion of the nucleotide sequence from the 5' end side thereof, the Protein-X gene is also probably expressed in these organs.

Meanwhile, the protein that interacts with Protein-X is encoded by clone PJ01087, which was extracted from the cDNA analysis information database (containing information of cDNA derived from human brain almost completely mRNA) of the Kazusa DNA Research Institute, as a gene having homology of 96% with the rat PSD95/SAP90-associated protein 3 (SAPAP-3) (GenBank: Accession No: U67139). This protein was obtained as expressed product of *Escherichia coli* which was transformed with a plasmid carrying the PJ01087 gene. Hereinafter, this protein is referred to as the PJ01087 protein, and the gene that codes for the protein is referred to as the PJ01087 gene. In addition, the PJ01087 protein can be referred to as Protein-Y. The PJ01087 gene comprises 3705 bps (SEQ ID NO: 6 in the Sequence Listing), which contains the full length of the ORF comprising 2940 bps; the gene product thereof comprises 979 amino acid residues (SEQ ID NO: 3 in the Sequence Listing). In addition, it was revealed that Protein-Y is expressed specifically in the brain, by reverse transcription polymerase chain reaction (RT-PCR) and enzyme-linked immunosorbent assay (ELISA).

Protein-Y markedly amplified the signal from the NMDA receptor in the presence of Protein-X. The signal amplification caused by Protein-Y and Protein-X was considerably greater than the amplification caused by Protein-X alone. On the other hand, the signal amplification by Protein-Y was not observed in the presence of PSD-95. From the foregoing, it was revealed that Protein-Y interacts with Protein-X, and that the signal transduction resulting from the stimulation of the NMDA receptor by a ligand is markedly amplified by the interaction.

In the present specification, Protein-Y interacting with Protein-X means that both proteins mutually exert an activity in a certain scheme, resulting in enhancement of the function of each protein. The function of each protein can be exemplified by the promotion of physiological functions of membrane receptors and ion channels (such as the promotion of the stabilization of the membrane receptors and the acceleration of receptor mediated signal transduction). The membrane receptor and the ion channel can be exemplified by NMDA receptor. The interaction schemes can be exemplified by binding, temporary binding, or the formation of a complex via another substance: however it is not limited thereto. Since the binding of Protein-Y to Protein-X was not observed in immunoprecipitation analyses, the interaction between the two proteins may not be accomplished through binding, but through some alternative scheme.

These findings suggest that a signal of a membrane receptor, for example, a signal of an NMDA receptor, can be amplified by the overexpression of Protein-X or by promoting the biological activity thereof (for example, the interaction with another protein or a guanylate kinase activity). In addition, it is suggested that an abnormal signal from a membrane receptor, for example, an abnormal signal from of an NMDA receptor, can be normalized by inhibiting the expression of Protein-X or by inhibiting the biological activity thereof (for example, the interaction with another protein or a guanylate kinase activity). Similarly, a signal from a membrane receptor can be amplified by the overexpression of Protein-Y or by promoting the biological activity thereof. In addition, an abnormal signal of a membrane receptor can be normalized by inhibiting the expression of Protein-Y or by inhibiting the biological activity thereof. That is to say, the signal transduction, and the like, of a membrane receptor can be controlled by inhibiting or promoting the interaction of Protein-X with the membrane receptor or with a protein that participates in the receptor mediated signal transduction. Herein, "control" means "inhibition" or "promotion." In addition, "promotion" includes the common meanings of "enhancement" and "amplification." For instance, the NMDA receptor mediated signal transduction and the like can be controlled by inhibiting or promoting the interaction of Protein-X with the NMDA receptor, or inhibiting or promoting the interaction of Protein-X with Protein-Y. Therefore, Protein-X and Protein-Y, as well as an inhibitor and a promoting agent for the expression thereof or for the biological activity thereof, can be applied to the prevention, improvement, or treatment of a disease: caused by an anomaly in Protein-X; caused by an anomaly in Protein-Y; caused by an anomaly in both proteins; or caused by an anomaly in the interaction between the two proteins. That is to say, they can be used for a prophylactic agent, an improving agent, or a therapeutic agent of these diseases, as well as in a prevention method, an improvement method, or a treatment method. A disease based on an anomaly of a membrane receptor mediated signal transduction (where Protein-X and/or Protein-Y is implicated) can be cited as such a disease. More specifically, it can be exemplified by neurodegenerative diseases such as Alzheimer's disease, Parkinson's disease, and polyglutamine disease; however it is not limited thereto.

### Polypeptides or Peptides

In the present specification, a longer chain peptide (having any number of peptides containing two or more amino acids that bind to one another through a peptide bond or a modified peptide bond), such as a protein, is called a polypeptide; a shorter chain peptide (sometimes also referred to as oligopeptide or oligomer) is simply called a peptide. In the present specification, an amino acid can sometimes be represented by three letters.

The polypeptide according to the present invention is a gene product of the Protein-X gene; it can be obtained by expressing the gene in a cell such as *Escherichia coli*. In addition, it can be a polypeptide obtained by chemical synthesis based on the amino acid sequence of the polypeptide of interest; it can also be a polypeptide derived from cells or from any tissue where these cells are exist. The polypeptide can be a polypeptide comprising an amino acid sequence that is identical or substantially identical to the amino acid sequence set forth in SEQ ID NO: 1 or 2 in the Sequence Listing. As the amino acid sequence that is substantially identical to the amino acid sequences set forth in SEQ ID NO: 1 or 2 in the Sequence Listing, it is not limited as long as the polypeptide can at least bind to an NMDA receptor/2B subunit, and can, for example be a polypeptide that contains a polypeptide having the amino acid sequences set forth in SEQ ID NO: 1 or 2 in the Sequence Listing. Alternatively, it can be a polypeptide that has an amino acid sequence homologus to the polypeptide having the amino acid sequences set forth in SEQ ID NO: 1 or 2 in the Sequence Listing, with 70% or greater, preferably 80% or greater, more preferably 90% or greater, and even more preferably 95% or greater, and that is capable of binding to an NMDA receptor/2B subunit. A polypeptide further having a guanylate kinase activity is more preferred. In addition, a polypeptide possessing one PDZ domain is further preferable.

Binding to an NMDA receptor/2B subunit can be measured by any well-known method such as the Overlay method, as described in the examples below. In addition, the guanylate kinase activity can be measured by any well-known method. Techniques for determining the amino acid sequence homology are well-known, for example, a method that directly determines the amino acid sequence, or a method that determines the nucleotide sequence of the cDNA and then infers the amino acid sequence encoded thereby. Needless to say, homologous gene products of animal species other than human are also included in the present invention. In addition, based on a polypeptide thus specified and using an indicator (for example, binding to the NMDA receptor/2B subunit and/or guanylate kinase activity), a polypeptide can be provided, which includes an amino acid sequence having a mutation, which can be a deletion, a substitution, an addition, or an insertion of one or more amino acids, for instance one to 100, preferably one to 30, more preferably one to 20, even more preferably one to 10, and particularly preferably one to several amino acids. These polypeptides are also included in the polypeptides comprising an amino acid sequence that is substantially identical to the amino acid sequences set forth in SEQ ID NO: 1 or 2 in the Sequence Listing. As such a polypeptide, for example, the polypeptide having the amino acid sequence set forth in SEQ ID NO: 2 in the Sequence Listing is a polypeptide comprising an amino acid sequence that is substantially identical to the amino acid sequence set forth in SEQ ID NO: 1 in the Sequence Listing. The aforementioned polypeptides having a mutation can be those that exist naturally, or those into which a mutation has been introduced. In addition, the present invention can also include a polypeptide that comprises an amino acid sequence substantially identical to the amino acid sequence set forth in SEQ ID NO: 1 in the Sequence Listing, and does not interact with the polypeptide having the amino acid sequence set forth in SEQ ID NO: 3 in the Sequence Listing. Such a polypeptide is inferred to inhibit the interaction of Protein-X with Protein-Y by competing with Protein-X. Thus, such a polypeptide probably inhibits the NMDA receptor mediated signal transduction.

Another polypeptide according to the present invention is a gene product of the Protein-Y gene; it can be obtained by expressing the gene in a cell such as *Escherichia coli*. In addition, it can be a polypeptide obtained by chemical synthesis based on the amino acid sequence of the polypeptide of interest; it can also be a polypeptide derived from cells or from any tissue where these cells are present. The polypeptide can be a polypeptide comprising an amino acid sequence that is identical or substantially identical to the amino acid sequence set forth in SEQ ID NO: 3 in the Sequence Listing. As the amino acid sequence that is substantially identical to the amino acid sequence set forth in SEQ ID NO: 3 in the Sequence Listing, it is not limited as long as the polypeptide can at least interact with the polypeptide having the amino acid sequence set forth in SEQ ID NO: 1 in the Sequence Listing, and can, for instance be a polypeptide that contains a polypeptide having the amino acid sequence set forth in SEQ ID NO: 3 in the Sequence Listing. Alternatively, it can be a polypeptide that has an amino acid sequence homologous to the polypeptide having the amino acid sequence set forth in SEQ ID NO: 3 in the Sequence Listing, with 70% or greater, preferably 80% or greater, more preferably 90% or greater, and even more preferably 95% or greater, and that is capable interacting with the polypeptide having the amino acid sequence set forth in SEQ ID NO: 1 in the Sequence Listing. In addition, it can also be a polypeptide that amplifies NMDA receptor mediated signal transduction in the presence of the polypeptide having the amino acid sequence set forth in SEQ ID NO: 1 in the Sequence Listing. In this case, the amino acid sequence of SAPAP-3 is not included in the present invention.

In addition, in terms of the concept of a module (the structural unit determined by the tertiary structure of the protein, which often works also as a functional unit), a polypeptide comprising at least five consecutive amino acids among the amino acid sequence set forth in SEQ ID NO: 3 in the Sequence Listing, can also be used for the interacting peptide. The number of amino acid residues of the module is known to be 15 on average; however considering its function of being capable of working as an antigen or the like, the five amino acid residues may form a sufficient functional unit. The interaction with the polypeptide having the amino acid sequence set forth in SEQ ID NO: 1 in the Sequence Listing, can be assayed, for example, by co-expressing the polypeptide, a polypeptide that is the object of the assay, and the NMDA receptor together in a cell, followed by measuring the intracellular signal transduction resulting from the stimulation of the NMDA receptor by a ligand; the mesurement can performed by using a well-known method (see Example 4).

Techniques for determining the amino acid sequence homology are well-known. For example, a method that directly determines the amino acid sequence, or a method that determines the nucleotide sequence of the cDNA and then infers the amino acid sequence encoded thereby can be used. Based on a polypeptide thus specified and using an indicator (for example, the interaction with the polypeptide having the amino acid sequence set forth in SEQ ID NO: 3 in the Sequence Listing; the promotion of the NMDA receptor mediated signal transduction; the amplification of the NMDA receptor mediated signal transduction in the presence of the polypeptide having the amino acid sequence set forth in SEQ ID NO: 1 in the Sequence Listing), a polypeptide can be provided. The polypeptide can include an amino acid sequence having a mutation, which can be a deletion, a substitution, an addition, or an insertion of one or more amino acids, for example, one to 100, preferably one to 30, more preferably one to 20, even more preferably one to 10, and particularly preferably one to several amino acids. These polypeptides are also included in the polypeptides comprising an amino acid sequence that is substantially identical to the amino acid sequence set forth in SEQ ID NO: 3 in the Sequence Listing. In this case, the amino acid sequence of SAPAP-3 is not included in the present invention. The peptides having a mutation can be those that exist naturally, or those into which a mutation has been introduced.

Methods for introducing a mutation such as a deletion, a substitution, an addition, and/or an insertion are well-known. For instance, site-directed mutagenesis, homologous gene recombination, primer extension or polymerase chain reaction (PCR) can be used alone or in appropriate combination, according to the methods described in manuals such as Sambrook et al. Eds., Molecular Cloning: A Laboratory Manual, 2nd Ed., Cold Spring Harbor Press, 1989; Masami Muramatsu Ed., Laboratory Manual Genetic Engineering, Maruzen Co. Ltd., 1988; Ehrlich, H.E. Ed., PCR Technology - Principles and Applications for DNA Amplification, Stockton Press, 1989. These methods may be modified for use. For instance, Ulmer's technique (Science, 1983, Vol. 219, p.666 et seq.) can be used. Insuring that the fundamental characteristics (physical properties, functions, biological activity, or immunological activity and the like) of these polypeptides are not changed by the introduction of such mutation(s), as reciprocal substitution among, for example, homologous amino acids (polar amino acids, nonpolar amino acids, hydrophobic amino acids, hydrophilic amino acids, positively charged amino acids, negatively charged amino acids, aromatic amino acids, and the like) is readily inferred. Furthermore, these polypeptides can be altered to the extent that no significant functional alteration is involved, such as modifying their constituent amino group or carboxyl group and the like.

The polypeptide comprising an amino acid sequence that is substantially identical to the amino acid sequence set forth in SEQ ID NO: 1 in the Sequence Listing, can be a polypeptide with a function or an activity that is promoted or decreased compared to the polypeptide having the amino acid sequence set forth in SEQ ID NO: 1 in the Sequence Listing. For instance, the ability thereof to form a complex with an NMDA receptor, the guanylate kinase activity thereof, and/or the extent of the interaction thereof with the polypeptide having the amino acid sequence set forth in SEQ ID NO: 3 in the Sequence Listing, can be different. More specifically, for instance, the magnitude of the promotion of the NMDA receptor mediated signal transduction can be different, the function thereof can be additionally promoted, or the function thereof can be decreased.

The polypeptide comprising an amino acid sequence that is substantially identical to the amino acid sequence set forth in SEQ ID NO: 3 in the Sequence Listing, can be a polypeptide with a function or an activity that is promoted or more decreased compared to the polypeptide having the amino acid sequence set forth in SEQ ID NO: 3 in the Sequence Listing. For instance, the extent of the interaction thereof with the polypeptide having the amino acid sequence set forth in SEQ ID NO: 1 in the Sequence Listing, can be different, the interaction can be promoted, or the interaction can be decreased. More specifically, for instance, the magnitude of the promotion of the NMDA receptor mediated signal transduction can be different, the function thereof can be promoted, or the function thereof can be decreased.

The present invention also includes the polypeptides to which a different type of protein or substance has been ligated, for example, a carrier and the like. For instance, a different type of protein or peptide (for example, alkaline phosphatase, β-galactosidase, glutathione S-transferase (GST), or an Fc fragment of an immunoglobulin such as immunoglobulin G (IgG)) can be ligated to the N-terminus or the C-terminus thereof, directly or indirectly via a linker peptide, by using well-known methods such as gene engineering techniques, in order to facilitate the detection or purification of the polypeptide and the like of the present invention, or in order to add a different function,

In addition, the present invention includes a polypeptide or a peptide that has a partial sequence of the polypeptide having the amino acid sequence set forth in SEQ ID NO: 1 or 3 in the Sequence Listing. The polypeptide or the peptide that has the partial sequence comprises consecutive amino acids of 5 or more, preferably 8 or more, more preferably 12 or more, further preferably 15 amino acids or more, as the minimum unit thereof. For example, a polypeptide or a peptide comprising a minimum active unit (region or domain) related to the function of Protein-X or Protein-Y is also provided in the present invention. The polypeptide or the peptide that has the aforementioned partial sequence can be used, for instance, to inhibit the function of Protein-X and/or Protein-Y. More specifically, for example, the peptide being capable of binding to an NMDA receptor/2B subunit can inhibit the binding of Protein-X to the NMDA receptor/2B subunit. In addition, for example, a peptide that is a partial peptide of Protein-X, or Protein-Y, and contains a region that is involved in the interaction of both proteins, can inhibit the interaction. Alternatively, it can inhibit the amplification of the NMDA receptor mediated signal transduction that is generated in the presence of Protein-Y and Protein-X. Therefore, such a peptide can inhibit the promotion of the NMDA receptor mediated signal transduction. In addition, the polypeptide or the peptide that has the aforementioned partial sequence is useful as a reagent in, for example, identifying a substance that controls the biological activity (binding to the NMDA receptor/2B subunit, guanylate kinase activity, and/or interaction with Protein-Y) of Protein-X or a polypeptide that has the same biological activity as Protein-X. In addition, it is useful as a reagent in, for example, identifying a substance that controls the biological activity (such as interaction with Protein-X) of Protein-Y or a polypeptide that has the same biological activity as Protein-Y. If it is a peptide recognized immunologically, such as an epitope peptide, it can be used to produce antibodies specific to Protein-X or Protein-Y, by using it singly as an antigen, or by linking it to a carrier (for example, keyhole limpet hemocyanin or egg-white albumin), as described below.

### Polynucleotides

In one aspect of the present invention, the polynucleotide of the present invention means a polynucleotide containing a nucleotide sequence coding for each of the polypeptides or peptides of the present invention, or a complementary nucleotide sequence thereof. For instance, it can be a polynucleotide comprising the nucleotide sequence coding for the polypeptide having the amino acid sequence set forth in SEQ ID NO: 1, 2, or 3 in the Sequence Listing, or a complementary nucleotide sequence thereof. Preferably, it is a polynucleotide comprising the nucleotide sequence set forth in SEQ ID NO: 4, 5, or 6 in the Sequence Listing, or a complementary nucleotide sequence thereof. In the present invention, polynucleotides comprising complementary nucleotide sequences can be referred to as complementary strands.

In another aspect, the present invention includes polynucleotides that hybridize under stringent conditions to the region corresponding to the aforementioned polynucleotide, preferably the polynucleotide comprising the nucleotide sequence set forth in SEQ ID NO: 4, 5, or 6 in the Sequence Listing, or the complementary nucleotide sequence thereof. Hybridization conditions can follow the publications, for instance, Sambrook et al. eds. Molecular Cloning: A Laboratory Manual, Second Edition, Cold-Spring Harbor Laboratory Press, 1989, and the like. These polynucleotides do not necessarily need to be complementary sequences as far as they hybridize to the target polynucleotide, preferably the polynucleotide comprising the nucleotide sequence set forth in SEQ ID NO: 4, 5, or 6 in the Sequence Listing, or to the complementary nucleotide sequence thereof. For instance, it can be a polynucleotide having a nucleotide sequence homologous to the polynucleotide comprising the nucleotide sequence set forth in SEQ ID NO: 4, 5, or 6 in the Sequence Listing, or the complementary sequence thereof, with at least 70% or greater, preferably 80% or greater, more preferably 90% or greater, and even more preferably 95% or greater. In this case, the nucleotide sequence of SAPAP-3 is not included in the present invention. In addition, the polynucleotides of the present invention include polynucleotides or oligonucleotides having a nucleotide sequence of consecutive nucleotides of 10 or more, preferably 15 or more, and more preferably 20 or more, which correspond to a specified region of the nucleotide sequence of the aforementioned polynucleotide, or the complementary nucleotide sequence thereof.

These polynucleotides provide genetic information useful in manufacturing the polypeptides and the like of the present invention, or can also be used as reagents or standards related to nucleic acids. For instance, they can be used as nucleic acids coding for Protein-X or Protein-Y, for example as probes or primers for detecting the gene or the mRNA thereof, or as antisense oligonucleotides to control the gene expression, and the like. In this sense, the polynucleotides and the oligonucleotides of the present invention include those corresponding to translated-regions as well as those corresponding to untranslated-regions. Here, the screening for the polynucleotides coding for the polypeptides of the present invention can be performed, for example, by verifying the expressed protein using a well-known protein expression system and taking the biological activity thereof as an indicator. The biological activities to be used as indicators are exemplified by binding to an NMDA receptor/2B subunit; by the guanylate kinase activity;or by interaction with the polypeptide having the amino acid sequence set forth in SEQ ID NO: 1 or 3 in the Sequence Listing. The interaction can be evaluated, for instance by measuring the promotion of the NMDA receptor mediated signal transduction. More specifically, measurement can be performed by taking as an indicator the function of amplifying the NMDA receptor mediated signal transduction in the presence of two types of polypeptides having the respective amino acid sequences set forth in SEQ ID NO: 1 and 3 in the Sequence Listing. As examples of well-known protein expression systems, cell-free protein expression systems that use the technology of the ribosome system derived from embryo or rabbit reticulocytes and the like (Nature, 1957, Vol. 179, p.160-161) can be cited.

### Recombinant Vectors

Recombinant vectors can be obtained by transfecting the polynucleotides of the present invention into adequate vector DNAs. The vector DNAs to be used can be selected appropriately depending on the type of host and purpose of use. In addition to vector DNAs extracted from those naturally present, vector DNAs can be those that lack a part of the DNA, other than that required for multiplication. Examples include: chromosome, episome and virus derived vectors, such as bacterial plasmid-derived, bacteriophage-derived, transposon-derived, yeast episome-derived, insertion element-derived, and yeast chromosomal element-derived; virus-derived vectors such as those derived from baculovirus, papovavirus, SV40, vaccinia virus, adenovirus, fowlpox virus, pseudorabies virus and retrovirus; as well as vectors combining the same, such as vectors derived from plasmid and bacteriophage genetic elements, including cosmid and phagemid and the like. In addition, expression vectors, cloning vectors, and the like can be used depending on the purpose.

A recombinant vector has, as components, the target gene sequence and gene sequences that carry information regarding replication and control (such as a promoter, ribosome binding site, terminator, signal sequence and enhancer), and is produced by combining them by using the well-known methods. As the method for transfecting the polynucleotides of the present invention into the previously mentioned vector DNAs, well-known methods can be adopted. For instance, a method can be used, which comprises: selecting an adequate restriction enzyme, treating DNA to cleave it at specific sites, mixing the DNA with vector DNA that has been treated in the same way, and re-ligating with a ligase. Otherwise, a desired recombinant vector can also be obtained by ligating an adequate linker to the target polynucleotide, and inserting it into the multicloning site of a vector that is suitable for the purpose.

### Transformants

Transformants can be obtained by introducing the vector DNA containing aforementioned polynucleotide into a host being well-known to one skilled in the art; the introducing methods are well-known to one skilled in the art. The vector DNA introduced into a host can be one type of vector DNA, or two or more types of vector DNA. For instance, it can be the vector DNA containing the polynucleotide set forth in SEQ ID NO: 4 in the Sequence Listing; the vector DNA containing the polynucleotide set forth in SEQ ID NO: 6; or both vector DNAs. As examples of host, *Escherichia coli*, yeast, *Bacillus subtilis*, insect cells, or animal cells and the like can be cited. As preferable systems when carrying out introduction of the gene, the method of integration into chromosomes can be cited if gene stability is to be considered; however, for simplicity, auto-replicating systems that use extranuclear genes can be employed. Introduction of a vector DNA into a host cell can be carried out by a standard method, for instance, that described in Sambrook et al. eds., Molecular Cloning: A Laboratory Manual, Second Edition, Cold-Spring Harbor Laboratory Press, 1989. Concretely, calcium phosphate transfection, DEAE-dextran-mediated transfection, microinjection, cationic lipid-mediated transfection, electroporation, transduction, scrape loading, ballistic introduction, infection, and the like can be cited.

The polypeptides or peptides of the present invention can be provided when an expression vector is used as the vector DNA for use in transformation of the host. The transformant (into which an expression vector DNA containing aforementioned polynucleotide has been introduced) can be cultured under culture conditions and by culture methods that are suitable for each host and well-known to one skilled in the art. Culturing can be performed by referring to the function (for example binding to an NMDA receptor/2B subunit, guanylate kinase activity, or interaction with the polypeptide having the amino acid sequence set forth in SEQ ID NO: 1 or 3 in the Sequence Listing) of the polypeptide or peptide of the present invention that is expressed by the transformant. Alternatively, culturing can be performed by referring to the quantity of polypeptides or peptides generated inside or outside the host; a passage culture or a batch culturing can also be performed by referring to the quantity of transformant in the culture medium.

### Collection and/or purification of the polypeptides and peptides

The collection and/or purification of the polypeptides or peptides of the present invention from the culture media in which the transformant was cultured, can be performed by referring to the function (for example, binding to an NMDA receptor/2B subunit, guanylate kinase activity, or interaction with the polypeptide having the amino acid sequence set forth in SEQ ID NO: 1 or 3 in the Sequence Listing) of the polypeptide or peptide. Fractionation methods based on differences in solubility using ammonium sulfate or alcohol and the like, gel filtration, ion column chromatography, affinity chromatography, and the like can be cited as methods of collection and/or purification, which are used alone or in combination. Preferably, a method can be used, in which polypeptides or peptides are specifically adsorbed and collected by using polyclonal antibodies or monoclonal antibodies that can be prepared against the polypeptides or the peptides based on the information of their amino acid sequences.

### Antibodies

The antibodies can be produced by using the aforementioned polypeptides or peptides as antigens. The antigen can be the polypeptides or the peptides, or a fragment thereof; antigens can be composed of at least 8, preferably at least 10, more preferably at least 12, further preferably 15 or more amino acids. In order to produce antibodies that are specific to the aforementioned polypeptides and/or peptides, it is preferable to use regions comprising amino acid sequences that are unique to the polypeptides or peptides. This region of the amino acid sequence does not necessarily need to be homologous or identical to the amino acid sequences of the polypeptides or peptides. Sites that are exposed to the exterior of the tertiary structure of the protein are preferred. Even if the amino acid sequences at the exposed sites are discontinuous in the primary structure, it suffices as far as they are amino acid sequences that are continuous on the exposed site. There are no particular limits on the antibodies, as long as they immunologically bind or recognize the polypeptides and/or peptides. Whether or not this binding or recognition occurs is determined by the well-known antigen-antibody-binding reaction.

In order to produce an antibody, a well-known method for antibody production can be employed. For example, the antibody can be obtained by administering the antigen to an animal with or without linking such to a carrier, in the presence or absence of an adjuvant, to induce immunity such as a humoral response and/or cell-mediated response. The carrier is not limited in particular, as long as it does not exert a harmful effect by itself on the host and is capable of enhancing antigenicity. Cellulose, polymeric amino acids, albumin, keyhole limpet hemocyanin (KLH), and the like can be given as examples of carriers. Examples of the adjuvant can be Freund complete adjuvant (FCA), Freund incomplete adjuvant (FIA), Ribi (MPL), Ribi (TDM), Ribi (MPL+TDM), *Bordetella pertussis* vaccine, muramyl dipeptide (MDP), aluminum adjuvant (ALUM), and combinations thereof. Mouse, rat, rabbit, goat, horse, and the like are suitable animals for immunization.

Polyclonal antibodies can be obtained from the serum of the animals subjected to the aforementioned immunization means, by any suitable method for collecting antibodies. As a preferable means, the antibodies are obtained by the immune-affinity chromatography method.

Monoclonal antibodies can be produced by collecting antibody-producing cells (for instance, a lymphocyte derived from a spleen or a lymph node) from the animal that was subjected to the aforementioned immunization, followed by the introduction of a well-known transformation technique using a permanently proliferating cell (for example, myeloma strain such as P3X63Ag8 cells.) For example, an antibody-producing cell is fused with a permanently proliferating cell to produce a hybridoma by methods being well-known to one skilled in the art, and then cloning of the hybridoma is performed, followed by selecting hybridoma that produces an antibody recognizing specifically the aforementioned polypeptides and/or peptides. The antibody can be collected from the cultured solution of the hybridoma.

The polyclonal antibody or monoclonal antibodies thus obtained, which can recognize and bind to the aforementioned polypeptides and/or peptides, can be used as a purification antibody, reagent, or labeling marker for the polypeptides or peptides.

Among the polyclonal or monoclonal antibodies that recognize and bind to the polypeptides and/or the peptides, for example, the antibodies that directly bind to Protein-X and inhibit the biological activity thereof (for example, the binding activity to other proteins such as an NMDA receptor, guanylate kinase activity, and/or interaction with Protein-Y) are useful in the elucidation, prevention, improvement, or treatment of various diseases caused by an anomaly in Protein-X and/or the function thereof. In addition, antibodies that directly bind to Protein-Y and inhibit the function thereof (for example at least the interaction with Protein-X) are useful in the elucidation, prevention, improvement, or treatment of various diseases caused by an anomaly in Protein-Y and/or the function thereof. For instance, antibodies that inhibit the promotion of the NMDA receptor mediated signal transduction, wherein the promotion was caused by the interaction of Protein-X with Protein-Y, can be cited as examples of such antibodies. More specifically, antibodies that inhibit the amplification of the NMDA receptor mediated signal transduction in the presence of both Protein-X and Protein-Y can be cited.

### Identification method of Compounds

The polypeptides or peptides of the present invention, the polynucleotides of the invention, the vectors into which these polynucleotides have been transfected, the transformants into which these vectors have been introduced, the protein synthesis system using these, or the antibodies that immunologically recognize these polypeptides and/or the peptides, provide a method that is effective in identifying an inhibiting agent or a promoting agent of the functions of the aforementioned polypeptides or the aforementioned peptides, and/or identifying an inhibiting agent or promoting agent of the expression of the aforementioned polynucleotides, when they are used alone or in combination. The identification method of the present invention can be established using pharmaceutical screening systems being well-known in the art. According to the identification method of the invention, screening for an antagonist by drug design based on the tertiary structure of the aforementioned polypeptide or peptide of the invention, screening for an inhibiting agent or a promoting agent of the expression at the gene level using the protein synthesis system, or screening for a substance recognized by an antibody using an antibody, and the like, is possible.

For example, compounds can be identified which promote or inhibit the activation of Protein-X and of polypeptides comprising substantially the same amino acid sequence as Protein-X, by using polypeptides or peptides derived from Protein-X; selecting conditions that allow for interactions of a test compound with these polypeptides or peptides; bringing these polypeptides or peptides to contact with the compound under these conditions; and detecting the presence, the absence, or the change in a signal generated by the interaction. Compounds can be identified which promote or inhibit the interaction of Protein-X with another protein that binds to the polypeptides or the peptides (for example, an NMDA receptor), by using an assay system that measures the interaction; adding the test compound thereto; and detecting the presence, the absence, or the change in a signal generated by the interaction. The assay system can be established using screening systems being well-known in the art. For instance, the method described below in Example 3 can be used. In addition, when using the guanylate kinase activity of Protein-X as an indicator, compounds that promote or inhibit the guanylate kinase activity can be identified. Measurement of guanylate kinase activity can be carried out according to the well-known methods (Cook, P.F., et al., Biochemistry, 1982, Vol. 21 p. 5794 ff.; Wright, D.E., et al., Proc. Natl. Acad. Sci. USA, 1981, Vol. 78, p. 6048 ff.; Corbin, J.D., et al., Methods in Enzymology, 1974, Vol. 38, p. 287).

In addition, using polypeptides or peptides derived from Protein-Y, compounds can be identified as described above, which inhibit or promote the activity of Protein-Y and of a polypeptide having the same physiological activity as that of Protein-Y. Compounds that inhibit or promote the interaction of Protein-X with Protein-Y can be identified using polypeptides or peptides derived from Protein-X and polypeptides or peptides derived from Protein-Y, by using an assay system that measures the interaction thereof; adding the test compound thereto; and detecting the presence, the absence, or the change in a signal generated by the interaction. Concretely, compounds can be identified which inhibit or further promote the promotion of the NMDA receptor mediated signal transduction, wherein the promotion is caused by the interaction of Protein-X with Protein-Y, Alternatively, for example, compounds can be identified which inhibit or promote the amplification of the NMDA receptor mediated signal transduction in the presence of both Protein-X and Protein-Y. The assay system can be established using screening systems beint well-known in the art. For example, the method described below in Example 6 can be used.

In addition, compounds can be identified which interact with the polynucleotides of the present invention and inhibit or promote the expression thereof, by selecting conditions that allow the interaction of the polynucleotides with the test compound; bringing the polynucleotides to contact with the test compound under the conditions; using a system that uses a signal and/or a marker that can detect the expression of the polynucleotides; and detecting the presence, the absence, or the change of the signal and/or marker. Compounds can be identified which inhibit or promote the expression of these polynucleotides, such as the Protein-X or Protein-Y, by further incorporating the method of using transformants described below.

In addition, compounds can be identified which inhibit or promote the expression of the polynucleotides (such as the Protein-X gene or the Protein-Y gene), by bringing the transformants of the present invention into contact with a test compound or a compound identified above under appropriate conditions; and detecting the presence, the absence, or the change of the expression of the polynucleotides of the present invention (such as the Protein-X gene or the Protein-Y gene). The detection of the presence, the absence, or the change of the expression of the Protein-X gene or the Protein-Y gene can be performed simply by measuring the function of the expressed gene product. For example, the detection of the presence, the absence, or the variation of the expression of the Protein-X gene can be performed by measuring the guanylate kinase activity thereof, as an indicator. Alternatively, in order to detect the presence, the absence, or the change of the expression of the Protein-X gene or the Protein-Y gene, a system well-known in the art (which uses a signal or a marker for detection) can be introduced to detect the presence, the absence, or the change of the signal or marker. Herein, a signal indicates an entity that is directly detected by the physical properties or the chemical properties thereof, and a marker indicates an entity that is detected indirectly taking the physical properties or the chemical properties thereof as indicators. Any substances well-known (such as Luciferase and green fluorescence protein (GFP) and the like) can be used for the signal: any substances well-known (such as a reporter gene (such as the chloramphenicol acetyltransferase (CAT) gene) or a detection tag (such as His-tag)) can be used for the marker. The signals or markers can be ligated to the target gene sequence, and transfected into a vector; the obtained vector can be used to produce a transformant by transforming a host cell therewith. In addition, methods for utilizing and for detecting these signals or markers are well-known to those skilled in the art.

Compounds (that inhibit or promote the expression or function of Protein-Y or Protein-Y) can be identified in an experimental system wherein a fusion protein of Protein-X or Protein-Y with a His-tag is expressed (for example in *Escherichia coli*) as shown in the examples, by adding the test compound to the experimental system and then detecting the His-tag.

Another example is a method that comprises transforming a cell with the Protein-X gene, the Protein-Y gene, and the NMDA receptor gene for co-expression thereof, adding the test compound, and measuring the intracellular signal transduction due to the stimulation of the NMDA receptor by a ligand using a well-known method in the art. Using such a cell in which the aforementioned genes are co-expressed, compounds that inhibit or promote the expression of Protein-X or Protein-Y can be identified, in addition to compounds that inhibit or promote the function of Protein-X or Protein-Y.

The methods and experimental systems given as examples in the foregoing were given for the sake of exemplifying by concrete description thereof. The compound identification methods and the experimental systems used in the methods of the present invention are not restricted thereby.

### A controlling agent of interaction of Protein-Y with Protein-X, and a pharmaceutical composition

In the present invention, it was found that Protein-X binds to an NMDA receptor/2B subunit and amplifies the NMDA receptor mediated signal transduction. Protein-Y also remarkably amplifies the membrane receptor mediated signal transduction (for instance an NMDA receptor mediated signal transduction) in the presence of Protein-X. From the foregoing, it was inferred that, when a membrane receptor/ion channel (for example, an NMDA receptor), is stimulated by a ligand thereof or the like, Protein-Y accumulates in the vicinity of the receptor, and then stabilizes the receptor and/or promotes the receptor mediated signal transduction through an interaction with Protein-X. Protein-X and Protein-Y can be involved in physiological functions such as neurotransmitter release, wherein the function are triggered as a result of the signal transduction mediated by a membrane receptor (for instance of an NMDA receptor) that they are involved in. Therefore, by inhibiting or promoting the interaction of Protein-Y with Protein-X, physiological functions that Protein-Y and Protein-X are involved in (for example, at least the NMDA receptor mediated signal transduction) can be controlled. In addition, NMDA receptor mediated signal transduction can be controlled by inhibiting or promoting the binding of the NMDA receptor to Protein-X.

The present invention thus provides an agent and a method for controlling NMDA receptor mediated signal transduction, wherein the agent and the method utilizes a means for inhibiting or promoting the binding of Protein-X to the NMDA receptor, and/or, a means for inhibiting or promoting the interaction of Protein-X with Protein-Y. Herein, a controlling agent is a collective term to designate an inhibiting agent, an antagonist, or a promoting agent and the like. In addition, a controlling method means an inhibiting method or a promoting method and the like. To promote the binding of Protein-X to an NMDA receptor, for instance, Protein-X itself or the gene thereof can be used. To promote the interaction of Protein-X and/or of Protein-Y, these proteins themselves or the genes thereof can be used. For instance, since the binding of Protein-X to an NMDA receptor or the interaction of Protein-X with Protein-Y is promoted by overexpressing Protein-X, or by overexpressing both Protein-X and Protein-Y, or by promoting the function thereof, the NMDA receptor mediated signal transduction can thereby be promoted. Inhibition of the binding of Protein-X to the NMDA receptor, or inhibition of the interaction between Protein-X and/or of Protein-Y is acceptable pratically, for instance by inhibiting the expression of Protein-X and/or Protein-Y. An abnormal signal of a membrane receptor can be normalized by the inhibition of the expression of the proteins, or the inhibition of the function thereof.

In addition, for instance, a compound identified by the aforementioned method can be cited as a means for inhibiting or promoting the binding of Protein-X to the NMDA receptor, or the interaction of Protein-X with Protein-Y. The compound can be used as an inhibiting agent or a promoting agent of the binding of Protein-X to an NMDA receptor, or as an inhibiting agent or a promoting agent of the interaction of Protein-X with Protein-Y. Furthermore, it can be used as an inhibiting agent or a promoting agent of NMDA receptor mediated signal transduction. More specifically, the compound can be used as an inhibiting agent or a promoting agent of the amplification of NMDA receptor mediated signal transduction in the presence of both Protein-X and Protein-Y of the present invention. A polypeptide or a peptide comprising the minimum activity unit (region or domain) related to the function of the polypeptide of the present invention, and being capable of inhibiting the interaction of Protein-X with Protein-Y can be given as examples of compounds using as inhibitors. Such polypeptides or peptides can be identified, for example, by designing based on the amino acid sequence of Protein-Y, synthesizing by a peptide synthesis method being well-known in the art, and assaying by the aforementioned detection method. In addition, a polypeptide comprising an amino acid sequence that is substantially identical to the amino acid sequence set forth in SEQ ID NO: 1 in the Sequence Listing, and not interacting with the polypeptide having the amino acid sequence set forth in SEQ ID NO: 3 in the Sequence Listing, can be used as an inhibitor. In addition, an antibody that inhibits the interaction of Protein-Y with Protein-X can also be given as one example of the aforementioned compound.

The aforementioned screened compounds and controlling agents can be used, singly or in combination thereof, as reagents or ingredients for pharmaceutical compositions. The compounds and controlling agents can be used in the elucidation of biological functions or diseases that Protein-X and/or Protein-Y as well as the genes thereof are involved in. For example, the elucidation of the mechanism of the NMDA receptor signal transduction, or the elucidation of neurodegenerative diseases and diseases caused by an anomaly in the NMDA receptor mediated signal transduction are contemplated.

In addition, the compounds identified by the aforementioned method can be used as candidate compounds for a controlling agent (for instance, an inhibiting agent, an antagonist, or a promoting agent) of the activity of Protein-X or of a polypeptide that has the same biological activity as that of Protein-X, for example, of the binding activity to other proteins such as an NMDA receptor, or of the guanylate kinase activity. In addition, they can also be used as candidate compounds for a controlling agent (for instance, an inhibiting agent, an antagonist, or a promoting agent) related to the expression at the gene level of Protein-X or of a polypeptide that has the same biological activity as that of Protein-X. Alternatively, they can be used as candidate compounds for a controlling agent, for instance, an inhibiting agent, an antagonist, or a promoting agent of the activity of Protein-Y or of a polypeptide that has the same biological activity as that of Protein-Y (for example, interaction with Protein-X). In addition, they can also be used as candidate compounds for a controlling agent (for instance, an inhibiting agent, an antagonist, or a promoting agent) related to the expression at the gene level of Protein-Y or of a polypeptide that has the same biological activity as that of Protein-Y. For example, antisense oligonucleotides of the Protein-X gene or the Protein-Y gene can be given as examples of compounds that inhibit such an expression. The antisense oligonucleotide is obtained from the oligonucleotides (designed by referring to the nucleotide sequence of the Protein-X gene or the Protein-Y gene), by selecting oligomers that specifically inhibit the expression of the Protein-X gene or the Protein-Y gene with an expression system of the Protein-X gene or the Protein-Y gene. Herein, "specifically inhibit the expression of the Protein-X gene or the Protein-Y gene," means to powerfully inhibit the expression of the Protein-X gene or the Protein-Y gene, while not inhibiting or weakly inhibiting the expression of other genes.

The candidate compound screened in such a manner can be prepared as a pharmaceutical composition by further selecting it while taking into account the balance between biological usefulness and toxicity. In addition, the polypeptides or the peptides of the invention, the polynucleotides of the invention or complementary strands thereof, vectors that contain the polynucleotides or complementary strands thereof, as well as antibodies that immunologically recognize the polypeptides or the peptides of the present invention, can be used in the elucidation, diagnosis, prevention, improvement, or treatment of various disease conditions caused by an anomaly in the expression or function of the polypeptide of the present invention, for example, neurodegenerative diseases. That is to say, the present invention provides a pharmaceutical composition that contains at least one among the aforementioned substances, for use singly or in combination thereof.

As an anomaly in the expression, excess or decrease in the expression can be cited. As anomalies in the functions, anomalies in the binding of Protein-X to an NMDA receptor, or anomalies in the interaction of Protein-X with Protein-Y, can be cited. These anomalies result in anomalies in NMDA receptor mediated signal transduction. More specifically, those result in excess, decrease, loss, or the like of the NMDA receptor mediated signal transduction. As neurodegenerative diseases, Alzheimer's disease, Parkinson's disease, and polyglutamine disease, and the like can be cited: however neurodegenerative diseases are not restricted to the above.

Protein-X possesses a single PDZ domain, while PSD-95 possesses three. This single PDZ domain was shown to also bind to an NMDA receptor/2B subunit. Therefore, Protein-X can probably support for PSD-95-like signal transduction and exhibit binding selectivity to other receptors and the like according to the number of PDZ domains. A scheme for enhancement of signals and normalization of abnormal signals that is derived from this inferred function can also be used.

One method for treating abnormal conditions related to the expression of Protein-X and/or Protein-Y of the present invention, and/or a decrease, loss, or the like of the functions thereof is characterized by administering Protein-X and/or Protein-Y, or a polypeptide having the same functions as these proteins, or a therapeutically effective dose of a compound that activates the genes coding therefore, together with a pharmaceutically acceptable carrier, and thereby improving an abnormal condition. Alternatively, using gene therapy, Protein-X and/or Protein-Y or a polypeptide having the same function as these proteins can be produced within a cell of a subject. A well-known method can be used for the gene therapy; for example, a replication-defective retroviral vector containing the polynucleotide of the invention (that is produced by transfecting the polynucleotide to the vector as described above) can be used in the gene therapy. In addition, for instance, using DNA or RNA that codes for the target protein, cells derived from the subject can be treated ex vivo, for instance, using a retroviral plasmid vector, and then the cells can be introduced into the subject.

When the expression of Protein-X and/or Protein-Y and/or the functions thereof are excessive, an effective dose of the aforementioned inhibiting compound can be administered together with a pharmaceutically acceptable carrier into a subject to inhibit the function of these proteins. For instance, the binding of Protein-X to an NMDA receptor, and/or the promotion of the NMDA receptor mediated signal transduction by the interaction of Protein-X with Protein-Y, thereby improving the abnormal condition. In addition, expression block can be used to inhibit the expression of the gene coding for the endogenous polypeptide. The expression of the gene can be inhibited using an oligonucleotide that has been generated in a cell or separately administered, which can include the antisense sequence of the gene. These oligonucleotides can be designed and synthesized, based on the aforementioned polynucleotides of the present invention. The oligonucleotides can be administered by themselves; otherwise, a related oligomer can be expressed in vivo.

Functional defects such as excess, decrease, or loss of expression and/or function of Protein-X and/or Protein-Y, lead to anomalies in the control of physiological functions in which Protein-X and/or Protein-Y are involved, for instance, neurotransmitter release provoked as a result of the membrane receptor mediated signal transduction, which causes disease conditions. Therefore, the present invention is useful in the elucidation of the biological functions in which Protein-X and/or the PJ1087 protein are involved, for instance, the elucidation of the control of intracellular signal transduction and neurotransmitter release. In addition, the present invention is extremely useful for a prophylactic agent, an improving agent, or a therapeutic agent, as well as a prevention method, an improvement method, or a treatment method for diseases provoked by an anomaly in the control of intracellular signal transduction or neurotransmitter release in which Protein-X and/or Protein-Y are involved. For instance, diseases caused by an anomaly of the NMDA receptor mediated signal transduction, or neurodegenerative diseases. Furthermore, it is extremely useful for the measurement method used as a means for diagnosing the diseases.

Recently, observations were obtained in relation to the mechanism of memory recovery in the brain, in an experiment using a knockout mouse. That is, an NMDA receptor in the CA3 region of the hippocampus was playing an important role in memory recovery (Nakazawa, K., et al., Science, 2002, Vol. 297, No. 5579, pp. 211-218). This function of the NMDA receptor, when normalized or facilitated, can prevent, improve, or treat symptoms of decreased ability for memory in diseases related to memory recovery such as Alzheimer's disease. From the forgoing, the present invention provides a medicament for use in the prevention, the improvement, or the treatment of diseases related to memory recovery, such as Alzheimer's disease, to be developed.

It is preferable to prescribe the pharmaceutical compositions, the controlling agent, the prophylactic agents, the therapeutic agents, or the improving agents of the present invention, in combination with an adequate medicinal carrier. Such a prescription can include a therapeutically effective dose of the polypeptides or the peptides of the invention, the polynucleotides of the invention or the complementary strands thereof, the vectors comprising the polynucleotides or the complementary strands thereof, the antibodies that immunologically recognize the polypeptides or the peptides of the invention, the aforementioned compounds, the aforementioned controlling agents, prophylactic agents, therapeutic agents, improving agents, or pharmaceutical compositions, and a pharmaceutically acceptable carrier or excipient. Examples of such a carrier can include physiological saline, buffered physiological saline, dextrose, water, glycerol, ethanol, and mixtures thereof, but are not limited thereto. It is adequate to select a prescription that is suited to the administration route, and such prescriptions are well-known to those skilled in the art. These pharmaceutical compositions, controlling agents, prophylactic agents, therapeutic agents, or improving agents can be used alone or together with another compound or medicament that is needed in the treatment.

The administration form of the pharmaceutical compositions, controlling agents, prophylactic agents, therapeutic agents, or improving agents of the present invention can be a systemic administration or a topical administration. One preferred mode of systemic administration is injection, for example, venous injection. Other injection routes such as subcutaneous, intramuscular, or intraperitoneal injection can also be used. Other modes of administration can be oral administration, if an enteric formulation or capsule formulation can be suitably formulated. In addition, per mucosal administration or per cutaneous administration using a permeating agent such as bile salt, fusidic acid, or other surfactants can also be used. Topical administration can be in the forms of plaster, paste, gel and similar form.

The required dosage range depends on the efficacy of the polypeptides or the peptides of the invention, the polynucleotides of the invention or the complementary strands thereof, the vectors comprising the polynucleotides or the complementary strands thereof, the antibodies that immunologically recognize the polypeptides or the peptides of the invention, the aforementioned compounds, controlling agents, prophylactic agents, improving agents, therapeutic agents, or pharmaceutical compositions, the administration route, the characteristics of the prescription, the characteristics of the conditions of the subject, and the assessment by the physician in charge. More specifically, an adequate dose is, for example, a range between 0.1 µg and 100 µg per 1 kg of the body weight of the subject. However, these doses can be modified using general conventional experiments for optimization well-known in the field.

For formulations, formulation means that are well-known should be introduced in accordance with the physical properties of each subject such as a peptide, protein, oligonucleotide, antibody, or compound. More specifically, a formulation method for, for instance, powdered drug, pills, tablets, capsule formulations, aqueous solution formulations, ethanol solution formulations, liposome formulations, fat emulsions, or clathrates such as cyclodextrin can be used.

Powdered drugs, pills, capsule formulations, and tablets can be manufactured using excipients such as lactose, glucose, sucrose, and mannitol; disintegrants such as starch and sodium alginate; lubricants such as magnesium, stearate, and talc; binders such as polyvinyl alcohol, hydroxypropyl cellulose, and gelatin; surfactants such as fatty acid ester; and plasticizers such as glycerin. A solid pharmaceutical carrier can be used to manufacture a tablet or a capsule.

Suspending agents can be manufactured using water; sugars such as sucrose, sorbitol, and fructose; glycols such as PEG; and oils.

Injectable solutions can be prepared using a carrier comprising a saline solution, a glucose solution, or a mixture of salt water and glucose solution.

Inclusion into liposomes can be performed, for instance, by adding a solution wherein the substance of interest has been dissolved in a solvent (such as ethanol), to a solution wherein phospholipids have been dissolved in an organic solvent (such as chloroform), then removing the solvent by evaporation, adding a phosphate-buffered solution thereto, and after agitating, sonicating, and centrifugating, filtrating the supernatant for recovery.

Creation of fat emulsions can be performed, for example, by mixing and heating the substance of interest, oil ingredients (vegetable oils such as bean oil, sesame oil, and olive oil as well as MCT and the like), emulsifying agent (such as phospholipids) and the like to obtain a solution, then adding a required amount of water and emulsifying/homogenizing by an emulsifier (homogenizer, for instance, high-pressure-spray type or sonicating type and the like). In addition, this can also be lyophilized. Further, when preparing a fat emulsion, an emulsification helper can be added; examples of an emulsification helper include glycerin and sugars (for instance, glucose, sorbitol, fructose, and the like).

Creation of cyclodextrin clathrates can be performed, for instance, by adding a solution wherein cyclodextrin has been dissolved in water and the like by heating to a solution wherein the substance of interest has been dissolved in a solvent (such as ethanol), then cooling, filtrating the deposition resulting from cooling, and dry-sterilizing. In so doing, the cyclodextrin to be used should be suitably selected from cyclodextrins with different void diameters (α, β, and γ types) according to the size of the substance.

### Measurement method and reagent for diagnosis

The polypeptides or the peptides of the present invention, the polynucleotides of the invention or the complementary strands thereof, or the antibodies that immunologically recognize the polypeptides or the peptides can be used singly as diagnostic markers or reagents and the like. When these are reagents, they can contain a substance such as buffering solution, salt, stabilization agent, and/or antiseptic agent. The polypeptides or the peptides of the invention can be a cell in which these have been expressed by a genetic engineering method, cell-free synthesis products, chemical synthesis products, or those prepared from the cells and biological samples, as well as those that are further purified therefrom. In addition, if the functions of the polypeptides or peptides of the present invention, for instance, binding to an NMDA receptor, interaction with Protein-X, or interaction with Protein-Y, or for example the amplification of the NMDA receptor mediated signal transduction due to the interaction are not inhibited, the polypeptides or the peptides of the invention may have another protein or peptide, for example such as β-gatactosidase, immunoglobulin Fc fragment from IgG or the like, His-tag, Myc-tag, HA-tag or FLAG-tag, ligated to the N-terminal side or the C-terminal side, directly or indirectly via a linker peptide or the like, using genetic engineering techniques or the like. In addition, if the functions of the polynucleotides of the present invention, for instance, expression of the encoded polypeptide and the functions of the expressed polypeptide are not inhibited, the polynucleotides of the present invention may have a signal such as luciferase or green fluorescent protein (GFP), or a reporter gene such as chloramphenicol acetyl transferase (CAT) gene ligated to the 5' end side or the C-terminal side. In addition, the present invention also provides a reagent kit comprising one or more containers that are filled with one or more kinds of these reagents. Furthermore, for formulations, formulation means that are well-known in the art should be introduced, in accordance with each of a polypeptide, peptide, protein, polynucleotide, or antibody, and the like.

These reagents and kits can be used in the identification methods of the present invention. In addition, these reagents and kits can be used for quantitatively and/or qualitatively measuring the polypeptides or peptides of the present invention, or polynucleotides coding for any one of these. The measurement method can be established using methods well-known to those skilled in the art. Examples of methods that can be used to measure the polypeptides or the peptides include radio immunoassay, competitive binding assay, Western blot analysis, and ELISA. In addition, the polynucleotides can be detected and quantified at the nucleic acid level, for example the RNA level, using, for instance, amplification, PCR, RT-PCR, RNase protection, Northern blotting, and other hybridization methods.

The aforementioned reagents, reagent kits, and measurement methods can be used in the detection method of diseases caused by an anomaly in the expression of the polypeptides or peptides of the present invention, for instance, Protein-X and/or Protein-Y, or the function thereof. Alternatively, they can be used in detection methods of various pathologies caused by a mutation and the like of the DNA coding therefor. Examples of the diseases include neurodegenerative diseases, such as Alzheimer's disease, Parkinson's disease, and polyglutamine disease.

Examples of samples to be measured include cells, blood, urine, saliva, spinal fluid, tissue biopsy, or necropsy materials and the like derived from an individual. In addition, the nucleic acids to be measured can be obtained from each of the aforementioned samples by nucleic acid preparation methods well-known in the art. For the nucleic acid, genomic DNA can be used directly for detection, or it may be enzymatically amplified prior to analysis by PCR or other amplification methods. RNA or cDNA can be used in a similar manner. Deletions and insertions can be detected in comparison with the normal genotype, based on the changes in the sizes of the amplification products. Point mutations can be identified by hybridizing the amplified DNA to a labeled DNA that codes for the polypeptide of the invention.

That is to say, with a sample derived from an individual, the disease can be diagnosed, for instance, by detecting the presence of the corresponding nucleic acid to the polynucleotide that codes for the polypeptide or the peptide of interest using the interaction therewith and responsiveness thereto; determining the quantity present thereof; and/or identifying the variation thereof; as well as determining the in vivo distribution of the polypeptide or peptide in the individual; and/or detecting the presence of the polypeptide or peptide; determining the quantity present thereof; and/or detecting the variation thereof.

Furthermore, the aforementioned diseases can be examined and diagnosed by qualitatively or quantitatively measuring the polypeptides or peptides of the present invention or the nucleic acid coding for any one of these, as diagnostics markers. That is to say, by using the aforementioned detection method, a method for examining or diagnosing the diseases can further be performed.

### EXAMPLES

The present invention will be further described in the following examples. However, the present invention is not limited to these examples.

### Example 1

### Isolation/identification of a novel PSD gene (Protein-X gene)

Cloning was performed using a template cDNA that was prepared by reverse transcription polymerase chain reaction (RT-PCR) from human brain-derived mRNA based on clone hj02537, which was extracted as a gene having a PDZ domain, an SH3 domain and a GK domain, via bioinformatics from the cDNA analysis information database (containing information of cDNA derived from human brain almost completely mRNA) of the Kazusa DNA Research Institute, to verify the in vivo expression of the Protein-X gene of the present invention.

First, human brain-derived mRNA was used to perform a reverse transcription reaction using the Super Script II kit (Gibco BRL). After mixing 0.2 µl of human brain-derived mRNA (1 µg/µl), 1 µl of oligo (dT) primer (0.5 µg/µl) and 10.8 µl of H₂O; the mixture was heated for 10 minutes at 70°C, cooled on ice; and then 4 µl of 5x First strand buffer, 2 µl of 0.1 M dithiothreitol (DTT) and 1 µl of 10 mM deoxynucleotide trisphosphate (dNTP) mix were added. Next, 1 µl of Super Script II (200 U/µl) was added, and the reaction was allowed to take place for 10 minutes at room temperature, for 50 minutes at 42°C, and for 15 minutes at 70°C to prepare cDNA.

Then, based on the nucleotide sequence of clone hj02537, the following primers were designed and synthesized. In the primer sequence, the underlined ATG is a translation initiation codon.

### Primers

Pr-HJf (SEQ ID NO: 7):

5'-GGGGACAAGTTTGTACAAAAAAGCAGGCTTAATGCCAGCTTTGTCAACGG-3'

Pr-HJf (SEQ ID NO: 8):

5'-GGGGACCACTTTGTACAAGAAAGCTGGGTCCATCTTGGAGATAGAGCGG-3'

The Advantage 2 PCR kit (Clontech) was used for the polymerase chain reaction (PCR). After mixing 2 µl of 10× Advantage 2 PCR buffer, 0.2 µl of the above-mentioned Pr-HJf (53.09 pmol/µl), 0.3 µl of the above-mentioned Pr-HJr (38.59 pmol/µl), 1 µl of cDNA prepared above, 1.6 µl of 1.25 mM dNTP mix, 0.4 µl of 50× polymerase mix and 14.5 µl of H₂O (20 µl in total), PCR reaction was performed under the following conditions.

### Conditions for carrying out PCR

Pre-step (pre): 95°C for 1 minute
Step 1: 95°C for 30 seconds
Step 2: 68°C for 2 minutes
(Steps 1-2 for 25 cycles)
Post-step (post): 68°C for 5 minutes

The obtained PCR product was used to conduct cloning using the Gate way system (Invitrogen). After adjusting 16 µl of the PCR product to 100 µl with TE buffer followed by adding 50µl of 30% PEG8000/30mM MgCl₂, the mixture was centrifuged at room temperature (at 15,000 rpm for 10 minutes). The pellet was washed with 70% ethanol, dried, and dissolved in 30 µl of TE buffer. Thereafter, 1 µl of BP clonase reaction buffer, 1 µl of the PCR product, 0.5 µl of entry vector (pDONR 201:150 ng/µl) and 1.5 µl of TE buffer were mixed on ice (4 µl in total) followed by adding 1 µl of BP clonase enzyme mix, and then the reaction was allowed to take place for 4.5 hours at 25°C. After the reaction, 0.5 µl of Proteinase K was added, and the reaction solution was incubated for 10 minutes at 37°C to inactivate the enzyme mix. Competent cells JM109 were transformed with 1 µl of this reaction solution, and pDONR 201/hj02537 #7 and pDONR 201/hj02537 #9 were obtained. From the nucleotide sequences of these clones, expression in the brain was verified.

### Example 2

### Expression of the novel PSD (Protein-X)

In order to express Protein-X, an expression vector and an expression system of *Escherichia coli* were established using the Gate way system (Invitrogen). The protein-coding region was amplified using the Advantage 2 PCR kit using the clone hj02537 as a template. After mixing 2 µl of 10× Advantage 2 PCR buffer, 0.2 µl of Pr-HJf (53.09 pmol/µl), and 0.3 µl of Pr-HJr (38.59 pmol/µl), which were constructed in Example 1, 2 µl of clone hj02537 (1 ng/µl), 1.6 µl of 1.25 mM dNTP mix, 0.4 µl of 50× polymerase mix, and 13.5 µl of H2O (two samples of 20 µl in total were prepared), the PCR reaction was carried out in the same way as in Example 1.

After adjusting 35 µl of the PCR product to 100 µl with TE buffer followed by adding 50 µl of 30% PEG8000 / 30 mM MgCl₂, the mixture was centrifuged (at 15,000 rpm for 10 minutes) at room temperature. The pellet was washed with 70% ethanol, dried, and dissolved in 50 µl of TE buffer. Thereafter, 1 µl of BP clonase reaction buffer, 1 µl of the PCR product, 0.5 µl of entry vector (pDONR 201:150 ng/µl), and 1.5 µl of TE buffer were mixed on ice (4 µl in total). Then 1 µl of BP clonase enzyme mix was added, and the reaction was allowed to take place overnight at 25°C. After the reaction, 0.5 µl of Proteinase K was added, and the reaction solution was incubated for 10 minutes at 37°C. to inactivate the enzyme mix. Competent cells JM109 were transformed with 1 µl of this reaction solution, and pDONR 201/hj02537 #1 was obtained.

Next, using pDONR 201/hj02537 #1, a vector that expresses Protein-X as a fusion protein with a 6×His-tag was constructed. After mixing 1 µl of LR clonase reaction buffer, 1 µl of pDONR 201/hj02537 #1 (50 ng/µl), 0.5 µl of 6×His-tag expression vector (pDEST17:150 ng/µl) and 1.5 µl of TE buffer (4 µl in total) on ice, 1 µl of LR clonase enzyme mix was added, and then the reaction was allowed to take place for 2 hours at 25°C. After the reaction, 0.5 µl of Proteinase K was added, and the reaction solution was incubated for 10 minutes at 37°C to inactivate the enzyme mix. Competent cells (BL21-SI) were transformed with 1 µl of this reaction solution, and three clones (pDEST17/hj02537 #9, pDEST17/hj02537 #10, and pDEST17/hj02537 #11) were obtained.

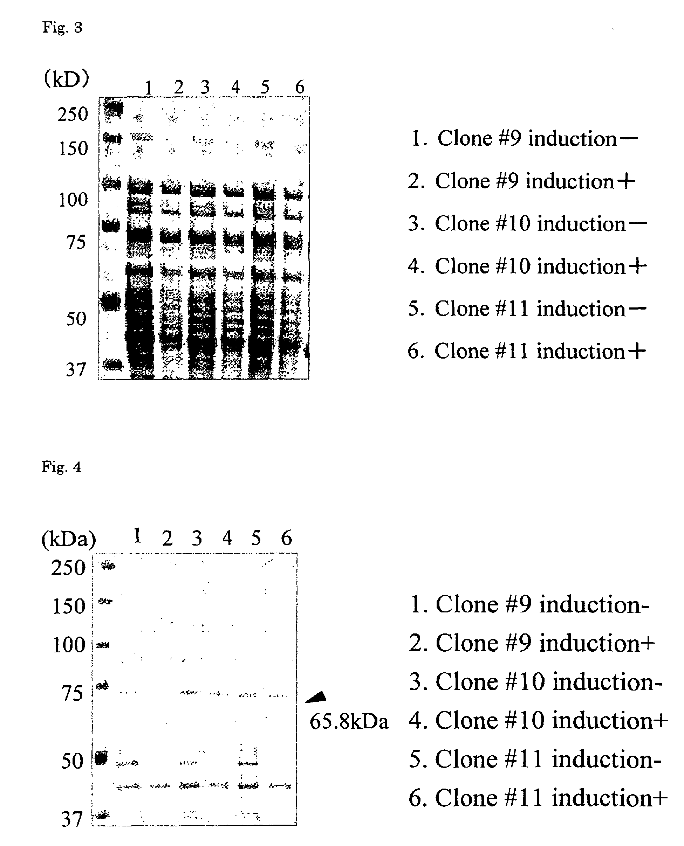

The three clones were tested for the presence or absence of induction by NaCl, for the production of the target protein and for preparation of soluble protein. Each *Escherichia coli* was cultured by agitating overnight at 37°C in 2 ml of LB medium containing ampicillin (hereinafter referred to as LB-Amp), to which NaCl (NaCl-) was not added. After culturing a total volume of 3 ml of LB-Amp (NaCl-), to which 300 µl of this pre-cultured solution had been added, under agitation for 2 hours at 37°C, 180 µl of 5 M NaCl was added thereto (a final concentration of 0.3 M), and cultured under agitation for a further 2 hours, at 37°C. As a control, 180 µl of H₂O was added instead of NaCl, and the culture proceeded in the same way. Thereafter, two samples of 1.2 ml of each cultured solution were prepared and centrifuged (at 15,000 rpm for 10 minutes at 4°C), and separated into supernatants (hereinafter referred to as soluble fractions) and pellets. The pellets were suspended in 200 µl of 2% SDS / 20 mM Tris (pH 7.4), or 20 mM Tris (pH 7.4), and then sonicated and centrifuged again (for 10 minutes at 15,000 rpm at 10°C or 4°C). This supernatant of the centrifugation (hereinafter can be referred to as an insoluble fraction) was mixed with an equal volume of 2× sample buffer (2% SDS / 50 mM Tris (pH 6.8) / 30% glycerol / 0.01% Bromophenol Blue (BPB)) containing 10% β-mercaptoethanol (ME); the mixture was boiled for 2 minutes, it was then subjected to SDS-polyacrylamide gel electrophoresis (SDS-PAGE) over a 7.5% polyacrylamide gel; and the proteins were detected by Coomassie Brilliant Blue (CBB) staining. In addition, the above-mentioned soluble fraction was also subjected to SDS-PAGE to carry out protein detection. As a result, shown in Fig. 2, a band at approximately 65.8 kDa was detected, which is expected to be a fusion protein of the human Protein-X with the 6×His-tag. It was considered that the fusion protein was present in small amounts in the soluble fraction of the cultured *Escherichia coli* mentioned above (Fig. 3), but was present in large amounts in the insoluble fraction.

In order to test the expression of the fusion protein of Protein-X, with the 6×His-tag in the soluble fraction, the culturing of the aforementioned *Escherichia coli* was performed at 25°C. Each *Escherichia coli* was cultured overnight in 2 ml of LB-Amp (NaCI-), under agitation at 25°C. After culturing a total volume of 2.2 ml of LB-Amp (NaCI-), to which 200 µl of this pre-cultured solution had been added, under agitation for 4 hours at 25°C, 132 µl of 5 M NaCl was added (a final concentration of 0.3 M), and culture under agitation at 25°C for 2 more hours. As a control, 132 µl of H₂O was added instead of NaCl, and cultured in the same way. Thereafter, two samples of 1ml of the cultured solution were prepared and centrifuged (at 15,000 rpm for 10 minutes at 4°C), and separated into supernatants and pellets. The pellets were suspended in 200 µl of 2% SDS / 20 mM Tris (pH 7.4) and 20 mM Tris (pH 7.4), and then sonicated and centrifuged again (at 15,000 rpm for 10 minutes at 10°C or 4°C). The supernatants of the centrifugation were mixed with an equal volume of 2× sample buffer containing 10% β-ME; the mixture was boiled for 2 minutes and subjected to SDS-PAGE over a 7.5% polyacrylamide gel; and the fusion proteins were detected in the same way as described above. As shown in Fig. 4, the fusion protein of Protein-X with the 6×His-tag was also expressed in the soluble fraction of the culture at 25°C.

The human Protein-X obtained as described above, based on the clone hj02537, was proven to comprise 576 amino acid residues as set forth in SEQ ID NO: 1 in the Sequence Listing, and possess a PDZ domain from isoleucine (Ile) 139 to glycine (Gly) 219, an SH3 domain from methionine (Met) 231 to arginine (Arg) 296, and a GK domain from threonine (Thr) 404 to asparagine (Asn) 500, in the amino acid sequence thereof.

### Example 3

### Binding of the novel PSD (Protein-X) to an NMDA receptor/2B subunit

The binding activity of Protein-X to an NMDA receptor/2B subunit was examined using the Overlay method.

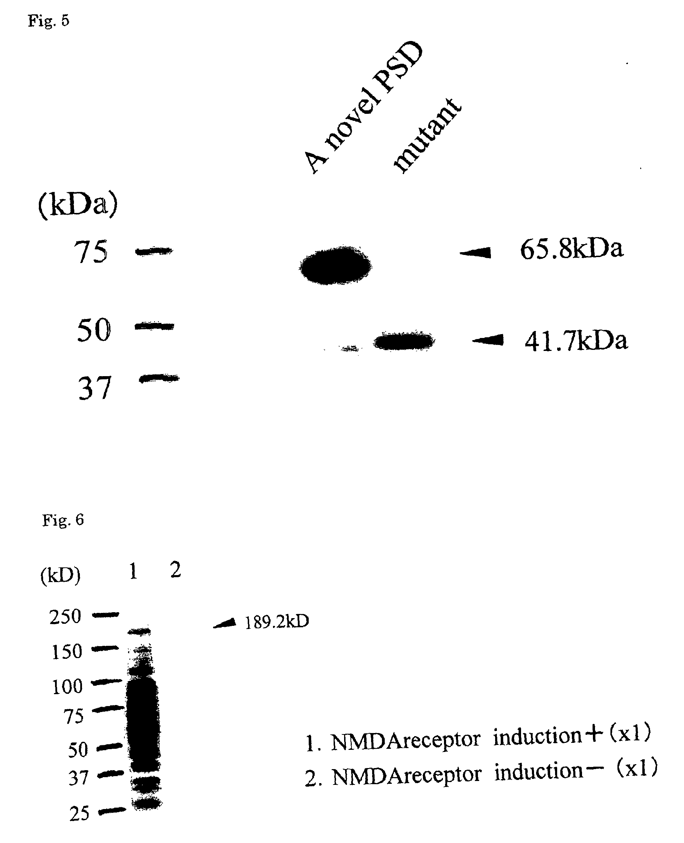

First, *Escherichia coli* (pDEST17/hj02537 #11) that expresses the fusion protein of Protein-X with a 6xHis-tag was cultured overnight in 2 ml of LB-Amp (NaCl-) at 25°C. After culturing a total of 2.2 ml of LB-Amp (NaCl-), to which 200 µl of this pre-cultured solution had been added, under agitation for 4 hours at 25°C, 132 µl of 5 M NaCl was added thereto (a final concentration of 0.3 M), and cultured under agitation for 2 more hours at 25°C to induce the production of Protein-X. After culturing, *Escherichia coli* was collected; 200 µl of extraction buffer (1% Triton X / 10 mM Tris (pH 7.5) / 150 mM NaCl / 1 mM PMSF (phenylmethylsulfonylfluoride)) was added; and after sonication and incubation on ice for 20 minutes, centrifugation was performed (at 15,000 rpm for 10 minutes at 4°C). The supernatant of the centrifugation was collected and used as a sample containing Protein-X. In addition, a fraction of the supernatant of the centrifugation was used to carry out SDS-PAGE in the same way as described in Example 2, to verify the expression and the expressed amount of Protein-X by Western blotting (Fig. 5), in order to determine the quantity of sample to use. The Western blot was carried out as described below. First, after electrophoresis, the proteins were transferred by the semi-dry method (for 1 hour at 100 mA) onto a wet PVDF transfer membrane, and the membrane was blocked with 5% skim milk / TBS-T for 1 hour at room temperature. Thereafter, a mouse anti-His antibody (Amersham Pharmacia Biotech) that was diluted 1000-fold with 5% skim milk / TBS-T was added, and allowed to react for 1 hour at room temperature. After the reaction, the transfer membrane was washed three times with TBS-T for 10 minutes at room temperature. Then a secondary antibody (anti-mouse IgG antibody-horseradish per oxidase (HRP) (Cell Signaling)) that was diluted 3000-fold with 5% skim milk / TBS-T was added, and was allowed to react for 1 hour at room temperature. After that, a 10-minute wash with TBS-T was performed three times at room temperature. Thereafter, the signal was detected using the ECL kit and Hyperfilm.

*Escherichia coli* pDEST17/hjPDZ (-) #2, a mutant that lacks the PDZ domain, which is the region that binds to an NMDA receptor/2B subunit, was prepared to be used as a negative control as described below. pDEST17/hjPDZ (-) #2 was cultured in 2 ml of LB-Amp (NaCl-) overnight at 37°C. After culturing, a total of 2.2 ml of LB-Amp (NaCl-), to which 200 µl of this pre-cultured solution had been added, under agitation for 4 hours at 37°C, 132 µl of H₂O was then added thereto, and cultured under agitation for a further 2 hours at 37°C, to induce the production of the Protein-X mutant. After culturing, *Escherichia coli* was collected and treated as described above to prepare samples containing the Protein-X mutant. In addition, the expression and the expressed amount thereof were determined in the same way as described above (Fig. 5).

*Escherichia coli* pDEST15/NMDA receptor #5, which expresses the fusion protein of NMDA receptor/2B subunit with GST (hereinafter can be referred to as GST-NMDA receptor/2B subunit), was prepared as described below, and cultured overnight in 2 ml of LB-Amp (NaCl-) at 37°C. After culturing a total of 3 ml of LB-Amp (NaCl-), to which 300 µl of this pre-cultured solution had been added, under agitation for 4 hours at 37°C, 180 µl of 5 M NaCl was then added thereto (a final concentration of 0.3 M), and cultured under agitation for 2 more hours at 37°C, to induce the production of the fusion protein of NMDA receptor/2B subunit with GST. As a control, H₂O was added instead of NaCl, and the culture proceeded in the same way. After culturing, the *Escherichia coli* was collected, 200 µl of 2% SDS / 20 mM Tris (pH 7.5) was added, and the resulting solution was sonicated and centrifuged (at 15,000 rpm for 10 minutes at 10°C). The supernatant of the centrifugation was collected and used as a sample containing an NMDA receptor/2B subunit. In addition, to verify the expression and the expressed amount of the target protein, a portion of the supernatant of the centrifugation was collected and diluted one-fold and one-half-fold with 2% SDS / 20 mM Tris (pH 7.5). Each diluted supernatant of the centrifugation and 3× sample buffer (NEB) containing 10% β-ME were mixed at a proportion of 2:1, the mixtures were boiled for 2 minutes and subjected to electrophoresis over a 5%-20% polyacrylamide gel. After electrophoresis, the proteins were transferred onto a wet PVDF transfer membrane (Poly screen (NEN)) (for 1.5 hours at 300 mA in a bath), and the membrane was blocked with 5% skim milk / TBS-T (TBS-T: 10 mM Tris (p H7.5) / 150 mM NaCl / 0.05% Tween 20) for 1 hour at room temperature. Thereafter, a goat anti-GST antibody (Amersham Pharmacia Biotech) that was diluted 1000-fold with 5% skim milk / TBS-T, was added to the incubation solution, and allowed to react for 1 hour at room temperature. After the reaction, the transfer membrane was washed three times with TBS-T for 10 minutes at room temperature. Then a secondary antibody (anti-goat IgG antibody-HRP, Santa Cruz Biotechnology) that was diluted 2000-fold with 5% skim milk / TBS-T was added, and allowed to react for 1 hour at room temperature. After that, a 10-minute wash with TBS-T was performed three times at room temperature. Thereafter, the signal was detected using the ECL kit and Hyper Film Signal (Amersham Pharmacia Biotech) (Fig. 6).

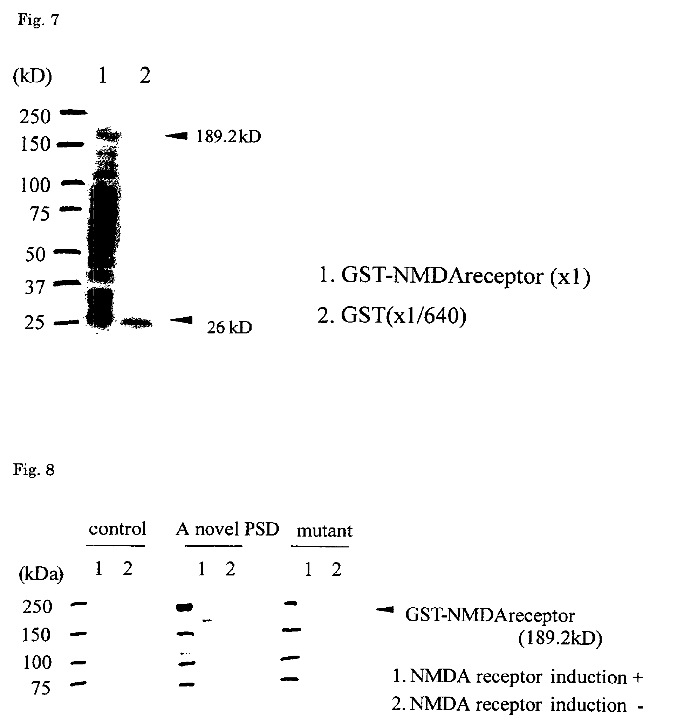

In addition, in order to use as a negative control, *Escherichia coli* DH5α (derived from pGEX-4T-3 from Amersham Pharmacia Biotech) expressing only GST was cultured in 8 ml of LB-Amp overnight at 37°C. After culturing a total of 22 ml of LB-Amp, to which 2ml of this pre-cultured solution had been added, for 1.5 hours at 37°C until the value at OD600 reached to approximately 1.0, 42 µl of 500mM IPTG (isopropyl 1-thio-β-D-galactoside) was added thereto (a final concentration of 1mM), and cultured under agitation for 2 more hours at 37°C. After culturing, 1.5 ml samples were taken from each, and the bacteria were collected. This *Escherichia coli* was treated as described above, and the obtained supernatant of the centrifugation was used as a sample. In addition, to verify the expression and the expressed amount of the target protein, a fraction of the supernatant of the centrifugation was collected and diluted ×1/40, ×1/80, ×1/160, ×1/320, ×1/640 and ×1/1280 with 2% SDS / 20 mM Tris (pH 7.5). Using each diluted supernatant of the centrifugation, the expression of GST was verified in the same way as described above (Fig. 7).

Then, the binding activity of Protein-X to an NMDA receptor/2B subunit was examined using the Overlay method. Concerning samples containing a GST-NMDA receptor/2B subunit (those induced with NaCl and those uninduced) or a GST prepared as described above, stock solutions were used for samples containing a GST-NMDA receptor/2B subunit (induced and uninduced) and ×1/640 diluted solution with 2% SDS / 20 mM Tris (pH 7.5) was used for sample containing GST, based on the results of verification of the expressed amounts by Western blotting as described above (Figs. 6 and 7). Each sample and 3× sample buffer (NEB) containing 10% β-ME were mixed at a proportion of 2:1, and the mixtures were boiled for 2 minutes and then subjected to electrophoresis over a 5%-20% polyacrylamide gel. After electrophoresis, the proteins were transferred onto a wet PVDF transfer membrane (for 1.5 hours at 300 mA in a bath), and the membrane was blocked with 5% skim milk / TBS-T for 2 hour at room temperature.

Subsequently, 3 ml of 1 mM PMSF / TBS-T was added to 20 µl of sample containing Protein-X, prepared as described above and 40 µl of extraction buffer, or to 60 µl of sample containing the Protein-X mutant prepared as described above, to make a mixture. As a control, a mixture obtained by adding 3 ml of 1 mM PMSF / TBS-T to 60 µl of extraction buffer was used. Each mixture was used and allowed to react with each of the transfer membranes for 2 hours at 4°C to bind thereto. Thereafter, a 5-minute wash with TBS-T was performed three times at room temperature, and the membranes were blocked again with 5% skim milk / TBS-T for 1 hour at room temperature. After blocking, a mouse anti-His antibody (Amersham Pharmacia Biotech) was diluted 1000-fold with 5% skim milk / TBS-T and added; and this composition was allowed to react for 1 hour at room temperature. After the reaction, the transfer membranes were washed using TBS-T for 10 minutes at room temperature; the wash was performed three times; then a secondary antibody (anti-mouse IgG antibody-HRP) (Cell Signaling) was diluted 3000-fold with 5% skim milk / TBS-T, then added and allowed to react for 1 hour at room temperature. After that, a 10-minute wash with TBS-T was performed three times at room temperature. Thereafter, a signal was detected using the ECL kit and Hyper film.

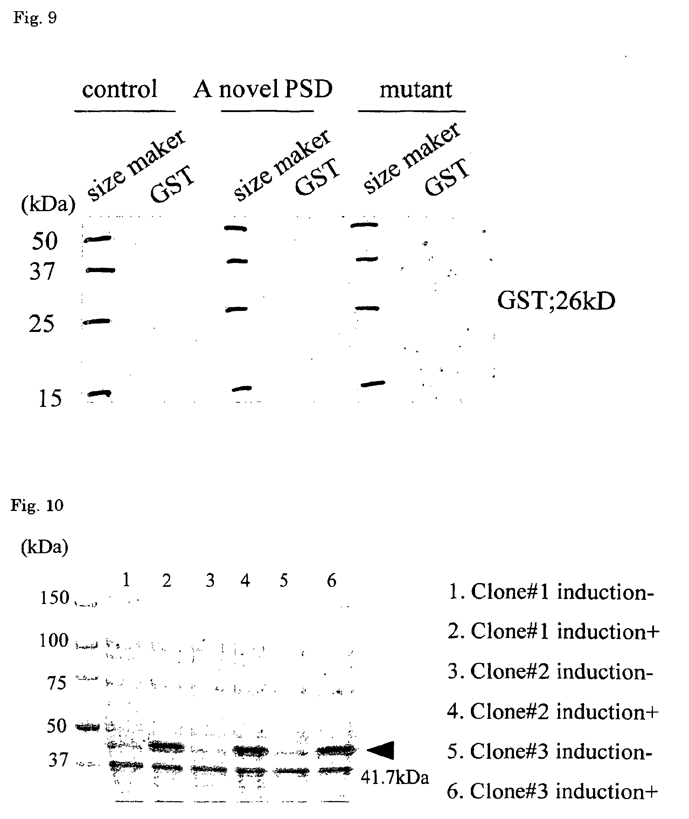

As a result, a signal was detected at the position of the band for the full-length GST-NMDA receptor/2B subunit (189.2 kDa) on the transfer membrane to which Protein-X was bound, which showed the binding of Protein-X to the NMDA receptor/2B subunit. When the control and the Protein-X mutant were used, no signal was detected; that is to say, no binding was observed (Fig. 8). No band was detected in any of the transfer membranes for which binding to GST was tested, which showed that no binding to GST was observed (Fig. 9). From the foregoing, it is suggested that Protein-X binds to an NMDA receptor/2B subunit via the PDZ domain thereof. In addition, Fig. 6 suggests that an NMDA receptor/2B subunit is a protein that is susceptible to degradation. However, from the experimental result described above, wherein a signal was detected only at the position of the band for the full-length NMDA receptor/2B subunit (Fig. 8), as well as that GST is located at the N-terminus, the region that binds to Protein-X is expected to be at the C-terminus of the NMDA receptor/2B subunit, which agrees with information from the literature (Nagano, T. et al., Journal of Biochemistry, 1998, Vol. 124, pp. 869-875). Proteins that possess a PDZ domain recognize sequences comprising three amino acid residues, where a serine (Ser) or a threonine (Thr) is followed by another amino acid that is followed by a valine (Val) (SXV or TXV), which are present at the C-terminal of the target protein (tSXV motif). The C-terminal amino acid sequence of the NMDA receptor/2B subunit used in the present Example was SDV.

### Preparation of the novel PSD (Protein-X) mutant

The mutant, which lacks the PDZ domain that is the region for binding to the NMDA receptor/2B subunit from the Protein-X, was prepared as described in the following.

First, based on the nucleotide sequence of clone hj02537, the following primers were designed and synthesized.

### Primers

Pr-Dpdz-F1 (SEQ ID NO: 9):

5'-GGGGACAAGTTTGTACAAAAAAGCAGGCTGCAGCAAAGAGGAGACACCATCAAA

-3'

PCR was carried out using the Advantage 2 PCR kit with the clone hj02537 as a template. After mixing 2 µl of 10× Advantage 2 PCR buffer, 0.2 µl of Pr-Dpdz-F1 (58.64 pmol/µl) and 0.3 µl of Pr-HJr (38.59 pmol/µl), 1 µl of clone hj02537 (1 ng/µl), 1.6 µl of 1.25 mM dNTP mix, 0.4 µl of 50× polymerase mix, and 14.5 µl of H₂O (20 µl in total), a PCR reaction was carried out under the conditions described in Example 1.

Thereafter, 16 µl of the PCR product was adjusted to 50 µl with TE buffer, and then 25 µl of 30% PEG8000 / 30 mM MgCl2 was added thereto. The mixture was centrifuged at room temperature (at 15,000 rpm for 10 minutes), then, the pellet was washed with 70% ethanol, dried, and dissolved in 30 µl of TE buffer. Subsequently, 1 µl of BP clonase reaction buffer, 1 µl of the PCR product, 0.5 µl of entry vector (pDONR 201: 150 ng/µl) and 1.5 µl of TE buffer were mixed on ice (4 µl in total). 1 µl of BP clonase enzyme mix was added thereto, and then the reaction was allowed to take place for 2.7 hours at 25°C. After the reaction, 0.5 µl of Proteinase K was added, and the reaction solution was incubated for 10 minutes at 37°C, to inactivate the enzyme mix. Competent cells (DH5α) were transformed with 1 µl of this reaction solution, and pDONR 210/hjPDZ(-) #2 was obtained.

Next, using pDONR 210/hjPDZ(-) #2, a vector that expresses a fusion protein of a Protein-X mutant, that lacks the PDZ domain, with a 6×His-tag, was constructed. After mixing 1 µl of LR clonase reaction buffer, 1 µl of pDONR 201/hjPDZ(-) #2 (50 ng/µl), 0.5 µl of 6×His-tag expression vector (pDEST17:150 ng/µl) and 1.5 µl of TE buffer (4 µl in total) on ice, 1 µl of LR clonase enzyme mix was added, and then the reaction was allowed to take place for 0.5 hours at 25°C. After the reaction, 0.5 µl of Proteinase K was added, and the reaction solution was incubated for 10 minutes at 37°C, to inactivate the enzyme mix. Competent cells (BL21-SI) were transformed with 1 µl of this reaction solution, and three clones (pDEST17/hjPDZ(-) #1, pDEST17/hjPDZ(-) #2 and pDEST17/hjPDZ(-) #3) were obtained.

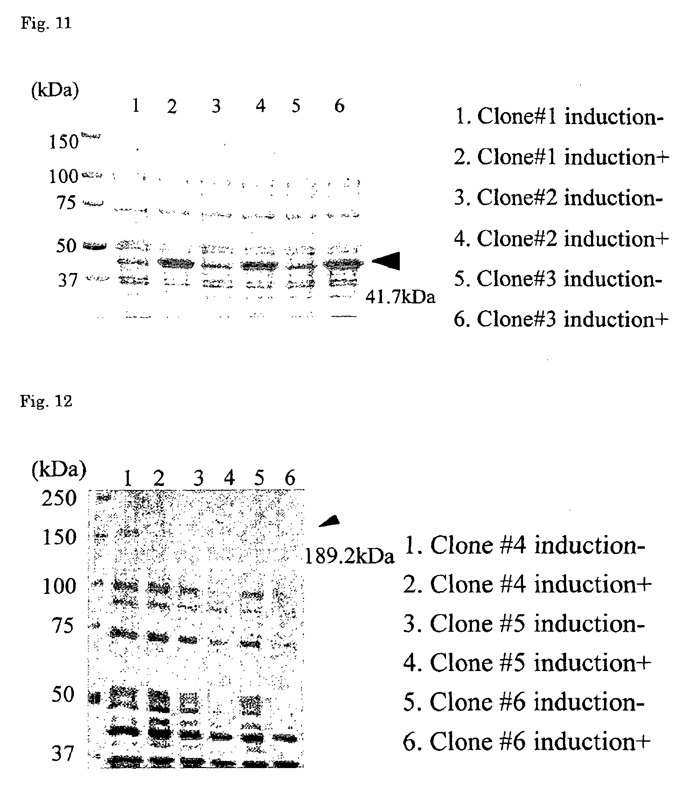

The three clones were tested for the presence or absence of induction by NaCl of the production of the target protein and also for the preparation of a soluble protein. Each *Escherichia coli* was cultured overnight in 2 ml of LB-Amp (NaCl-) under agitation at 37°C. After culturing, a total volume of 3.3 ml of LB-Amp (NaCl-), to which 300 µl of this pre-cultured solution had been added, under agitation for 2 hours at 37°C, 180 µl of 5 M NaCl was then added thereto (a final concentration of 0.3 M), and cultured under agitation for a further 2 hours, at 37°C. As a control, 180 µl of H2O was added, and the culture proceeded in the same way. Thereafter, two samples of 1.5 ml of each cultured solution were prepared and centrifuged (at 15,000 rpm for 10 minutes at 4°C). The pellets were suspended in 150 µl of 2% SDS/20 mM Tris (pH 7.4) and 20 mM Tris (pH 7.4), then sonicated and centrifuged again (at 15,000 rpm for 10 minutes at 10°C or 4°C). The supernatants of the centrifugation were mixed with an equal volume of 2× sample buffer containing 10% β-mercaptoethanol. The mixture was boiled for 2 minutes, subjected to SDS-PAGE on 5%-20% polyacrylamide gel, and the target protein was detected in the same way as described in Example 2. As a result shown in Fig. 10, a band at approximately 41.7 kDa was detected, which is expected to be a fusion protein of the human Protein-X mutant protein with the 6xHis-tag. The fusion protein was observed both in the soluble fraction and in the insoluble fraction of the cultured *Escherichia coli* mentioned above. In addition, the expression thereof was also observed in the soluble fraction when the culture was performed at 25°C (Fig. 11).

### Preparation of an NMDA receptor/2B subunit

The preparation of an NMDA receptor/2B subunit was carried out using clone fj07108 (Kazusa DNA Research Institute), which contains a portion thereof, as described below. Since clone fj07108 was lacking the 347 amino acid residues on the N-terminal side, this region was first subcloned, by RT-PCT using human brain-derived mRNA as a template.

PCR was carried out using an Advantage 2 PCR kit in the same way as in Example 1, using a template cDNA that was reverse-transcribed from human brain-derived mRNA using superscript II with an oligo(dT) primer and random primers, and PCR primers (Pr-NMDAR2Bf and Pr-NMDAR2B (RV), described below) that were designed based on the nucleotide sequence downstream from the AfIII site at position 352 of clone fj07108. The PCR primers were designed taking into consideration of the region upstream from the initiation codon and recombination. Thereafter, the PCR product, which ends were blunted and phosphorylated using the BKL kit (TaKaRa), was then introduced into pBluescript (SK-) (Smal digested) to give pBS(SK-)/NMDAr-N #10 and pBS(SK-)/NMDAr-N #25. The identification of the nucleotide sequence revealed that pBS(SK-)/NMDAr-N #10 had two mutations due to PCR, however, no amino acid substitution occurred. However, since the guanine (G) at 144 bp in the nucleotide sequence was lacking, a recombination was performed using pBS(SK-)/NMDAr-N #25 and exploiting the Smal site present in the region downstream therefrom and the BamHI site of pBluescript(SK-), and a new pBS(SK-)/NMDAr-N #3 was obtained. The nucleotide sequence thereof was determined, which was identical to that of the NMDA receptor/2B subunit (Accession No: NM_000843) registered in GenBank.

### Primers

Pr-NMDAR2Bf (SEQ ID NO: 10):

5'-TTTGGCTTCTACAAACCAAGGGAG-3'

Pr-NMDAR2B (RV) (SEQ ID NO: 11):

5'-TCCAATCATACCATTCCAGGTTCCA-3'

Thereafter, the multicloning site (MCS) (pBluescript) of clone fj07108 was digested with Xhol, which ends were then blunted using the Blunting kit (TaKaRa), and was digested with AfIII. Subsequently, 1.5 kbp fragment, which was obtained by digesting the MCS of pBS(SK-)/NMDAr-N #3 with Xbal and blunting the ends thereof followed by digesting with AfIII, was introduced into the clone fj07108 digested with AfIII as described above to give pBS/NMDAr #2. pBS/NMDAr #2 is a vector that contains the full-length NMDA receptor/2B subunit.

The protein-coding region of the target gene was amplified using the Advantage HF2 PCR kit (Clontech) with pBS/NMDAr #2 as a template. The primers were designed and synthesized, based on the full-length nucleotide sequence of the NMDA receptor/28 subunit. PCR was performed by mixing 2.5 µl of 10× Advantage HF2 PCR buffer, 0.4 µl of Pr-2Bf (52.94 pmol/µl), 0.6 µl of Pr-2Br (37.44 pmol/µl), 0.2 µl of pBS/NMDAr #2 (0.48 µg/µl), 2.5 µl of dNTP mix, 0.5 µl of 50× polymerase mix, and 18.3 µl of H₂O (25 µl in total).

### Primers

Pr-2Bf (SEQ ID NO: 12):

5'-GGGGACAAGTTTGTACAAAAAAGCAGGCTTAATGAAGCCCAGAGCGGAG-3'

Pr-2Br (SEQ ID NO: 13):

5'-GGGGACCACTTTGTACAAGAAAGCTGGGTCCTGTTCCCTCACTCAGAC-3'

### Conditions for carrying out PCR

Pre-step (pre): 95°C for 1 minute
Step 1: 95°C for 30 seconds
Step 2: 58°C for 30 seconds
Step 3: 72°C for 5 minutes
(Steps 1-3 for 2 cycles)
Step 4: 95°C for 30 seconds
Step 5: 68°C for 5 minutes
(Steps 4 and 5 for 20 cycles)
Post-step (post): 68°C for 5 minutes

Then, the PCR product was subjected to electrophoresis (1% agarose gel). A band of the target gene of approximately 4.5 kbp was isolated. After that, it was extracted with a Gen elute EtBr minus spin column (SIGMA), purified, dried, and then dissolved in 10 µl of TE buffer. After adjusting to 100 µl with TE buffer, 50 µl of 30% PEG8000 / 30 mM MgCl₂ was added. The mixture was centrifuged at room temperature (at 15,000 rpm for 10 minutes), and then the pellet was washed with 70% ethanol, dried, and dissolved in 20 µl of TE buffer. Thereafter, 1 µl of BP clonase reaction buffer, 1 µl of the PCR product, 0.5 µl of entry vector (pDONR 201:150 ng/µl), and 1.5 µl of TE buffer were mixed on ice (4 µl in total); 1 µl of BP clonase enzyme mix was added thereto; and then the reaction was allowed to take place for 1.7 hours at 25°C. After the reaction, 0.5 µl of Proteinase K was added, and the reaction solution was incubated for 10 minutes at 37°C to inactivate the enzyme mix. Competent cells (DH5α) were transformed with 1 µl of this reaction solution, and pDONR 201/NMDA receptor #1 was obtained.

When the nucleotide sequence of pDONR 210/NMDA receptor #1 was determined, mutations that were believed to be caused by PCR were present at several positions, which includes mutations accompanied by amino acid substitutions. Therefore, pDONR 210/NMDA receptor #1 was digested with Mfel and Aatll to remove the mutation region, and a fragment of approximately 3.4 kbp resulting from a digestion of pBS/NMDAr #2 with Mfel and Aatll was introduced thereto to obtain pDONR 210/NMDA receptor #2. Since there were two positions in the protein-coding region derived from clone fj07108, where the nucleotides were different from those of the NMDA receptor/2B subunit registered in GenBank as Accession NO: NM_000843 (the base at 2664 bp from the initiation codon (C→T; Thr→Thr) and the base at 3499 bp (A→G; Ile→Val)), subcloning was performed by RT-PCR using human brain-derived mRNA and primer sets of the primers Pr-R2B-5 and Pr-NMDAr-R8, and of Pr-R2B-7 and Pr-NMDAr-R12. Then the nucleotide sequence of pBS/NMDAr #2 was determined. As a result, the nucleotide sequence obtained from the human brain-derived mRNA, through the RT-PCR subcloning, was revealed to be identical to that of clone fj07108.

### Primers

Pr-R2B-5 (SEQ ID NO: 14):

5'-TGAGAAGAATGAGGTCATGAGCA-3'

Pr-NMDAr-R8 (SEQ ID NO: 15):

5'-GTCACAGTCGTAGAGCCCTA-3'

Pr-R2B-7 (SEQ ID NO: 16):

5'-AGTTCCGAACAAAGGAGAACTCAC-3'

Pr-NMDAr-R12 (SEQ ID NO: 17):

5'-GAGTTCTGACCCGTCACCGTCGTGG-3'

Next, a vector expressing a fusion protein of an NMDA receptor/2B subunit with GST was constructed. After mixing 1 µl of LR clonase reaction buffer, 1 µl of pDONR 201/NMDA receptor #2 (50 ng/µl), 0.5µl of GST expression vector (pDEST15: 150 ng/µl), and 1.5 µl of TE buffer (4 µl in total) on ice, 1 µl of LR clonase enzyme mix was added thereto, and then the reaction was allowed to take place for 1 hour at 25°C. After the reaction, 0.5 µl of Proteinase K was added, and the reaction solution was incubated for 10 minutes at 37°C, to inactivate the enzyme mix. Competent cells (BL21-SI) were transformed with 1 µl of this reaction solution, and three clones (pDEST15/NMDA receptor #4, pDEST15/NMDA receptor #5, and pDEST15/NMDA receptor #6) were obtained.

The three clones were tested for induction by NaCl, for the production of the target protein. Each *Escherichia coli* clone was cultured overnight in 2 ml of LB-Amp (NaCl-) under agitation at 37°C. After culturing a total of 3 ml of LB-Amp (NaCl-), to which 300 µl of this pre-cultured solution had been added, under agitation, for 4 hours at 37°C, 180 µl of 5 M NaCl was added thereto (a final concentration of 0.3 M), and cultured under agitation for a further 2 hours at 37°C. As a control, 180 µl of H₂O was added, and the culture proceeded in the same way. Thereafter, the cultured solution was centrifuged (at 15,000 rpm for 10 minutes at 4°C). The pellet was suspended in 200 µl of 2% SDS / 20 mM Tris (pH 7.4), sonicated, and centrifuged (at 15,000 rpm for 10 minutes at 10°C). The supernatant of the centrifugation was mixed with an equal volume of 2× sample buffer containing 10% β-ME. The mixture was boiled for 2 minutes and subjected to SDS-PAGE over a 5%-20% polyacrylamide gel. The target protein was detected using an anti-GST antibody in the same way as described above. The results shown in Fig. 12, a detected band at approximately 189.2 kDa, which is expected to be a fusion protein of the NMDA receptor/2B subunit with GST.

### Example 4

### Functional analysis of the novel PSD (Protein-X) in the context of the NMDA receptor

Protein-X was co-expressed with an NMDA receptor in a *Xenopus* oocyte, and the current response to the stimulation of the NMDA receptor by a ligand was measured to examine the action of Protein-X on the signal of the NMDA receptor.

The following plasmids were used in the present test.

### Plasmid DNA containing the novel PSD gene (Protein-X gene)

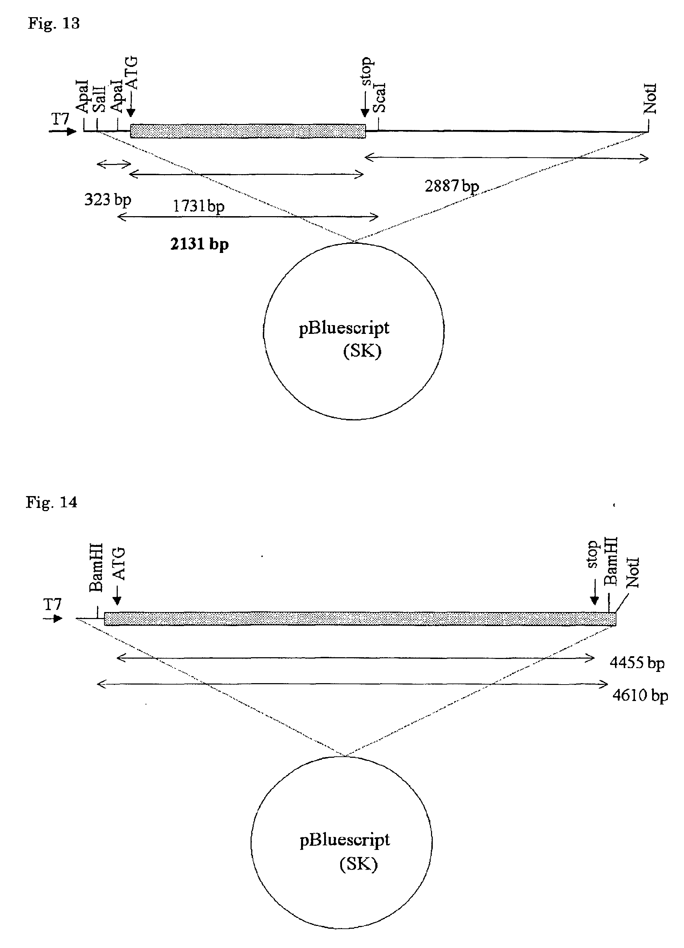

The cDNA corresponding to clone hj02537 extracted in Example 1 was selected from a cDNA library prepared from the human immature myeloid cell line KG-1 according to a method described in the literature (Nomura, N., et al., DNA Research, 1994, Vol. 1, pp. 27-35), and used (Fig. 13). To date, no information regarding the sequence of this clone has been disclosed.

### Plasmid DNA containing the NMDA receptor/2B subunit gene

pBS/NMDAr prepared in Example 3 was used (Fig. 14).

### Plasmid DNA containing the NMDA receptor I gene

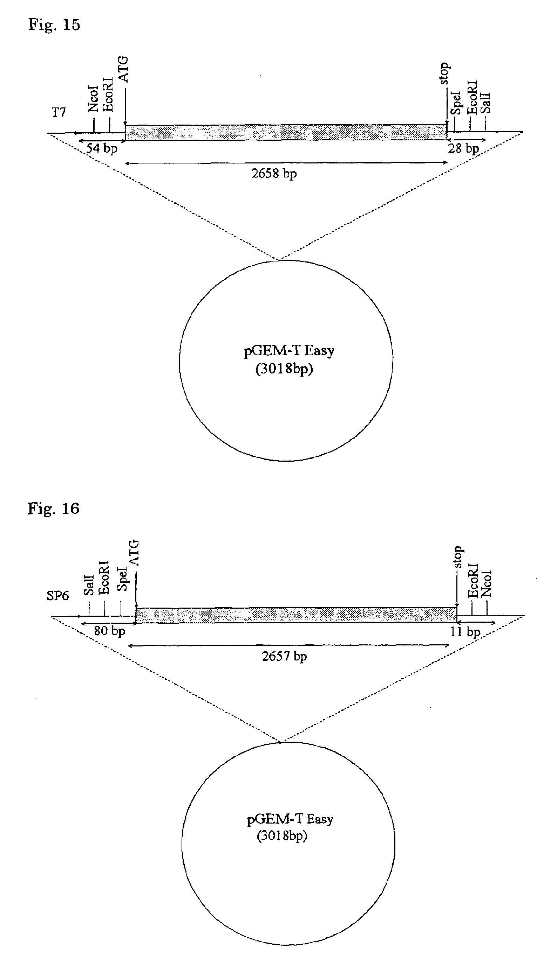

PCR was carried out using an Advantage 2 PCR kit in the same way as in Example 1, using a template cDNA that was reverse-transcribed from human brain-derived mRNA, using superscript II with an oligo(dT) primer and random primers, and PCR primers that were designed and synthesized based on the nucleotide sequence of the NMDA receptor I. Thereafter, using the obtained PCR product, the pGEM-T Easy Vector systems (Promega) was used to carry out TA cloning, and a pGEM-T Easy vector was obtained (Fig. 15). The vector retains a 2658 bps translated region of the NMDA receptor I between a position of 2 bp (CC) downstream from EcoRI, and a position of 2 bp (GG) upstream from Spel.

### Plasmid DNA containing the PSD-95 gene

PCR was carried out using an Advantage 2 PCR kit in the same way as in Example 1, using a template cDNA that was prepared from human brain-derived mRNAs in the same way as described above, and PCR primers that were designed and synthesized based on the nucleotide sequence of the PSD-95 gene. Thereafter, using the obtained PCR product, pGEM-T Easy Vector systems (Promega) was used to carry out TA cloning, and the pGEM-T Easy vector was obtained (Fig. 16). The vector retains a 2657 bp translated region of PSD-95 between a position of 3 bp (GAG) downstream from Spel, and a position of 4 bp (TTCC) upstream from EcoRl.

First, the plasmid DNA containing the NMDA receptor I gene was treated with Sall, while the plasmid DNAs containing other genes were treated with Notl, leading to linearization of the plasmids, and then phenol/chloroform purification was performed. Using the obtained DNA as a template, a MEGAscript kit (Ambion) was used according to the product instructions to carry out RNA synthesis reaction. In so doing, SP6 was used as an RNA polymerase for the PSD-95 gene, and T7 was used for other genes. After completion of the RNA synthesis reaction, the template DNA was removed by DNAase treatment. After the RNA synthesis reaction product was verified by 1% agarose gel electrophoresis, phenol/chloroform purification was performed. The synthesized RNA, after purification, was dissolved in sterile water, and prepared so as to obtain a total RNA concentration of 1 µg/µL. The RNA concentration was determined by optical density.

The Xenopus was purchased from Shiki leda Chemicals Co., Ltd., fatted for several weeks, and then oocytes were extracted. After the oocytes were treated with collagenase and then cultured overnight, the NMDA receptor RNA prepared as described above was micro-injected thereto by a well-known method.

In so doing, the NMDA receptor/2B subunit RNA and NMDA receptor I RNA were mixed with a ratio of 1:2 and prepared so as to give 20 ng per cell, and used. After injection, the oocytes were incubated for 48 hours at 20°C in a medium (115 mM NaCl, 2.5 mM KCI, 1.8 mM BaCl₂, and 10 mM HEPES, pH 7.2).

When co-expressing Protein-X or PSD-95 with the NMDA receptor, the NMDA receptor RNA was injected in the same way as described above, and then 10 ng per cell of Protein-X RNA or PSD-95 RNA was re-injected after 24 hours. Thereafter, incubation proceeded in a culture medium for 24 hours at 20°C.

Ligands were made to act on the NMDA receptor expressed in the oocytes; the influx of cations arising from the opening of the NMDA receptor ion channel as a result thereof was measured as a variation of the current by an electrophysiological measurement method. Herein, the measurements were carried out by the two-electrode voltage clamp method according to a reference article (FEBS Letter, 1999, Vol. 458, pp. 295-298). The BA buffer (115 mM NaCl, 2.5 mM KCI, 1.8 mM BaCl₂, and 10 mM HEPES, pH 7.2) was used as the measurement buffer, and the membrane potential was fixed at -70 mV.

A solution mixture of L-glutamic acid (Glu) and glycine (Gly) was used for ligands, which were made to act on the NMDA receptor by direct instillation to the oocytes. In addition, when quantitating the magnitude of the current, the value obtained by subtracting the mean value of the leak current prior to the stimulation from the maximum value of the inward current after stimulation by the ligands (hereinafter referred to as current variation), was used. The measurement of the current response was carried out using 5 to 6 cells per experiment. Within the same experiment, large differences in the waveform patterns due to the current responses were not observed between each cell.

First, different concentrations of ligands (10 µM Glu+10 µM Gly, 100 µM Glu+10 µM Gly, and 1000 µM Glu + 10 µM Gly) were made to act on the oocytes into which the NMDA receptor RNA was injected to verify ligand concentration dependency. As a result it was demonstrated that the experimental system was adequate for the measurement of the NMDA receptor signal. In the present experiment, after the ligand solution was added, no removal by washing was performed, which made the wave form pattern different from the one in the reference bibliography (FEBS Letter, 1999, Vol. 458, pp. 295-298), and the inward current tended not to be transient, in particular when stimulation was performed at high concentration of ligands. This is believed to be because the ligands and the receptor stayed bound to each other, which kept the NMDA receptor channel open, resulting in the continuation of the cationic influx.

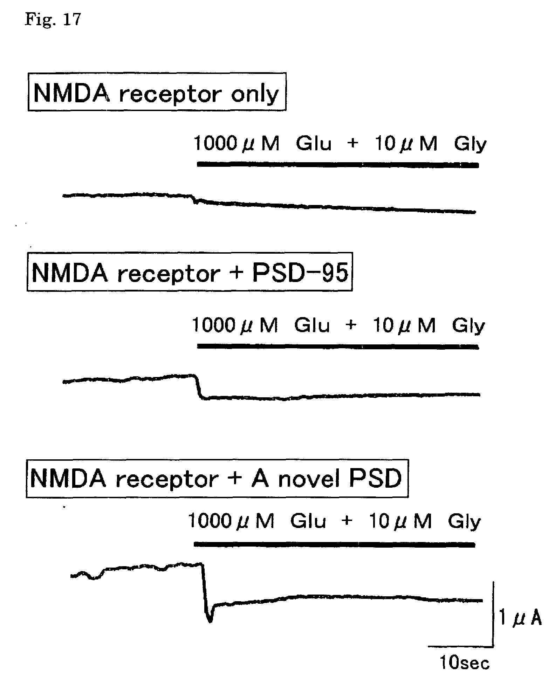

Next, a solution mixture of 1000 µM Glu and 10 µM Gly was used as ligands, to measure the current response in oocytes into which Protein-X RNA or PSD-95 RNA was co-injected with NMDA receptor RNA. The results are shown in Fig. 17 and Table 1. When Protein-X or PSD-95 was co-expressed with the NMDA receptor, it was observed that the current response to the ligand stimulation tended to be greater than that when the NMDA receptor was expressed by itself. In addition, when Protein-X was expressed, a current response approximately 1.5 times greater than that when PSD-95 was expressed was observed.

**Table 1**

| | Current variation (µA) | | |
|---|---|---|---|
| | NMDA-R | NMDA-R | NMDA-R |
| | | PSD-95 | Protein-X |
| #1 | 0.17 | 0.50 | 0.60 |
| #2 | 0.07 | 0.27 | 0.81 |
| #3 | 0.06 | 0.32 | 0.38 |
| #4 | 0.13 | 0.31 | 0.57 |
| #5 | 0.23 | 0.48 | 0.41 |
| #6 | -- | -- | 0.54 |
| Average | 0.13 | 0.38 | 0.55 |
| Standard deviation | 0.07 | 0.11 | 0.15 |

From the results of the above electrophysiological measurements, it was revealed that Protein-X forms an apparatus by binding to an NMDA receptor/2B receptor, and that Protein-X is involved in the signal generated by the stimulation of the NMDA receptor, for instance, opening of the receptor ion channel and the accompanying cationic influx, and in the promotion thereof.

### Example 5

### Isolation/identification of the PJ01087 gene (Protein-Y gene)

The gene of the present invention was isolated and identified based on clone PJ01087, which was extracted by using the human brain-derived long-strand cDNA analysis data (bioinformatics) of the Kazusa DNA Research Institute, as a gene having 96% homology to the rat PSD95/SAP90-associated protein 3 (SAPAP-3).

First, DHα competent cells were transformed with a pBluescript SK+ vector containing clone PJ01087 for amplification, and the length of the insert resulting from a Notl/Sal1 cut was verified to be approximately 3.7 kb. PCR was performed with this as the template, using the primers described below. The primers were designed and synthesized based on the nucleotide sequence of PJ01087, obtained from the information database described above. The PCR was carried out using an Advantage 2 PCR kit (Clontech) according to the user's manual.

### Primers

Forward primer (SEQ ID NO: 18):

5'-GGGGACAAGTTTGTACAAAAAAGCAGGCTTAATGAGGGGTTACCATGGC-3'

Reverse primer (SEQ ID NO: 19):

5'-GGGGACCACTTTGTACAAGAAAGCTGGGCTAGTACGGGTGGAGAACCG-3'

As a result, a PCR product of approximately 2.9 kb containing the full-length ORF of the PJ01087 clone was obtained. The PCR product was cloned into the entry vector pDONR™ 201, using the Gate way system (Invitrogen). The cloning was carried out according to the user's manual of the same system.

### Expression of the PJ01087 gene (the Protein-Y gene)

Absence of error in the entire length of the nucleotide sequence of the insert portion (approximately 2.9 kb) was verified in the obtained vector. Thereafter, recombination into expression vector pDEST™ 17, that makes a protein possible to be expressed with a His-tag at the N-terminus in Escherichia coli, and into expression vector pDEST™ 15, that makes a protein possible to be expressed with GST-tag in Escherichia coli, was performed. The recombination was carried out using the Gate way system (Invitrogen), according to the user's manual.

Induction of the expression of protein coded by the Protein-Y gene was carried out in Escherichia coli transformed with pDEST™ 17 or pDEST™ 15 that contains Protein-Y gene. Pre-culture was performed under agitation at 37°C in an LB medium containing ampicillin, until OD600 reached 0.5 to 1.0. NaCl was then added to 300 µl of the pre-cultured solution to obtain a final concentration of 0.3 M (NaCl(+)), and culture under agitation proceeded for 4 hours at 37°C to induce protein expression. As a control, H2O was added instead of NaCl, and culturing proceeded in the same way (NaCl(-)). Thereafter, each cultured solution was centrifuged to recover the bacteria. The bacteria were suspended in phosphate-buffered physiological saline (PBS), sonicated three times for 10 seconds, centrifuged again (at 15,000 rpm for 10 minutes at 10°C or at 4°C), and separated into a supernatant (hereinafter referred to as soluble fraction) and a pellet. The pellet was re-suspended in PBS (referred to as insoluble fraction).

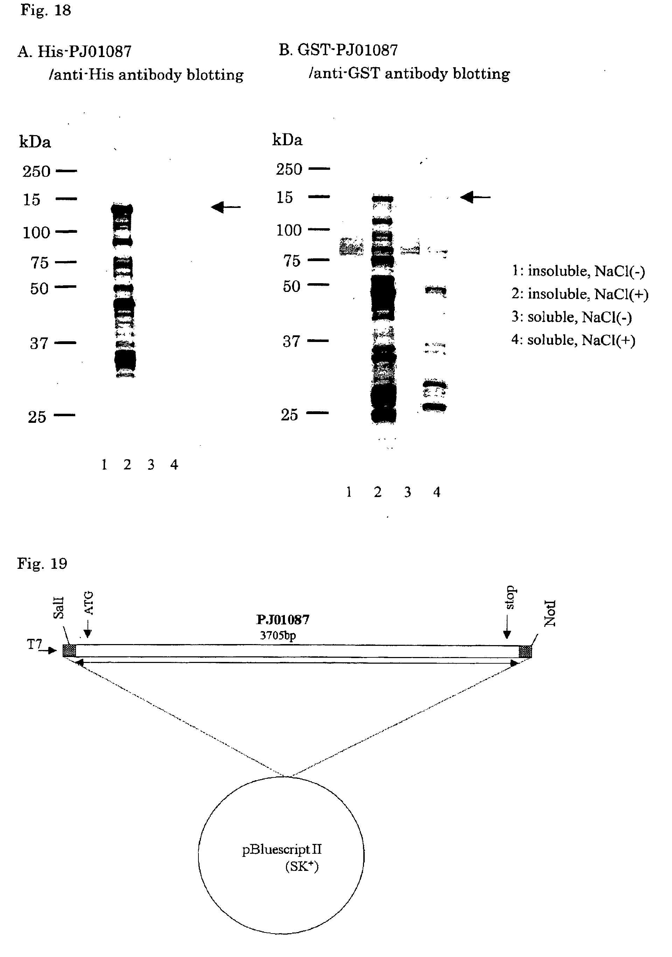

For each fraction, the protein was detected by Western blotting using antibodies against His-Tag and GST-Tag. The results show that in both tests using His-tag and GST-tag, an expression of approximately 115 kDa of the target protein and other proteins believed to be the decomposition products thereof were observed in the insoluble fraction of the Escherichia coli induced with NaCl (Figs. 18A and 18B). One cell-free protein expression system, Rapid Translation System RTS5000 (Roche Diagnostics), which is based on the protein expression system of Escherichia coli, was used to examine protein expression; however the target protein was still found in the insoluble fraction.

The human protein thus obtained comprises 979 amino acid residues as set forth in SEQ ID NO: 3 in the Sequence Listing. In addition, the gene coding for the protein comprises 3705 bases as set forth in SEQ ID NO: 6 in the Sequence Listing.

### Example 6

### Functional analysis of the PJ01087 gene product (the Protein-Y gene product) in the context of an NMDA receptor

The Protein-Y gene has 96% homology to SAPAP-3. Since SAPAP-3 is a protein related to rat PSD-95/SAP90, it was anticipated that the Protein-Y gene product would form a complex with human PSD-95 and would be involved in the constitution of the postsynaptic density. Since PSD-95 has been reported to be involved in the NMDA receptor mediated signal transduction, the Protein-Y gene and the PSD-95 gene were co-expressed with the NMDA receptor gene in Xenopus oocytes, and then the current response to the stimulation of the NMDA receptor by a ligand was measured to examine the effects of Protein-Y on the signal of the NMDA receptor. In so doing, a gene (SEQ ID NO: 4 in the Sequence Listing) coding for the novel PSD protein (Protein-X) (SEQ ID NO: 1 in the Sequence Listing) was used instead of PSD-95 and examined in the same way as above. Protein-X possesses a PDZ domain, an SH3 domain, and a GK domain as PSD-95 does, and was inferred to bind to an NMDA receptor resulting in signal transduction. The method used in the test was identical to the method described in Example 4.

The following plasmids were used in the present test.

### Plasmid DNA containing the PJ01087 gene (the Protein-Y gene)

The cDNA, corresponding to clone PJ01087 extracted in Example 5, was selected from a cDNA library prepared from the human immature myeloid cell line KG-1 according to a method described in the literature (Nomura, N., et al., DNA Research, 1994, Vol. 1, pp. 27-35), and was used in this experiment (Fig. 19).

### Plasmid DNA containing the Protein-X gene

The plasmid DNA described in Example 4 was used (Fig. 13).

### Plasmid DNA containing the NMDA receptor/2B subunit gene

The plasmid pBS/NMDAr (Fig. 14) prepared in Example 3 was used.

### Plasmid DNA containing the NMDA receptor I gene

The plasmid DNA prepared in Example 4 was used (Fig. 15).

### Plasmid DNA containing the PSD-95 gene

The plasmid DNA prepared in Example 4 was used (Fig. 16).

The effects of Protein-X and Protein-Y on the signal of the NMDA receptor were tested by the same method as in Example 4.

When co-expressing Protein-Y, PSD-95, and/or Protein-X with the NMDA receptor, the NMDA receptor RNA was injected into Xenopus oocytes in the same way as described in Example 4, and then 10 ng RNA per cell of Protein-Y, PSD-95, and/or Protein-X was re-injected after 24 hours. Thereafter, incubation proceeded in a culture medium for 24 hours at 20°C.

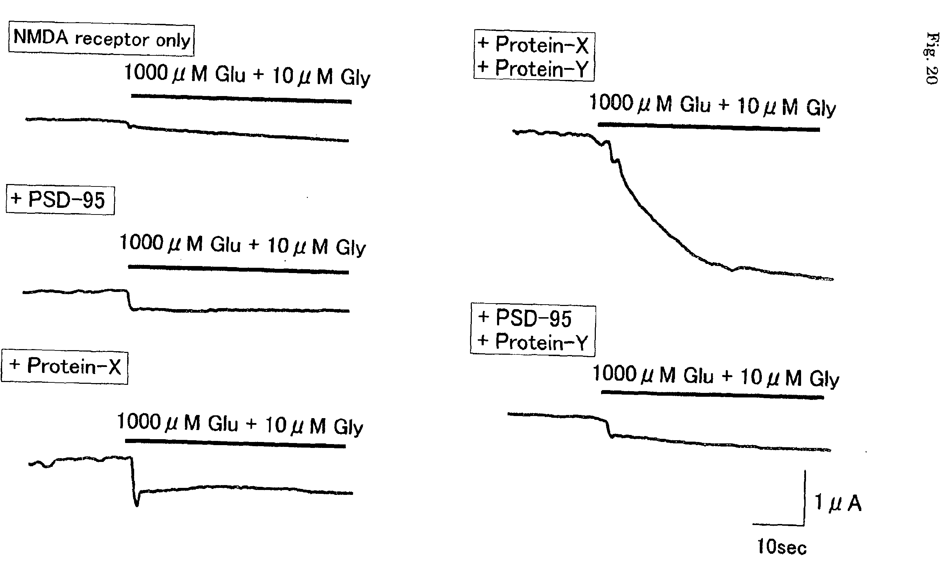

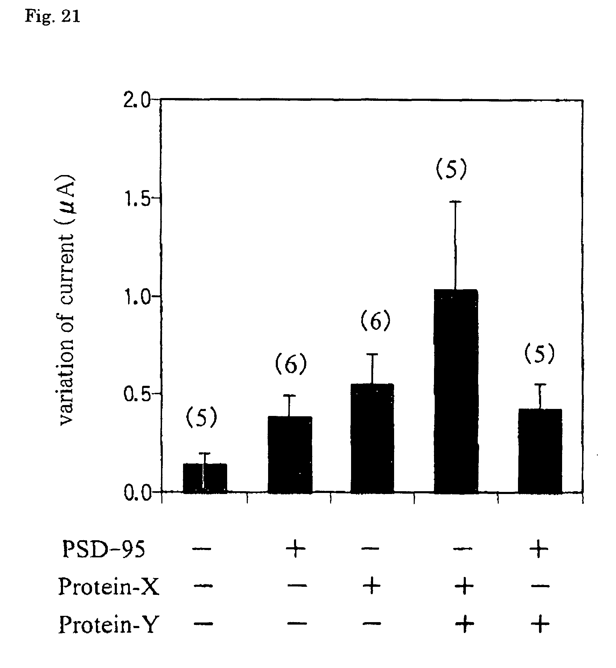

Ligands were made to act on the NMDA receptor expressed in the oocytes, and the influx of cations arising from the opening of the NMDA receptor ion channel as a result thereof was measured by the same method as described in Example 4. The results are shown in Figs. 20 and 21. As shown in Example 4, by co-expressing PSD-95 or Protein-X with the NMDA receptor, the current response to the ligand stimulation was observed to be greater than that when the NMDA receptor was expressed by itself. In addition, even by co-expressing Protein-Y and PSD-95 with an NMDA receptor, the current response did not differ from that observed when the PSD-95 was expressed. Meanwhile, when Protein-Y was co-expressed with Protein-X, a current response approximately seven to eight times greater than that when only Protein-X was expressed was obtained.

From the results of the above electrophysiological measurements, it was revealed that Protein-Y markedly amplifies the NMDA receptor mediated signal transduction in the presence of Protein-X. From the foregoing, it was revealed that Protein-Y amplifies the intracellular signal generated by the stimulation of the NMDA receptor, by an interaction with Protein-X that possesses a PDZ domain and is capable of binding to an NMDA receptor/2B subunit. Furthermore, it was revealed that it is involved in the opening of the NMDA receptor ion channel and the promotion of the accompanying cation influx.

### Example 7

### Presence or absence of binding of the PJ01087 Protein (Protein-Y) to Protein-X

The following proteins were prepared to test the binding of Protein-Y to Protein-X by immunoprecipitation.

### PJ01087 protein (Protein-Y)

200 µl of 1% Triton-X / 20 mM Tris (pH 7.4) was added to a pellet corresponding to 2 ml culture (see Example 5) of Escherichia coli wherein expression of the protein was carried out as a fusion protein with a His-tag. After sonication, centrifugation was performed for 5 minutes at 15,000 rpm, and the supernatant thereof was used as the sample (His-PJ01087).

### Protein-X

200 µl of 1% Triton-X / 20 mM Tris (pH 7.4) was added to a pellet corresponding to 2 ml culture of Escherichia coli, wherein expression of a fusion protein of Protein-X with GST was carried out using clone hj02537 described in Example 1 by the same method as in Example 5,. After sonication, centrifugation was performed at 15,000 rpm for 5 minutes and the supernatant thereof was used as a sample (GST-hj02537). In addition, using Escherichia coli wherein a fusion protein of a mutant resulting from the deletion of the guanylate kinase domain from Protein-X with GST was expressed, the mutant protein was prepared in the same way (GST-hj(GK-)).

### GST

As a control, GST alone was expressed, and a sample was prepared in the same way (derived from pGEX-4T-3, Amersham Biosciences).

### Treatment of each protein sample

To each of the aforementioned supernatants, 30 µl of Protein G-Sepharose equilibrated with 1% Triton-X / 20 mM Tris (pH 7.4) was added, and then agitated for 1 hour at 4°C. After agitation, the samples were centrifuged, and the supernatants thereof were used for binding assays.

### Binding assay

Each of the supernatants was dispensed as in (1 )-(4), adjusted to 200 µl with 1% Triton-X / 20 mM Tris (pH 7.4) and agitated overnight at 4°C.
(1) 25 µl His-PJ01087 + 16 µl GST-hj02537
(2) 25 µl His-PJ01087 + 100 µl GST-hj(GK-)
(3) 25 µl His-PJ01087 + 4 µl GST
(4) 25 µl His-PJ01087

After adding 1 µl of anti-GST antibody (SIGMA) and agitating for 1 hour at 4°C, a further 30 µl of Protein G-sepharose was added thereto, and agitation proceeded for an additional 6 hours at 4°C. Thereafter, the supernatant was removed by centrifugation, and Protein G-sepharose was washed three times with 500 µl of 1% Triton-X / 20 mM Tris (pH 7.4). After the washes, 30 µl of 2× sample buffer (2% SDS / 50 mM Tris (pH 6.8) / 30% glycerol / 0.01% bromophenol blue) containing 2-mercaptoethanol was added to Protein G-sepharose and mixed. The samples were boiled for 2 minutes, subjected to electrophoresis on a 5%-20% polyacrylamide gel, and transferred onto a PVDF membrane. Then Western blotting was performed with anti-GST antibodies and anti-His-tag antibodies.

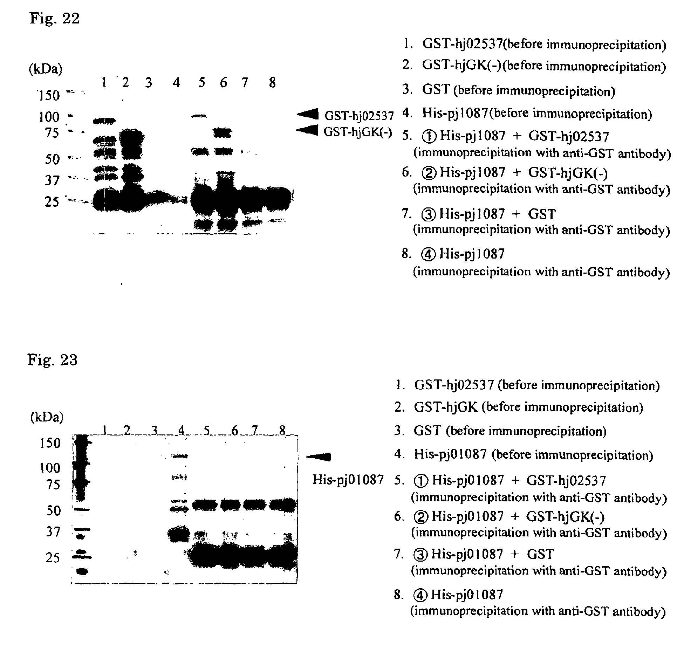

The result shows that Protein-X (GST-hj02537) and the Protein-X mutant (GST-hj (GK-)) were adsorbed onto Protein G-sepharose (Lanes 5 and 6 in Fig. 22). However, when Protein-Y (His-PJ01087) was reacted with Protein-X (GST-hj02537) or the Protein-X mutant (GST-hj (GK-)) to perform immunoprecipitation with an anti-GST antibody followed by detecting with an anti-His-tag antibody, no band showing Protein-Y was detected. Thus, no result showing that binding of Protein-Y to Protein-X exists could be obtained (lanes 5 or 6 in Fig. 23).

### Possibilities for Industrial Use

Clone hj02537 was extracted as a gene having a PDZ domain, an SH3 domain and a GK domain, by way of bioinformatics from the human brain-derived long-strand cDNA library analysis information database of the Kazusa DNA Research Institute, and the gene product (SEQ ID NO: 1) of this gene (SEQ ID NO: 4) was found to be a novel protein (Protein-X), which binds to an NMDA receptor/2B subunit. Protein-X is believed to be, from the characteristics of the amino acid sequence thereof, of a guanylate kinase family, which is cell membrane associated proteins, as is PSD-95. In addition, Protein-X was found to be involved in the signal arising from the stimulation of the NMDA receptor, and to have a promoting activity thereof.

Similarly, clone PJ01087 was extracted as a gene having 96% homology to the rat PSD95/SAP90-associated protein 3 (SAPAP-3). Then, the gene product (SEQ ID NO: 3) of this gene (SEQ ID NO: 6) was found to markedly amplify the signal transduced from the NMDA receptor, by interacting with Protein-X (SEQ ID NO: 1). The NMDA receptor mediated signal transduction was amplified more strongly in the presence of both Protein-X and Protein-Y than in the presence of Protein-X alone.

From the foregoing, the present invention is extremely useful for enabling the elucidation of biological functions or diseases in which Protein-X and Protein-Y, as well as the genes thereof, are involved. For example, it is useful for elucidation of the mechanism of an NMDA receptor mediated signal transduction, and elucidation of the mechanism of memory recall. Furthermore, it is useful for elucidation of diseases caused by an anomaly in the NMDA receptor mediated signal transduction or an anomaly in memory recollection, for example neurodegenerative diseases such as Alzheimer's disease, Parkinson's disease, and polyglutamine disease, as well as for prevention, treatment, or improvement of these diseases.

### SEQUENCE LISTING FREE TEXT

SEQ ID NO: 7 in the Sequence Listing: designed polynucleotide based on the sequence set forth in SEQ ID NO: 4 to be used as a primer
SEQ ID NO: 8 in the Sequence Listing: designed polynucleotide based on the sequence set forth in SEQ ID NO: 4 to be used as a primer
SEQ ID NO: 9 in the Sequence Listing: designed polynucleotide based on the sequence set forth in SEQ ID NO: 4 to be used as a primer
SEQ ID NO: 10 in the Sequence Listing: designed polynucleotide based on the partial nucleotide sequence of the NMDA receptor/2B subunit to be used as a primer
SEQ ID NO: 11 in the Sequence Listing: designed polynucleotide based on the partial nucleotide sequence of the NMDA receptor/2B subunit to be used as a primer
SEQ ID NO: 12 in the Sequence Listing: designed polynucleotide based on the nucleotide sequence of the NMDA receptor/2B subunit to be used as a primer
SEQ ID NO: 13 in the Sequence Listing: designed polynucleotide based on the nucleotide sequence of the NMDA receptor/2B subunit to be used as a primer
SEQ ID NO: 14 in the Sequence Listing: designed polynucleotide based on the nucleotide sequence of the NMDA receptor/2B subunit to be used as a primer
SEQ ID NO: 15 in the Sequence Listing: designed polynucleotide based on the nucleotide sequence of the NMDA receptor/2B subunit to be used as a primer
SEQ ID NO: 16 in the Sequence Listing: designed polynucleotide based on the nucleotide sequence of the NMDA receptor/2B subunit to be used as a primer
SEQ ID NO: 17 in the Sequence Listing: designed polynucleotide based on the nucleotide sequence of the NMDA receptor/2B subunit to be used as a primer
SEQ ID NO: 18 in the Sequence Listing: designed polynucleotide based on the sequence set forth in SEQ ID NO: 6 to be used as a primer
SEQ ID NO: 19 in the Sequence Listing: designed polynucleotide based on the sequence set forth in SEQ ID NO: 6 to be used as a primer

## Claims

1. An agent for controlling N-methyl-D-aspartate receptor mediated signal transduction, wherein the agent inhibits or promotes the binding of a polypeptide having the amino acid sequence set forth in SEQ ID NO: 1 in the Sequence Listing, to the N-methyl-D-aspartate receptor, and/or inhibits or promotes the interaction of a polypeptide having the amino acid sequence set forth in SEQ ID NO: 3 in the Sequence Listing, with the polypeptide having the amino acid sequence set forth in SEQ ID NO: 1 in the Sequence Listing.

2. An agent for inhibiting N-methyl-D-aspartate receptor mediated signal transduction, wherein the agent inhibits binding of a polypeptide having the amino acid sequence set forth in SEQ ID NO: 1 in the Sequence Listing, to the N-methyl-D-aspartate receptor, and/or inhibits interaction of a polypeptide having the amino acid sequence set forth in SEQ ID NO: 3 in the Sequence Listing, with the polypeptide having the amino acid sequence set forth in SEQ ID NO: 1 in the Sequence Listing.

3. An agent for enhancing N-methyl-D-aspartate receptor mediated signal transduction, wherein the agent promotes the binding of a polypeptide having the amino acid sequence set forth in SEQ ID NO: 1 in the Sequence Listing, to the N-methyl-D-aspartate receptor, and/or promotes the interaction of a polypeptide having the amino acid sequence set forth in SEQ ID NO: 3 in the Sequence Listing, with the polypeptide having the amino acid sequence set forth in SEQ ID NO: 1 in the Sequence Listing.

4. A method for controlling N-methyl-D-aspartate receptor mediated signal transduction, wherein the method comprises inhibiting or promoting the binding of a polypeptide having the amino acid sequence set forth in SEQ ID NO: 1 in the Sequence Listing, to the N-methyl-D-aspartate receptor, and/or inhibiting or promoting the interaction of the polypeptide having the amino acid sequence set forth in SEQ ID NO: 3 in the Sequence Listing, with the polypeptide having the amino acid sequence set forth in SEQ ID NO: 1 in the Sequence Listing.

5. A method for inhibiting N-methyl-D-aspartate receptor mediated signal transduction, wherein the method comprises inhibiting the binding of a polypeptide having the amino acid sequence set forth in SEQ ID NO: 1 in the Sequence Listing, to the N-methyl-D-aspartate receptor, and/or inhibiting the interaction of a polypeptide having the amino acid sequence set forth in SEQ ID NO: 3 in the Sequence Listing, with the polypeptide having the amino acid sequence set forth in SEQ ID NO: 1 in the Sequence Listing.

6. A method for promoting N-methy!-D-aspartate receptor mediated signal transduction, wherein the method comprises promoting the binding of a polypeptide having the amino acid sequence set forth in SEQ ID NO: 1 in the Sequence Listing, to the N-methyl-D-aspartate receptor, and/or by promoting the interaction of a polypeptide having the amino acid sequence set forth in SEQ ID NO: 3 in the Sequence Listing, with the polypeptide having the amino acid sequence set forth in SEQ ID NO: 1 in the Sequence Listing.

7. A polypeptide selected from the following polypeptides:
i. a polypeptide having the amino acid sequence set forth in SEQ ID NO: 1 or 2 in the Sequence Listing;
ii. a polypeptide containing the polypeptide set forth above in (i);
iii. a polypeptide having at least approximately 70% homology to the polypeptide set forth above in (i) at the amino acid sequence level and binding to an N-methyl-D-aspartate receptor/2B subunit; and
iv. a polypeptide having a mutation, which is a deletion, a substitution, an addition, or an insertion of one to several amino acids in the amino acid sequence of the polypeptide set forth above in (i), and binding to the N-methyl-D-aspartate receptor/2B subunit.

8. A polypeptide selected from the following polypeptides, wherein the polypeptide binds to an N-methyl-D-aspartate receptor/2B subunit:
i. a polypeptide having the amino acid sequence set forth in SEQ ID NO: 1 or 2 in the Sequence Listing;
ii. a polypeptide containing the polypeptide set forth above in (i);
iii. a polypeptide having at least approximately 70% homology to the polypeptide set forth above in (i) at the amino acid sequence level; and
iv. a polypeptide having a mutation, which is a deletion, a substitution, an addition, or an insertion of one to several amino acids in the amino acid sequence of the polypeptide set forth above in (i).

9. A polypeptide selected from the following polypeptides:
i. a polypeptide having the amino acid sequence set forth in SEQ ID NO: 3 in the Sequence Listing;
ii. a polypeptide containing the polypeptide set forth above in (i);
iii. a polypeptide having at least approximately 70% homology to the polypeptide set forth above in (i) at the amino acid sequence level and interacting with a polypeptide having the amino acid sequence set forth in SEQ ID NO: 1 in the Sequence Listing; and
iv. a polypeptide having a mutation, which is a deletion, a substitution, an addition, or an insertion of one to several amino acids in the amino acid sequence of the polypeptide set forth above in (i) and interacting with the polypeptide having the amino acid sequence set forth in SEQ ID NO: 1 in the Sequence Listing.

10. A polypeptide selected from the following polypeptides, wherein the polypeptide interacts with a polypeptide having the amino acid sequence set forth in SEQ ID NO: 1 in the Sequence Listing:
i. a polypeptide having the amino acid sequence set forth in SEQ ID NO: 3 in the Sequence Listing;
ii. a polypeptide containing the polypeptide set forth above in (i);
iii. a polypeptide having at least approximately 70% homology to the polypeptide set forth above in (i) at the amino acid sequence level; and
iv. a polypeptide having a mutation, which is a deletion, a substitution, an addition, or an insertion of one to several amino acids in the amino acid sequence of the polypeptide set forth above in (i).

11. A polypeptide selected from the following polypeptides, wherein the polypeptide amplifies N-methyl-D-aspartate receptor mediated signal transduction in the presence of a polypeptide having the amino acid sequence set forth in SEQ ID NO: 1 in the Sequence Listing:
i. a polypeptide having the amino acid sequence set forth in SEQ ID NO: 3 in the Sequence Listing;
ii. a polypeptide containing the polypeptide set forth above in (i);
iii. a polypeptide having at least approximately 70% homology to the polypeptide set forth above in (i) at the amino acid sequence level; and
iv. a polypeptide having a mutation, which is a deletion, a substitution, an addition, or an insertion of one to several amino acids in the amino acid sequence of the polypeptide set forth above in (i).

12. A polypeptide, which binds to an N-methyl-D-aspartate receptor/2B subunit and does not interact with a polypeptide having the amino acid sequence set forth in SEQ ID NO: 3 in the Sequence Listing, wherein the polypeptide is:
i. a polypeptide having at least approximately 70% homology to a polypeptide having the amino acid sequence set forth in SEQ ID NO: 1 or 2 in the Sequence Listing at the amino acid sequence level; or
ii. a polypeptide having a mutation, which is a deletion, a substitution, an addition, or an insertion of one to several amino acids in the amino acid sequence of the polypeptide set forth above in (i).

13. A peptide comprising at least 5 consecutive amino acid residues within the amino acid sequence set forth in SEQ ID NO: 1 or 2 in the Sequence Listing.

14. A peptide comprising at least 5 consecutive amino acid residues within the amino acid sequence set forth in SEQ ID NO: 1 or 2 in the Sequence Listing and binding to an N-methyl-D-aspartate receptor.

15. A peptide comprising at least 5 consecutive amino acid residues within the amino acid sequence set forth in SEQ ID NO: 1 in the Sequence Listing, and interacting with a polypeptide having the amino acid sequence set forth in SEQ ID NO: 3 in the Sequence Listing.

16. A peptide comprising at least 5 consecutive amino acid residues within the amino acid sequence set forth in SEQ ID NO: 3 in the Sequence Listing.

17. A peptide comprising at least 5 consecutive amino acid residues within the amino acid sequence set forth in SEQ ID NO: 3 in the Sequence Listing and interacting with a polypeptide having the amino acid sequence set forth in SEQ ID NO: 1 in the Sequence Listing.

18. The agent for controlling N-methyl-D-aspartate receptor mediated signal transduction according to Claim 1, wherein the agent comprises an effective dose of at least one kind of polypeptide or peptide selected from the polypeptides or peptides according to Claim 7 to Claim 17.

19. The agent for inhibiting N-methyl-D-aspartate receptor mediated signal transduction according to Claim 2, wherein the agent comprises an effective dose of at least one kind of polypeptide or peptide selected from the polypeptide according to Claim 12 and the peptides according to any one of Claims 13 to 17.

20. The agent for promoting N-methyl-D-aspartate receptor mediated signal transduction according to Claim 3, wherein the agent comprises an effective dose of at least one kind of polypeptide or peptide selected from the polypeptides according to any one of Claim 7 to Claim 11 and the peptides according to any one of Claims 13 to 17.

21. The method for controlling N-methyl-D-aspartate receptor mediated signal transduction according to Claim 4, wherein the method comprises using at least one kind of polypeptide or peptide selected from the polypeptides or peptides according to Claim 7 to Claim 17.

22. The method for inhibiting N-methyl-D-aspartate receptor mediated signal transduction according to Claim 5, wherein the method comprises using at least one kind of polypeptide or peptide selected from the polypeptide according to Claim 12 and the peptides according to any one of Claims 13 to 17.

23. The method for promoting N-methyl-D-aspartate receptor mediated signal transduction according to Claim 6, wherein the method comprises using at least one kind of polypeptide or peptide selected from the polypeptides according to any one of Claim 7 to Claim 11 and the peptides according to any one of Claims 13 to 17.

24. A polynucleotide comprising a nucleotide sequence that codes for the polypeptide according to any one of Claims 7, 8, and 12 or for the peptide according to any one of Claims 13 to 15, or a complementary nucleotide sequence thereof.

25. A polynucleotide having the nucleotide sequence set forth in SEQ ID NO: 4, or SEQ ID NO: 5 in the Sequence Listing, or a complementary nucleotide sequence thereof.

26. A polynucleotide that hybridizes to the polynucleotide according to Claim 24 or Claim 25 under stringent conditions.

27. A polynucleotide comprising a nucleotide sequence that codes for the polypeptide according to any one of Claims 9 to 11 or for the peptide according to Claim 16 or Claim 17, or a complementary nucleotide sequence thereof.

28. A polynucleotide having a nucleotide sequence set forth in SEQ ID NO: 6 in the Sequence Listing, or a complementary nucleotide sequence thereof.

29. A polynucleotide that hybridizes to the polynucleotide according to Claim 27 or Claim 28 under stringent conditions.

30. A recombinant vector containing the polynucleotide according to any one of Claims 24 to 26.

31. The recombinant vector according to Claim 30, wherein the recombinant vector is a recombinant expression vector.

32. A recombinant vector containing the polynucleotide according to any one of Claims 27 to 29.

33. The recombinant vector according to Claim 32, wherein the recombinant vector is a recombinant expression vector.

34. A transformant that has been transfected with the recombinant vector according to Claim 30 or Claim 31.

35. A transformant that has been transfected with the recombinant vector according to Claim 32 or Claim 33.

36. A transformant that has been transfected with a recombinant vector selected from the group consisting of the recombinant vector according to Claim 30 or Claim 31 and the recombinant vector according to Claim 32 or Claim 33.

37. A method for manufacturing the polypeptide according to any one of Claims 7, 8, and 12 or the peptide according to any one of Claims 13 to 15, wherein the method comprises a step of culturing a transformant that has been transfected with the recombinant vector according to Claim 31, or a cell-free protein synthesis means that uses the recombinant vector according to Claim 30 or Claim 31.

38. A method for manufacturing the polypeptide according to any one of Claims 9 to 11 or the peptide according to Claim 16 or Claim 17, wherein the method comprises a step of culturing a transformant that has been transfected with the recombinant vector according to Claim 33, or a cell-free protein synthesis means that uses the recombinant vector according to Claim 32 or Claim 33.

39. An antibody that immunologically recognizes the polypeptide according to any one of Claims 7, 8, and 12 and/or the peptide according to any one of Claim 13 to Claim 15.

40. An antibody that immunologically recognizes the polypeptide according to any one of Claims 7, 8, and 12 and/or the peptide according to any one of Claim 13 to Claim 15, wherein the antibody inhibits the function of said polypeptide.

41. An antibody that immunologically recognizes the polypeptide according to any one of Claims 9 to 11 and/or the peptide according to Claim 16 or Claim 17.

42. An antibody that immunologically recognizes the polypeptide according to any one of Claims 9 to 11 and/or the peptide according to Claim 16 or Claim 17, wherein the antibody inhibits the interaction of the polypeptide having the amino acid sequence set forth in SEQ ID NO: 1 in the Sequence Listing, with the polypeptide having the amino acid sequence set forth in SEQ ID NO: 3 in the Sequence Listing.

43. A method for identifying a compound that interacts with the polypeptide according to any one of Claims 7 to 11 and inhibits or promotes the function thereof, and/or a compound that interacts with the polynucleotide according to any one of Claims 24 to 29 and inhibits or promotes the expression thereof, wherein the method comprises using at least one selected from the group consisting of the polypeptide according to any one of Claims 7 to 11, the polynucleotide according to any one of Claims 24 to 29, the recombinant vector according to any one of Claims 30 to 33, the transformant according to any one of Claims 34 to 36, and the antibody according to any one of Claims 39 to 42.

44. A method for identifying a compound that interacts with the polypeptide according to any one of Claims 7 to 11 and inhibits or promotes the function thereof, and/or a compound that interacts with the polynucleotide according to any one of Claims 24 to 29 and inhibits or promotes the expression thereof, wherein the method comprises contacting the compound with the polypeptide or the polynucleotide under conditions where the interaction of the compound with the polypeptide or the interaction of the compound with the polynucleotide are allowed, and determining whether the compound interacts with the polypeptide or the polynucleotide and inhibits or promotes the function of the polypeptide or the expression of the polynucleotide by detecting presence, absence, or variation of a signal which results from the interaction of the compound with the polypeptide or the interaction of the compound with the polynucleotide.

45. A method for identifying a compound that interacts with the polypeptide according to any one of Claims 7 to 11 and inhibits or promotes the function thereof, and/or a compound that interacts with the polynucleotide according to any one of Claims 24 to 29 and inhibits or promotes the expression thereof, wherein the method comprises contacting the compound with the transformant according to any one of Claims 34 to 36, and determining whether the compound inhibits or promotes the expression or function of the polypeptide by using a system that uses a signal and/or a marker being capable of detecting presence or absence of the expression or the function of the polypeptide to detect presence, absence, or variation of the signal and/or marker.

46. A method for identifying a compound that inhibits or promotes the binding of a polypeptide having the amino acid sequence set forth in SEQ ID NO: 1 in the Sequence Listing, to an N-methyl-D-aspartate receptor, wherein the method comprises using at least one selected from the group consisting of the polypeptide according to Claim 7 or Claim 8, the polynucleotide according to any one of Claims 24 to 26, the recombinant vector according to Claims 30 or 31, the transformant according to Claim 34, and the antibody according to Claim 39 or Claim 40.

47. A method for identifying a compound that inhibits or promotes the interaction of a polypeptide having the amino acid sequence set forth in SEQ ID NO: 1 in the Sequence Listing, with a polypeptide having the amino acid sequence set forth in SEQ ID NO: 3 in the Sequence Listing, wherein the method comprises using at least one selected from the group consisting of the polypeptide according to any one of Claims 7 to 11, the polynucleotide according to any one of Claims 24 to 29, the recombinant vector according to any one of Claims 30 to 33, the transformant according to any one of Claims 34 to 36, and the antibody according to any one of Claims 39 to 42.

48. A compound identified by the identification method according to any one of Claims 43 to 47.

49. A compound that interacts with the polypeptide according to Claim 7 or Claim 8 and inhibits or promotes the function thereof.

50. A compound that interacts with the polynucleotide according to any one of Claims 24 to 26 and inhibits or promotes the expression thereof.

51. A compound that interacts with the polypeptide according to any one of Claims 9 to 11 and inhibits or promotes the interaction of the polypeptide with a polypeptide having the amino acid sequence set forth in SEQ ID NO: 1 in the Sequence Listing.

52. A compound that interacts with the polypeptide according to any one of Claims 9 to 11 and inhibits or promotes the amplification of the N-methyl-D-aspartate receptor mediated signal transduction in the presence of the polypeptide and a polypeptide having the amino acid sequence set forth in SEQ ID NO: 1 in the Sequence Listing.

53. A compound that interacts with the polynucleotide according to any one of Claims 27 to 29 and inhibits or promotes the expression thereof.

54. A pharmaceutical composition comprising an effective dose of at least one selected from the group consisting of the polypeptide according to any one of Claims 7 to 11, the peptide according to any one of Claims 13 to 17, the polynucleotide according to any one of Claims 24 to 29, the recombinant vector according to any one of Claims 30 to 33, the transformant according to any one of Claims 34 to 36, the antibody according to any one of Claims 39 to 42, the compound according to any one of Claims 48 to 53, the controlling agent according to Claim 1 or Claim 18, the inhibiting agent according to Claim 2 or Claim 19, and the agent of promotion according to Claim 3 or Claim 20.

55. An agent for preventing, treating, or improving a disease caused by an anomaly in the N-methyl-D-aspartate receptor mediated signal transduction, wherein the agent comprises an effective dose of at least one selected from the group consisting of the polypeptide according to any one of Claims 7 to 11, the peptide according to any one of Claims 13 to 17, the polynucleotide according to any one of Claims 24 to 29, the recombinant vector according to any one of Claims 30 to 33, the transformant according to any one of Claims 34 to 36, the antibody according to any one of Claims 39 to 42, the compound according to any one of Claims 48 to 53, the controlling agent according to Claim 1 or Claim 18, the inhibiting agent according to Claim 2 or Claim 19, and the agent of promotion according to Claim 3 or Claim 20.

56. An agent for preventing, treating, or improving a disease caused by an anomaly in memory recall, wherein the agent comprises an effective dose of at least one selected from the group consisting of the polypeptide according to any one of Claims 7 to 11, the peptide according to any one of Claims 13 to 17, the polynucleotide according to any one of Claims 24 to 29, the recombinant vector according to any one of Claims 30 to 33, the transformant according to any one of Claims 34 to 36, the antibody according to any one of Claims 39 to 42, the compound according to any one of Claims 48 to 53, the controlling agent according to Claim 1 or Claim 18, the inhibiting agent according to Claim 2 or Claim 19, and the agent of promotion according to Claim 3 or Claim 20.

57. An agent for preventing, treating, or improving a neurodegenerative disease, wherein the agent comprises an effective dose of at least one selected from the group consisting of the polypeptide according to any one of Claims 7 to 11, the peptide according to any one of Claims 13 to 17, the polynucleotide according to any one of Claims 24 to 29, the recombinant vector according to any one of Claims 30 to 33, the transformant according to any one of Claims 34 to 36, the antibody according to any one of Claims 39 to 42, the compound according to any one of Claims 48 to 53, the controlling agent according to Claim 1 or Claim 18, the inhibiting agent according to Claim 2 or Claim 19, and the agent of promotion according to Claim 3 or Claim 20.

58. An agent for preventing, treating, or improving Alzheimer's disease, wherein the agent comprises an effective dose of at least one selected from the group consisting of the polypeptide according to any one of Claims 7 to 11, the peptide according to any one of Claims 13 to 17, the polynucleotide according to any one of Claims 24 to 29, the recombinant vector according to any one of Claims 30 to 33, the transformant according to any one of Claims 34 to 36, the antibody according to any one of Claims 39 to 42, the compound according to any one of Claims 48 to 53, the controlling agent according to Claim 1 or Claim 18, the inhibiting agent according to Claim 2 or Claim 19, and the agent of promotion according to Claim 3 or Claim 20.

59. A method for preventing, treating, or improving a disease caused by an anomaly in the N-methyl-D-aspartate receptor mediated signal transduction, wherein the method comprises administering at least one selected from the group consisting of the polypeptide according to any one of Claims 7 to 11, the peptide according to any one of Claims 13 to 17, the polynucleotide according to any one of Claims 24 to 29, the recombinant vector according to any one of Claims 30 to 33, the transformant according to any one of Claims 34 to 36, the antibody according to any one of Claims 39 to 42, the compound according to any one of Claims 48 to 53, the controlling agent according to Claim 1 or Claim 18, the inhibiting agent according to Claim 2 or Claim 19, and the agent of promotion according to Claim 3 or Claim 20.

60. A method for preventing, treating, or improving a disease caused by an anomaly in memory recall, wherein the method comprises administering at least one selected from the group consisting of the polypeptide according to any one of Claims 7 to 11, the peptide according to any one of Claims 13 to 17, the polynucleotide according to any one of Claims 24 to 29, the recombinant vector according to any one of Claims 30 to 33, the transformant according to any one of Claims 34 to 36, the antibody according to any one of Claims 39 to 42, the compound according to any one of Claims 48 to 53, the controlling agent according to Claim 1 or Claim 18, the inhibiting agent according to Claim 2 or Claim 19, and the agent of promotion according to Claim 3 or Claim 20.

61. A method for preventing, treating, or improving a neurodegenerative disease, wherein the method comprises administering at least one selected from the group consisting of the polypeptide according to any one of Claims 7 to 11, the peptide according to any one of Claims 13 to 17, the polynucleotide according to any one of Claims 24 to 29, the recombinant vector according to any one of Claims 30 to 33, the transformant according to any one of Claims 34 to 36, the antibody according to any one of Claims 39 to 42, the compound according to any one of Claims 48 to 53, the controlling agent according to Claim 1 or Claim 18, the inhibiting agent according to Claim 2 or Claim 19, and the agent of promotion according to Claim 3 or Claim 20.

62. A method for preventing, treating, or improving Alzheimer's disease, wherein the method comprises administering at least one selected from the group consisting of the polypeptide according to any one of Claims 7 to 11, the peptide according to any one of Claims 13 to 17, the polynucleotide according to any one of Claims 24 to 29, the recombinant vector according to any one of Claims 30 to 33, the transformant according to any one of Claims 34 to 36, the antibody according to any one of Claims 39 to 42, the compound according to any one of Claims 48 to 53, the controlling agent according to Claim 1 or Claim 18, the inhibiting agent according to Claim 2 or Claim 19, and the agent of promotion according to Claim 3 or Claim 20.

63. A method for quantitatively or qualitatively measuring the polypeptide according to any one of Claims 7 to 11, or the polynucleotide according to any one of Claims 24 to 29.

64. A reagent kit comprising at least one selected from the group consisting of the polypeptide according to any one of Claims 7 to 11, the peptide according to any one of Claims 13 to 17, the polynucleotide according to any one of Claims 24 to 29, the recombinant vector according to any one of Claims 30 to 33, the transformant according to any one of Claims 34 to 36, and the antibody according to any one of Claims 39 to 42.


SEQUENCE LISTING

<110> DAIICHI PHARMACEUTICAL CO., LTD.
KAZUSA DNA RESEARCH INSTITUTE FOUNDATION

<120> A novel postsynaptic density protein

<130> GP02-1016PCT

<150> JP P2001-354678
<151> 2001-11-20

<150> JP P2002-46786
<151> 2002-02-22

<150> JP P2002-229863
<151> 2002-08-07

<160> 19

<170> PatentIn version 3.1

<210> 1
<211> 576
<212> PRT
<213> Homo sapiens

<400> 1

Met Pro Ala Leu Ser Thr Gly Ser Gly Ser Asp Thr Gly Leu Tyr Glu
1               5                   10                  15

Leu Leu Ala Ala Leu Pro Ala Gln Leu Gln Pro His Val Asp Ser Gln
            20                  25                  30

Glu Asp Leu Thr Phe Leu Trp Asp Met Phe Gly Glu Lys Ser Leu His
        35                  40                  45

Ser Leu Val Lys Ile His Glu Lys Leu His Tyr Tyr Glu Lys Gln Ser
    50                  55                  60

Pro Val Pro Ile Leu His Gly Ala Ala Ala Leu Ala Asp Asp Leu Ala
65                  70                  75                  80

Glu Glu Leu Gln Asn Lys Pro Leu Asn Ser Glu Ile Arg Glu Leu Leu
                85                  90                  95

Lys Leu Leu Ser Lys Pro Asn Val Lys Ala Leu Leu Ser Val His Asp
            100                 105                 110

Thr Val Ala Gln Lys Asn Tyr Asp Pro Val Leu Pro Pro Met Pro Glu
        115                 120                 125

Asp Ile Asp Asp Glu Glu Asp Ser Val Lys Ile Ile Arg Leu Val Lys
    130                 135                 140

Asn Arg Glu Pro Leu Gly Ala Thr Ile Lys Lys Asp Glu Gln Thr Gly
145                 150                 155                 160

Ala Ile Ile Val Ala Arg Ile Met Arg Gly Gly Ala Ala Asp Arg Ser
                165                 170                 175

Gly Leu Ile His Val Gly Asp Glu Leu Arg Glu Val Asn Gly Ile Pro
            180                 185                 190

Val Glu Asp Lys Arg Pro Glu Glu Ile Ile Gln Ile Leu Ala Gln Ser
        195                 200                 205

Gln Gly Ala Ile Thr Phe Lys Ile Ile Pro Gly Ser Lys Glu Glu Thr
    210                 215                 220

Pro Ser Lys Glu Gly Lys Met Phe Ile Lys Ala Leu Phe Asp Tyr Asn
225                 230                 235                 240

Pro Asn Glu Asp Lys Ala Ile Pro Cys Lys Glu Ala Gly Leu Ser Phe

                245                 250                 255

Lys Lys Gly Asp Ile Leu Gln Ile Met Ser Gln Asp Asp Ala Thr Trp
            260                 265                 270

Trp Gln Ala Lys His Glu Ala Asp Ala Asn Pro Arg Ala Gly Leu Ile
        275                 280                 285

Pro Ser Lys His Phe Gln Glu Arg Arg Leu Ala Leu Arg Arg Pro Glu
    290                 295                 300

Ile Leu Val Gln Pro Leu Lys Val Ser Asn Arg Lys Ser Ser Gly Phe
305                 310                 315                 320

Arg Arg Ser Phe Arg Leu Ser Arg Lys Asp Lys Lys Thr Asn Lys Ser
                325                 330                 335

Met Tyr Glu Cys Lys Lys Ser Asp Gln Tyr Asp Thr Ala Asp Val Pro
            340                 345                 350

Thr Tyr Glu Glu Val Thr Pro Tyr Arg Arg Gln Thr Asn Glu Lys Tyr
        355                 360                 365

Arg Leu Val Val Leu Val Gly Pro Val Gly Val Gly Leu Asn Glu Leu
    370                 375                 380

Lys Arg Lys Leu Leu Ile Ser Asp Thr Gln His Tyr Gly Val Thr Val
385                 390                 395                 400

Pro His Thr Thr Arg Ala Arg Arg Ser Gln Glu Ser Asp Gly Val Glu
                405                 410                 415

Tyr Ile Phe Ile Ser Lys His Leu Phe Glu Thr Asp Val Gln Asn Asn
            420                 425                 430

Lys Phe Ile Glu Tyr Gly Glu Tyr Lys Asn Asn Tyr Tyr Gly Thr Ser
        435                 440                 445

Ile Asp Ser Val Arg Ser Val Leu Ala Lys Asn Lys Val Cys Leu Leu
    450                 455                 460

Asp Val Gln Pro His Thr Val Lys His Leu Arg Thr Leu Glu Phe Lys
465                 470                 475                 480

Pro Tyr Val Ile Phe Ile Lys Pro Pro Ser Ile Glu Arg Leu Arg Glu
                485                 490                 495

Thr Arg Lys Asn Ala Lys Ile Ile Ser Ser Arg Asp Asp Gln Gly Ala
            500                 505                 510

Ala Lys Pro Phe Thr Glu Glu Asp Phe Gln Glu Met Ile Lys Ser Ala
        515                 520                 525

Gln Ile Met Glu Ser Gln Tyr Gly His Leu Phe Asp Lys Ile Ile Ile
    530                 535                 540

Asn Asp Asp Leu Thr Val Ala Phe Asn Glu Leu Lys Thr Thr Phe Asp
545                 550                 555                 560

Lys Leu Glu Thr Glu Thr His Trp Val Pro Val Ser Trp Leu His Ser
                565                 570                 575

<210>  2
<211>  491
<212>  PRT
<213>  Homo sapiens

<400>  2

Lys Pro Leu Asn Ser Glu Ile Arg Glu Leu Leu Lys Leu Leu Ser Lys
1               5                   10                  15

Pro Asn Val Lys Ala Leu Leu Ser Val His Asp Thr Val Ala Gln Lys
            20                  25                  30

Asn Tyr Asp Pro Val Leu Pro Pro Met Pro Glu Asp Ile Asp Asp Glu
        35                  40                  45

Glu Asp Ser Val Lys Ile Ile Arg Leu Val Lys Asn Arg Glu Pro Leu
    50                  55                  60

Gly Ala Thr Ile Lys Lys Asp Glu Gln Thr Gly Ala Ile Ile Val Ala
65                  70                  75                  80

Arg Ile Met Arg Gly Gly Ala Ala Asp Arg Ser Gly Leu Ile His Val
                85                  90                  95

Gly Asp Glu Leu Arg Glu Val Asn Gly Ile Pro Val Glu Asp Lys Arg
            100                 105                 110

Pro Glu Glu Ile Ile Gln Ile Leu Ala Gln Ser Gln Gly Ala Ile Thr
        115                 120                 125

Phe Lys Ile Ile Pro Gly Ser Lys Glu Glu Thr Pro Ser Lys Glu Gly
    130                 135                 140

Lys Met Phe Ile Lys Ala Leu Phe Asp Tyr Asn Pro Asn Glu Asp Lys
145                 150                 155                 160

Ala Ile Pro Cys Lys Glu Ala Gly Leu Ser Phe Lys Lys Gly Asp Ile
                165                 170                 175

Leu Gln Ile Met Ser Gln Asp Asp Ala Thr Trp Trp Gln Ala Lys His

            180                 185                 190

Glu Ala Asp Ala Asn Pro Arg Ala Gly Leu Ile Pro Ser Lys His Phe
        195                 200                 205

Gln Glu Arg Arg Leu Ala Leu Arg Arg Pro Glu Ile Leu Val Gln Pro
    210                 215                 220

Leu Lys Val Ser Asn Arg Lys Ser Ser Gly Phe Arg Arg Ser Phe Arg
225                 230                 235                 240

Leu Ser Arg Lys Asp Lys Lys Thr Asn Lys Ser Met Tyr Glu Cys Lys
                245                 250                 255

Lys Ser Asp Gln Tyr Asp Thr Ala Asp Val Pro Thr Tyr Glu Glu Val
            260                 265                 270

Thr Pro Tyr Arg Arg Gln Thr Asn Glu Lys Tyr Arg Leu Val Val Leu
        275                 280                 285

Val Gly Pro Val Gly Val Gly Leu Asn Glu Leu Lys Arg Lys Leu Leu
    290                 295                 300

Ile Ser Asp Thr Gln His Tyr Gly Val Thr Val Pro His Thr Thr Arg
305                 310                 315                 320

Ala Arg Arg Ser Gln Glu Ser Asp Gly Val Glu Tyr Ile Phe Ile Ser
                325                 330                 335

Lys His Leu Phe Glu Thr Asp Val Gln Asn Asn Lys Phe Ile Glu Tyr
            340                 345                 350

Gly Glu Tyr Lys Asn Asn Tyr Tyr Gly Thr Ser Ile Asp Ser Val Arg
        355                 360                 365

Ser Val Leu Ala Lys Asn Lys Val Cys Leu Leu Asp Val Gln Pro His
    370                 375                 380

Thr Val Lys His Leu Arg Thr Leu Glu Phe Lys Pro Tyr Val Ile Phe
385                 390                 395                 400

Ile Lys Pro Pro Ser Ile Glu Arg Leu Arg Glu Thr Arg Lys Asn Ala
                405                 410                 415

Lys Ile Ile Ser Ser Arg Asp Asp Gln Gly Ala Ala Lys Pro Phe Thr
            420                 425                 430

Glu Glu Asp Phe Gln Glu Met Ile Lys Ser Ala Gln Ile Met Glu Ser
        435                 440                 445

Gln Tyr Gly His Leu Phe Asp Lys Ile Ile Ile Asn Asp Asp Leu Thr
    450                 455                 460

Val Ala Phe Asn Glu Leu Lys Thr Thr Phe Asp Lys Leu Glu Thr Glu
465                 470                 475                 480

Thr His Trp Val Pro Val Ser Trp Leu His Ser
                485                 490

<210>  3
<211>  979
<212>  PRT
<213>  Homo sapiens

<400>  3

Met Arg Gly Tyr His Gly Asp Arg Gly Ser His Pro Arg Pro Ala Arg
1               5                   10                  15

Phe Ala Asp Gln Gln His Met Asp Val Gly Pro Ala Ala Arg Ala Pro
            20                  25                  30

Tyr Leu Leu Gly Ser Arg Glu Ala Phe Ser Thr Glu Pro Arg Phe Cys
        35                  40                  45

Ala Pro Arg Ala Gly Leu Gly His Ile Ser Pro Glu Gly Pro Leu Ser
    50                  55                  60

Leu Ser Glu Gly Pro Ser Val Gly Pro Glu Gly Gly Pro Ala Gly Ala
65                  70                  75                  80

Gly Val Gly Gly Gly Ser Ser Thr Phe Pro Arg Met Tyr Pro Gly Gln
                85                  90                  95

Gly Pro Phe Asp Thr Cys Glu Asp Cys Val Gly His Pro Gln Gly Lys
            100                 105                 110

Gly Ala Pro Arg Leu Pro Pro Thr Leu Leu Asp Gln Phe Glu Lys Gln
        115                 120                 125

Leu Pro Val Gln Gln Asp Gly Phe His Thr Leu Pro Tyr Gln Arg Gly
    130                 135                 140

Pro Ala Gly Ala Gly Pro Gly Pro Ala Pro Gly Thr Gly Thr Ala Pro
145                 150                 155                 160

Glu Pro Arg Ser Glu Ser Pro Ser Arg Ile Arg His Leu Val His Ser
                165                 170                 175

Val Gln Lys Leu Phe Ala Lys Ser His Ser Leu Glu Ala Pro Gly Lys
            180                 185                 190

Arg Asp Tyr Asn Gly Pro Lys Ala Glu Gly Arg Gly Gly Ser Gly Gly

        195                 200                 205

Asp Ser Tyr Pro Gly Pro Gly Ser Gly Gly Pro His Thr Ser His His
    210                 215                 220

His His His His His His His His His His Gln Ser Arg His Gly Lys
225                 230                 235                 240

Arg Ser Lys Ser Lys Asp Arg Lys Gly Asp Gly Arg His Gln Ala Lys
                245                 250                 255

Ser Thr Gly Trp Trp Ser Ser Asp Asp Asn Leu Asp Ser Asp Ser Gly
            260                 265                 270

Phe Leu Ala Gly Gly Arg Pro Pro Gly Glu Pro Gly Gly Pro Phe Cys
        275                 280                 285

Leu Glu Gly Pro Asp Gly Ser Tyr Arg Asp Leu Ser Phe Lys Gly Arg
    290                 295                 300

Ser Gly Gly Ser Glu Gly Arg Cys Leu Ala Cys Thr Gly Met Ser Met
305                 310                 315                 320

Ser Leu Asp Gly Gln Ser Val Lys Arg Ser Ala Trp His Thr Met Met
                325                 330                 335

Val Ser Gln Gly Arg Asp Gly Tyr Pro Gly Ala Gly Pro Gly Lys Gly
            340                 345                 350

Leu Leu Gly Pro Glu Thr Lys Ala Lys Ala Arg Thr Tyr His Tyr Leu
        355                 360                 365

Gln Val Pro Gln Asp Asp Trp Gly Gly Tyr Pro Thr Gly Gly Lys Asp
    370                 375                 380

Gly Glu Ile Pro Cys Arg Arg Met Arg Ser Gly Ser Tyr Ile Lys Ala
385                 390                 395                 400

Met Gly Asp Glu Glu Ser Gly Asp Ser Asp Gly Ser Pro Lys Thr Ser
                405                 410                 415

Pro Lys Ala Val Ala Arg Arg Phe Thr Thr Arg Arg Ser Ser Ser Val
            420                 425                 430

Asp Gln Ala Arg Ile Asn Cys Cys Val Pro Pro Arg Ile His Pro Arg
        435                 440                 445

Ser Ser Ile Pro Gly Tyr Ser Arg Ser Leu Thr Thr Gly Gln Leu Ser
    450                 455                 460

Asp Glu Leu Asn Gln Gln Leu Glu Ala Val Cys Gly Ser Val Phe Gly
465                 470                 475                 480

Glu Leu Glu Ser Gln Ala Val Asp Ala Leu Asp Leu Pro Gly Cys Phe
                485                 490                 495

Arg Met Arg Ser His Ser Tyr Leu Arg Ala Ile Gln Ala Gly Cys Ser
            500                 505                 510

Gln Asp Asp Asp Cys Leu Pro Leu Leu Ala Thr Pro Ala Ala Val Ser
        515                 520                 525

Gly Arg Pro Gly Ser Ser Phe Asn Phe Arg Lys Ala Pro Pro Pro Ile
    530                 535                 540

Pro Pro Gly Ser Gln Ala Pro Pro Arg Ile Ser Ile Thr Ala Gln Ser
545                 550                 555                 560

Ser Thr Asp Ser Ala His Glu Ser Phe Thr Ala Ala Glu Gly Pro Ala
                565                 570                 575

Arg Arg Cys Ser Ser Ala Asp Gly Leu Asp Gly Pro Ala Met Gly Ala
            580                 585                 590

Arg Thr Leu Glu Leu Ala Pro Val Pro Pro Arg Ala Ser Pro Lys Pro
        595                 600                 605

Pro Thr Leu Ile Ile Lys Thr Ile Pro Gly Arg Glu Glu Leu Arg Ser
    610                 615                 620

Leu Ala Arg Gln Arg Lys Trp Arg Pro Ser Ile Gly Val Gln Val Glu
625                 630                 635                 640

Thr Ile Ser Asp Ser Asp Thr Glu Asn Arg Ser Arg Arg Glu Phe His
                645                 650                 655

Ser Ile Gly Val Gln Val Glu Glu Asp Lys Arg Arg Ala Arg Phe Lys
            660                 665                 670

Arg Ser Asn Ser Val Thr Ala Gly Val Gln Ala Asp Leu Glu Leu Glu
        675                 680                 685

Gly Leu Ala Gly Leu Ala Thr Val Ala Thr Glu Asp Lys Ala Leu Gln
    690                 695                 700

Phe Gly Arg Ser Phe Gln Arg His Ala Ser Glu Pro Gln Pro Gly Pro
705                 710                 715                 720

Arg Ala Pro Thr Tyr Ser Val Phe Arg Thr Val His Thr Gln Gly Gln
                725                 730                 735

Trp Ala Tyr Arg Glu Gly Tyr Pro Leu Pro Tyr Glu Pro Pro Ala Thr
            740                 745                 750

Asp Gly Ser Pro Gly Pro Ala Pro Ala Pro Thr Pro Gly Pro Gly Ala
        755                 760                 765

Gly Arg Arg Asp Ser Trp Ile Glu Arg Gly Ser Arg Ser Leu Pro Asp
    770                 775                 780

Ser Gly Arg Ala Ser Pro Cys Pro Arg Asp Gly Glu Trp Phe Ile Lys
785                 790                 795                 800

Met Leu Arg Ala Glu Val Glu Lys Leu Glu His Trp Cys Gln Gln Met
                805                 810                 815

Glu Arg Glu Ala Glu Asp Tyr Glu Leu Pro Glu Glu Ile Leu Glu Lys
            820                 825                 830

Ile Arg Ser Ala Val Gly Ser Thr Gln Leu Leu Leu Ser Gln Lys Val
        835                 840                 845

Gln Gln Phe Phe Arg Leu Cys Gln Gln Ser Met Asp Pro Thr Ala Phe
    850                 855                 860

Pro Val Pro Thr Phe Gln Asp Leu Ala Gly Phe Trp Asp Leu Leu Gln
865                 870                 875                 880

Leu Ser Ile Glu Asp Val Thr Leu Lys Phe Leu Glu Leu Gln Gln Leu
                885                 890                 895

Lys Ala Asn Ser Trp Lys Leu Leu Glu Pro Lys Glu Glu Lys Lys Val
            900                 905                 910

Pro Pro Pro Ile Pro Lys Lys Pro Leu Arg Gly Arg Gly Val Pro Val

915                    920                    925

Lys Glu Arg Ser Leu Asp Ser Val Asp Arg Gln Arg Gln Glu Ala Arg
    930                    935                    940

Lys Arg Leu Leu Ala Ala Lys Arg Ala Ala Ser Phe Arg His Ser Ser
945                    950                    955                    960

Ala Thr Glu Ser Ala Asp Ser Ile Glu Ile Tyr Ile Pro Glu Ala Gln
                965                    970                    975

Thr Arg Leu

<210>  4
<211>  4941
<212>  DNA
<213>  Homo sapiens

<400>  4
gccgggctgg aggcgggggc cgggctccca ctgggctcgt gcgttctgcg cccgccgcgg      60

cggtgccgag cccgctggct cccgattgtc ctctgcggcg gtggcggtcg ctgcctcctt     120

gcctccgggc ccggggctgc aggggccaga gcgagtgcgc ctcctgcccg cggaccgcgc     180

cagcccagag cagaaacggc ttacaaaata tacagatctt ggtagacaac gtggctgcag     240

gctgttgaat tggaattccc tgtggctgtc cgaagcaggg tgtccggaga gcggtgggct     300

gacctgttcc tacaccttgc atcatgccag ctttgtcaac gggatctggg agtgacactg     360

gtctgtatga gctgttggct gctctgccag cccagctgca gccacatgtg gatagccagg     420

aagacctgac cttcctctgg gatatgtttg gtgaaaaaag cctgcattca ttggtaaaga     480

ttcatgaaaa actacactac tatgagaagc agagtccggt gcccattctc catggtgcgg     540

cggccttggc cgatgatctg gccgaagagc ttcagaacaa gccattaaac agtgagatca     600

gagagctgtt gaaactactg tcaaaaccca atgtgaaggc tttgctctct gtacatgata 660

ctgtggctca gaagaattac gacccagtgt tgcctcctat gcctgaagat attgacgatg 720

aggaagactc agtaaaaata atccgtctgg tcaaaaatag agaaccactg ggagctacca 780

ttaagaagga tgaacagacc ggggcgatca ttgtggccag aatcatgaga ggaggagctg 840

cagatagaag tggtcttatt catgttggtg atgaacttag ggaagtcaac gggataccag 900

tggaggataa aaggcctgag gaaataatac agattttggc tcagtctcag ggagcaatta 960

catttaagat tatacccggc agcaaagagg agacaccatc aaaagaaggc aagatgttta 1020

tcaaagccct ctttgactat aatcctaatg aggataaggc aattccatgt aaggaagctg 1080

ggctttcttt caaaaaggga gatattcttc agattatgag ccaagatgat gcaacttggt 1140

ggcaagcgaa acacgaagct gatgccaacc ccagggcagg cttgatcccc tcaaagcatt 1200

tccaggaaag gagattggct ttgagacgac cagaaatatt ggttcagccc ctgaaagttt 1260

ccaacaggaa atcatctggt tttagaagaa gttttcgtct tagtagaaaa gataagaaaa 1320

caaataaatc catgtatgaa tgcaagaaga gtgatcagta cgacacagct gacgtaccca 1380

catacgaaga agtgacaccg tatcggcgac aaactaatga aaaatacaga ctcgttgtct 1440

tggttggtcc cgtgggagta gggctgaatg aactgaaacg aaagctgctg atcagtgaca 1500

cccagcacta tggcgtgaca gtgccccata ccaccagagc aagaagaagc caggagagtg 1560

atggtgttga atacattttc atttccaagc atttgtttga gacagatgta caaaataaca 1620

agtttattga atatggagaa tataaaaaca actactacgg cacaagtata gactcagttc 1680

ggtctgtcct tgctaaaaac aaagtttgtt tgttggatgt tcagcctcat acagtgaagc 1740

atttaaggac actagaattt aagccctatg tgatatttat aaagcctcca tcaatagagc 1800

gtttgagaga aacaagaaaa aatgcaaaga ttatttcaag cagagatgac caaggtgctg 1860

caaaaccctt cacagaagaa gattttcaag aaatgattaa atctgcacag ataatggaaa 1920

gtcaatatgg tcatcttttt gacaaaatta taataaatga tgacctcact gtggcattca 1980

atgagctcaa aacaactttt gacaaattag agacagagac ccattgggtg ccagtgagct 2040

ggttacattc ataactaaga gaaatttcca taattgtctt tttctataga gtgcatgatg 2100

aaatcaatta cagttttggt agtagggttt ttaaatctat atcactgtca tagatgtaca 2160

atcttggttc aagttgaatg ctggttttgt ttgtatcttt ttacagcctt atttcaaatg 2220

ccatgtgtta gtataagatc cgaaatcaaa atatgcacag tactgtattc taagcaaaac 2280

ctcaaacctt ctcgttgtct tcaataccgc tctatctcca agatgaggct gaaattttca 2340

gagagactta gctagaggct tagtatgtat gggagttcag cgcttctgct ggtctcaggt 2400

gtggctgctg ctgtcgagtt tgaatgttag ctgttgaagg tatcaattca gcagccatga 2460

gcagctccag acagacaggt gagctctgct gtttctgggt ggatcatcac agatttagcc 2520

gggcaggcag taaggtgtcc tcttactatt caaaaagtgt agactttctt acatattcgc 2580

aatacgttca cagtgtgtgc attttaaaat aattcttaaa ggagtaactg aaattttacc 2640

ttgagtgaat ggccttcata atatagcttg agaagtcctt ttgagtacct gtcagtgact 2700

caacaacatt taataaggga aagtagactt ttaacagtta ttatatatgt aacgaaaagc 2760

ctttcctttg ggattaatat aagtaagaat ggtagccttg tggcaagaaa tgattacaaa 2820

ggatattttt atttgtaatt cctcagaaga caatttatga agtcacccaa aatgttattt 2880

tagctggttt tggatttttc caataaatta gaagaaggat ttctattcta aaacatgtaa 2940

aacctgtttt acatattact gatacaatta aagattattt ttcatctatg tgcaatagat 3000

cacccctctt taaattgctc taagatttat tttagaaaac ttttcatgtg atgttatttc 3060

tttgtcatca aaatgcttgt taacactgtc cagacaccat cctaaccttg ccattgttaa 3120

agaagtttag gaaagactct tatattgtaa atatttagat gggttctctc acttttcttt 3180

gatactactg attttcagca agtgaattat ataattcaaa atgctagaaa tgtctatccg 3240

ttctataaga gagcatatcc tgccgttctt gcatgcagtg aagccctgcc cgtcgaaaat 3300

cattgcatct gtgactttca aagtggaaaa aaaaatgtta tttttttgtt gatttgtaaa 3360

gagagtttaa atgtcatgtg aaaaaaaata gaatgtagta aaattctata tatttatgaa 3420

atattttaaa ggcatatttt tttaaatatc aaacggggct attcataaaa taaactgatt 3480

gtatgtcaag atgtcctaat ttaaaagagt agttttataa atcatggtca acattccatg 3540

taaatatttg agctttacaa agatagatcc aagtgtgcga tctgtgcact gcacatttga 3600

tagcattatt cactggtttt ccttcatact tgtcaagttc atattagaag cagagacaaa 3660

aaccactcca atggccttga cacatagtca caccaaaata gataatcaga ctaagtatta 3720

tataacaacg tgatccagac agtgagttct aagtgtatta attaaatagc aaaaaatttt 3780

gtttttaaaa aatgaaagag ggagggttgc caacctgaag tcttaagtag attgtttggg 3840

tagcatattt ccttctttga acattgtctg aaattttcta taaatcaaga cttcttgtac 3900

aaagaatgat gggacatatg taataattca ggcaatcacc tgagtaattt aggcagtcca 3960

aattctttac cctgaaatac ccacatttta aaaaaattgc agataattgc ttcagttatt 4020

tactttgggg acagagatat agtgtaaagt gggagaaact gagtcctttt ttggtggtgg 4080

taataataag atgttataaa aatataacat tttaaaaaag aaaggtccag accttaagcg 4140

cagagctaga acaatatttt ttaaataatg gggggaaaag ggggcacttt ggtaatttta 4200

gaaatcaggt agtatacttt tttttttttt tttgagacag ggtcttgcca tgttgctcag 4260

gctggtcttg aactcctggg ctcaagcaat cctcctgcct tggcctccca aagtgctggg 4320

attacaggca tgatccaccg tgcccagccg gtagacgtgg tcttaaaaac agtgtttaca 4380

tggccatctt gatgcttaga aagataattg attaaaattt aataaggcag ggccaactcc 4440

gagagttcat tgacaacggc agcaaaaagg ccctgaattc tgtactttct tcccccagcc 4500

tccttctcca gcaaggagaa tagcactcct ccctccagaa gccagctccc taagttggag 4560

ccactatgta agagaagagg aacgttcact ttttaaaatt catatattta aaaatcaaga 4620

ccaaaaagta aattctgtac tcctattatt gactgtagtc aatcaaacat aaaaaggtga 4680

aagtaaaatt taatttttta cccttatttt actgaccaat atggaagttc ttggtatctt 4740

taaggctgac cttcctggta ttgtgtaatg attgaatgta tctaaactgt aataatttga 4800

aactgacaaa cataaccttc tcagacttac aaaactatgt tctttctaaa gatacagatt 4860

tttattattt tattttgact aggaaggatt tataaataaa tgtaatgaaa aatctttgat 4920

cttaataaag taccttcaaa c 4941

<210> 5
<211> 4370
<212> DNA
<213> Homo sapiens

<400> 5
acaagccatt aaacagtgag atcagagagc tgttgaaact actgtcaaaa cccaatgtga 60

aggctttgct ctctgtacat gatactgtgg ctcagaagaa ttacgaccca gtgttgcctc 120

ctatgcctga agatattgac gatgaggaag actcagtaaa aataatccgt ctggtcaaaa 180

atagagaacc actgggagct accattaaga aggatgaaca gaccggggcg atcattgtgg 240

ccagaatcat gagaggagga gctgcagata gaagtggtct tattcatgtt ggtgatgaac 300

ttagggaagt caacgggata ccagtggagg ataaaaggcc tgaggaaata atacagattt 360

tggctcagtc tcagggagca attacattta agattatacc cggcagcaaa gaggagacac 420

catcaaaaga aggcaagatg tttatcaaag ccctctttga ctataatcct aatgaggata 480

aggcaattcc atgtaaggaa gctgggcttt ctttcaaaaa gggagatatt cttcagatta 540

tgagccaaga tgatgcaact tggtggcaag cgaaacacga agctgatgcc aaccccaggg 600

caggcttgat cccctcaaag catttccagg aaaggagatt ggctttgaga cgaccagaaa 660

tattggttca gcccctgaaa gtttccaaca ggaaatcatc tggttttaga agaagttttc 720

gtcttagtag aaaagataag aaaacaaata aatccatgta tgaatgcaag aagagtgatc 780

agtacgacac agctgacgta cccacatacg aagaagtgac accgtatcgg cgacaaacta 840

atgaaaaata cagactcgtt gtcttggttg gtcccgtggg agtagggctg aatgaactga 900

aacgaaagct gctgatcagt gacacccagc actatggcgt gacagtgccc cataccacca 960

gagcaagaag aagccaggag agtgatggtg ttgaatacat tttcatttcc aagcatttgt 1020

ttgagacaga tgtacaaaat aacaagttta ttgaatatgg agaatataaa aacaactact 1080

acggcacaag tatagactca gttcggtctg tccttgctaa aaacaaagtt tgtttgttgg 1140

atgttcagcc tcatacagtg aagcatttaa ggacactaga atttaagccc tatgtgatat 1200

ttataaagcc tccatcaata gagcgtttga gagaaacaag aaaaaatgca aagattattt 1260

caagcagaga tgaccaaggt gctgcaaaac ccttcacaga agaagatttt caagaaatga 1320

ttaaatctgc acagataatg gaaagtcaat atggtcatct ttttgacaaa attataataa 1380

atgatgacct cactgtggca ttcaatgagc tcaaaacaac ttttgacaaa ttagagacag 1440

agacccattg ggtgccagtg agctggttac attcataact aagagaaatt tccataattg 1500

tctttttcta tagagtgcat gatgaaatca attacagttt tggtagtagg gtttttaaat 1560

ctatatcact gtcatagatg tacaatcttg gttcaagttg aatgctggtt ttgtttgtat 1620

ctttttacag ccttatttca aatgccatgt gttagtataa gatccgaaat caaaatatgc 1680

acagtactgt attctaagca aaacctcaaa ccttctcgtt gtcttcaata ccgctctatc 1740

tccaagatga ggctgaaatt ttcagagaga cttagctaga ggcttagtat gtatgggagt 1800

tcagcgcttc tgctggtctc aggtgtggct gctgctgtcg agtttgaatg ttagctgttg 1860

aaggtatcaa ttcagcagcc atgagcagct ccagacagac aggtgagctc tgctgtttct 1920

gggtggatca tcacagattt agccgggcag gcagtaaggt gtcctcttac tattcaaaaa 1980

gtgtagactt tcttacatat tcgcaatacg ttcacagtgt gtgcatttta aaataattct 2040

taaaggagta actgaaattt taccttgagt gaatggcctt cataatatag cttgagaagt 2100

ccttttgagt acctgtcagt gactcaacaa catttaataa gggaaagtag acttttaaca 2160

gttattatat atgtaacgaa aagcctttcc tttgggatta atataagtaa gaatggtagc 2220

cttgtggcaa gaaatgatta caaaggatat ttttatttgt aattcctcag aagacaattt 2280

atgaagtcac ccaaaatgtt attttagctg gttttggatt tttccaataa attagaagaa 2340

ggatttctat tctaaaacat gtaaaacctg ttttacatat tactgataca attaaagatt 2400

atttttcatc tatgtgcaat agatcacccc tctttaaatt gctctaagat ttattttaga 2460

aaacttttca tgtgatgtta tttctttgtc atcaaaatgc ttgttaacac tgtccagaca 2520

ccatcctaac cttgccattg ttaaagaagt ttaggaaaga ctcttatatt gtaaatattt 2580

agatgggttc tctcactttt ctttgatact actgattttc agcaagtgaa ttatataatt 2640

caaaatgcta gaaatgtcta tccgttctat aagagagcat atcctgccgt tcttgcatgc 2700

agtgaagccc tgcccgtcga aaatcattgc atctgtgact ttcaaagtgg aaaaaaaaat 2760

gttatttttt tgttgatttg taaagagagt ttaaatgtca tgtgaaaaaa aatagaatgt 2820

agtaaaattc tatatattta tgaaatattt taaaggcata tttttttaaa tatcaaacgg 2880

ggctattcat aaaataaact gattgtatgt caagatgtcc taatttaaaa gagtagtttt 2940

ataaatcatg gtcaacattc catgtaaata tttgagcttt acaaagatag atccaagtgt 3000

gcgatctgtg cactgcacat ttgatagcat tattcactgg ttttccttca tacttgtcaa 3060

gttcatatta gaagcagaga caaaaaccac tccaatggcc ttgacacata gtcacaccaa 3120

aatagataat cagactaagt attatataac aacgtgatcc agacagtgag ttctaagtgt 3180

attaattaaa tagcaaaaaa ttttgttttt aaaaaatgaa agagggaggg ttgccaacct 3240

gaagtcttaa gtagattgtt tgggtagcat atttccttct ttgaacattg tctgaaattt 3300

tctataaatc aagacttctt gtacaaagaa tgatgggaca tatgtaataa ttcaggcaat 3360

cacctgagta atttaggcag tccaaattct ttaccctgaa atacccacat tttaaaaaaa 3420

ttgcagataa ttgcttcagt tatttacttt ggggacagag atatagtgta aagtgggaga 3480

aactgagtcc ttttttggtg gtggtaataa taagatgtta taaaaatata acattttaaa 3540

aaagaaaggt ccagacctta agcgcagagc tagaacaata ttttttaaat aatgggggga 3600

aaagggggca ctttggtaat tttagaaatc aggtagtata cttttttttt tttttttgag 3660

acagggtctt gccatgttgc tcaggctggt cttgaactcc tgggctcaag caatcctcct 3720

gccttggcct cccaaagtgc tgggattaca ggcatgatcc accgtgccca gccggtagac 3780

gtggtcttaa aaacagtgtt tacatggcca tcttgatgct tagaaagata attgattaaa 3840

atttaataag gcagggccaa ctccgagagt tcattgacaa cggcagcaaa aaggccctga 3900

attctgtact ttcttccccc agcctccttc tccagcaagg agaatagcac tcctccctcc 3960

agaagccagc tccctaagtt ggagccacta tgtaagagaa gaggaacgtt cactttttaa 4020

aattcatata tttaaaaatc aagaccaaaa agtaaattct gtactcctat tattgactgt 4080

agtcaatcaa acataaaaag gtgaaagtaa aatttaattt tttaccctta ttttactgac 4140

caatatggaa gttcttggta tctttaaggc tgaccttcct ggtattgtgt aatgattgaa 4200

tgtatctaaa ctgtaataat ttgaaactga caaacataac cttctcagac ttacaaaact 4260

atgttctttc taaagataca gatttttatt attttatttt gactaggaag gatttataaa 4320

taaatgtaat gaaaaatctt tgatcttaat aaagtacctt caaacagaat 4370

<210> 6
<211> 3705
<212> DNA
<213> Homo sapiens

<400> 6
agcctaactt cagaaggctt ctgcctggtg gtggggatcc tgacatcaag gatgggacac 60

cctggatgga ggttcctggg gcctggcccc caagactatg aagagccttt gctgaggcca 120

tgaggggtta ccatggcgac cgaggcagcc atccccgccc agcccgcttt gctgaccaac 180

agcatatgga cgtgggccct gctgccaggg ccccatacct gctgggctcc agggaggcct 240

tctccaccga gccccgcttc tgtgccccga gagctggcct gggacacatt tctcctgaag 300

ggcccctgag cctgagtgag gggccgtcgg taggccctga gggagggcca gcgggggccg 360

gggttggggg gggtagcagc accttcccca ggatgtaccc tggccagggc cccttcgaca 420

cctgtgaaga ctgtgtgggc cacccacagg gcaagggtgc cccccgcctg cctcctacac 480

tcctggatca gtttgaaaag cagttgccag ttcaacaaga tggcttccac acactaccat 540

accagcgagg gccagcaggg gcagggcccg ggccagcgcc agggacgggc actgccccag 600

agccccgcag tgagagccct agccgcatcc ggcacctggt tcattctgtg cagaagctct 660

ttgccaagtc ccactctctg gaggcgccgg ggaagcggga ctataatggg cccaaggctg 720

agggaagagg tggctctgga ggagacagct accccggccc gggctctgga ggcccccaca 780

cctcccatca ccaccatcac caccaccatc accaccacca ccagtcccgg cacggcaaga 840

ggagcaagag caaggaccgc aagggggatg ggcggcacca ggccaagtcc acaggctggt 900

ggagttccga tgacaacttg gacagtgata gcggcttcct ggcgggtggg aggccccctg 960

gggagcctgg tggtcccttc tgcctggagg gtccagatgg gtcctaccgg gacttgagct 1020

tcaaggggcg ctcgggcggg tcggaaggcc gctgccttgc ctgcactggc atgtccatgt 1080

cactggatgg acagtcggtc aagcgaagtg cctggcatac catgatggtc agccagggcc 1140

gggatggata cccgggggcc gggccaggca aggggctcct gggtccggag accaaggcca 1200

aagccaggac ttatcactat ctgcaggtgc cgcaagatga ctgggggggt taccccaccg 1260

gtggcaagga tggggagatc ccctgccgca ggatgcggag cggcagctac atcaaagcca 1320

tgggggatga ggagagcgga gactcagacg gcagccccaa gacatctccc aaagcagtcg 1380

cccgacgctt caccacccgt cgctcctcca gcgtggacca ggccaggatc aactgctgtg 1440

tcccaccccg gatccacccc cggagctcca tccctggcta cagccgttcc ctcaccactg 1500

gacagctcag cgatgagttg aaccagcagc tggaggccgt gtgcgggtcg gtgtttgggg 1560

agctggagtc ccaggccgtg gacgccctgg acctgcccgg ctgtttccgc atgcggagcc 1620

acagctacct ccgggccatc caggccggct gctctcaaga cgacgactgc ctgcccctcc 1680

tcgctacccc tgccgctgtc tcagggaggc ccggctcctc cttcaacttc agaaaggccc 1740

cgccccccat cccgccggga agccaggccc cgccccgcat ctccatcacc gcccagagca 1800

gcaccgactc cgcgcacgag agcttcacgg cggccgaggg ccccgcccgg cgctgcagct 1860

ccgccgacgg gctggacggc cccgccatgg gtgcgcgcac actggagttg gcgccggtgc 1920

cgccccgggc cagccccaag ccccccacac tcatcatcaa gaccatccct ggcagggagg 1980

agctgcggag cctggcgcgg cagcggaagt ggcggccgtc cattggggtg caggtggaga 2040

cgatctcaga ttcggacacc gagaacagga gccggaggga gttccactct attggcgtgc 2100

aggtggaaga ggacaagagg cgagcaaggt tcaagcgctc caatagtgtg acggctggcg 2160

tgcaggcaga cctggagctg gagggcctgg caggcctggc cacggtggcc acagaagaca 2220

aggccctgca gtttggacgc tccttccaga ggcacgcctc tgagccccag cctgggcccc 2280

gggcccccac ctactcagtc ttccgcacgg tccacacgca gggccagtgg gcctaccgcg 2340

agggctaccc actgccgtac gagccgccgg ccaccgatgg gtcgcccggc cctgcccccg 2400

cccccacccc cggccctggg gccggccgcc gtgactcctg gatagagcgc ggttcacgta 2460

gcctccccga ctcaggccgc gcatccccct gcccacgcga cggcgagtgg ttcatcaaga 2520

tgctgcgggc agaggtggag aagctggagc actggtgcca gcagatggag cgtgaggcgg 2580

aggactatga gctacccgag gagatcctgg agaagatccg cagtgctgtg ggcagcacac 2640

aacttctcct gtcccagaag gttcagcagt tcttccggct gtgtcagcaa agcatggatc 2700

ccactgcgtt ccctgtgccc accttccagg acctggcggg tttctgggac ctcctacagc 2760

tctccatcga ggatgtgacc ctcaagttcc tggagctaca gcaactcaag gccaacagct 2820

ggaaactcct ggagcctaag gaggagaaga aggtccctcc gccgatacca aagaagcccc 2880

tgcggggccg gggcgtgccg gtgaaggagc gctccctgga ctccgtggac cggcagcggc 2940

aggaagcgcg caagcggctc ctggcggcca agcgcgccgc ttccttccgc cacagctcgg 3000

ccaccgagag cgccgacagc atcgagatct acatccccga ggcccagacc aggctgtgac 3060

cggtccggcc cgcccagccc ggcccgggcc cgcggttctc cacccgtact gtacacccag 3120

cgtcgaggtc actgtgaacg cgggccgccc cgtgcgcccg ccccaccggc accggacgcc 3180

ccggcccccg ggcccgtcac actctcgtgg gttttttacc ttcctgatcc cacgcgaagg 3240

cgcccgggct gggcaggggg ccgtgcctct ccgccctgcg cccctcacct ggatcccctg 3300

cccacctggt ccgacgcttt gtccccacct cctccccatg ggcaccatct ctgccattct 3360

ttcccccacg ggccaggccg ggccgggtcc ctcatctggg ctctgcgtcc ccccctcccc 3420

caccccgcgg ggctgggctt cgtggggatc aagcttcgtg gctttttatg aagaatcccg 3480

aaccctgcct aggagcccgc cccaccctcc caggggctcc atcctcagcc ctctgcccac 3540

tgggcccagg gaccacagtg gctggaccaa cccaggacca gggcgcctgg gcctctcccc 3600

tttcccagcg gctggggagg ggagatgggg gcttcccctc accacacctg tggctgttcc 3660

cacatcccct tgaatatccc aggaaaaata aaacggcaga actgc 3705

<210> 7
<211> 50
<212> DNA
<213> Artificial

<220>
<223> Designed polynucleotide based on the sequence of SEQ ID NO:4 for
      use as a primer

<400> 7
ggggacaagt ttgtacaaaa aagcaggctt aatgccagct ttgtcaacgg 50

<210> 8
<211> 49
<212> DNA
<213> Artificial

<220>

<223>  Designed polynucleotide based on the sequence of SEQ ID NO:4 for
      use as a primer

<400>  8
ggggaccact ttgtacaaga aagctgggtc catcttggag atagagcgg                49


<210>  9
<211>  54
<212>  DNA
<213>  Artificial

<220>
<223>  Designed polynucleotide based on the sequence of SEQ ID NO:4 for
      use as a primer

<400>  9
ggggacaagt ttgtacaaaa aagcaggctg cagcaaagag gagacaccat caaa          54


<210>  10
<211>  24
<212>  DNA
<213>  Artificial

<220>
<223>  Designed polynucleotide based on the partial base sequence of
      NMDA receptor/2B subunit for use as a primer

<400>  10
tttggcttct acaaaccaag ggag                                           24


<210>  11
<211>  25
<212>  DNA
<213>  Artificial

<220>
<223>  Designed polynucleotide based on the partial base sequence of
      NMDA receptor/2B subunit for use as a primer

<400>  11
tccaatcata ccattccagg ttcca                                          25

<210>  12
<211>  49
<212>  DNA
<213>  Artificial

<220>
<223>  Designed polynucleotide based on the base sequence of NMDA
       receptor/2B subunit for use as a primer

<400>  12
ggggacaagt ttgtacaaaa aagcaggctt aatgaagccc agagcggag                49


<210>  13
<211>  48
<212>  DNA
<213>  Artificial

<220>
<223>  Designed polynucleotide based on the base sequence of NMDA
       receptor/2B subunit for use as a primer

<400>  13
ggggaccact ttgtacaaga aagctgggtc ctgttccctc actcagac                 48


<210>  14
<211>  23
<212>  DNA
<213>  Artificial

<220>
<223>  Designed polynucleotide based on the base sequence of NMDA
       receptor/2B subunit for use as a primer

<400>  14
tgagaagaat gaggtcatga gca                                            23


<210>  15
<211>  20
<212>  DNA
<213>  Artificial

<220>
<223>  Designed polynucleotide based on the base sequence of NMDA
      receptor/2B subunit for use as a primer

<400>  15
gtcacagtcg tagagcccta                                                    20


<210>  16
<211>  24
<212>  DNA
<213>  Artificial

<220>
<223>  Designed polynucleotide based on the base sequence of NMDA
      receptor/2B subunit for use as a primer

<400>  16
agttccgaac aaaggagaac tcac                                               24


<210>  17
<211>  25
<212>  DNA
<213>  Artificial

<220>
<223>  Designed polynucleotide based on the base sequence of NMDA
      receptor/2B subunit for use as a primer

<400>  17
gagttctgac ccgtcaccgt cgtgg                                              25


<210>  18
<211>  49
<212>  DNA
<213>  Artificial

<220>
<223>  Designed polynucleotide based on the sequence of SEQ ID NO:6 for
      use as a primer

<400>  18
ggggacaagt ttgtacaaaa aagcaggctt aatgaggggt taccatggc                    49

<210> 19
<211> 48
<212> DNA
<213> Artificial

<220>
<223> Designed polynucleotide based on the sequence of SEQ ID NO:6 for
use as a primer

<400> 19
ggggaccact ttgtacaaga aagctgggct agtacgggtg gagaaccg 48